# EUROPEAN PATENT APPLICATION

(11) **EP 4 497 755 A1**
(43) Date of publication of application: **29.01.2025**
(21) Application number: 23382786.4
(22) Date of filing: 28.07.2023
(51) Int. Cl.: C07K 16/22, A61P 35/00, A61K 39/395

(54) **ANTI-WNT-11 ANTIBODIES**

(71) Applicant: Asociación Centro de Investigación Cooperativa en Biociencias - CIC bioGUNE, 48160 Derio (ES)
(72) Inventor: KYPTA, Robert, E-48160 Derio, Vizcaya (ES); VIVANCO RUIZ, María del Mar, E-48160 Derio, Vizcaya (ES); SÁNCHEZ YAGÜE, Saray, E-48980 Santurtzi, Vizcaya (ES); GORROÑO ETXEBARRIA, Irantzu, E-48160 Derio, Vizcaya (ES)
(74) Representative: ABG Intellectual Property Law, S.L.

(57) **Abstract**

The invention relates to an agent binding specifically to the sequence shown in SEQ ID NO: 1, SEQ ID NO: 2 or SEQ ID NO: 3 wherein said agent is an immunoglobulin agent or a non-immunoglobulin agent selected from the group consisting of a peptide aptamer, a nucleic acid aptamer, a DARPin, an affibody, and an anticalin. The invention aslo relates to a nucleic acid, an expression cassette, a vector, a cell and a nanoparticle comprising the agent of the invention. The invention also relates to a pharmaceutical composition and to its use in preventing and/or treating cancer, metastasis or fibrosis. The invention also relates to a method for screening compounds capable of inhibiting Wnt-11.

## Description

### FIELD OF THE INVENTION

The invention relates to the field of cancer therapies.

### BACKGROUND OF THE INVENTION

Current therapies for cancer have improved patient survival. However, deaths remain high in patients whose tumors have metastasized or have become resistant to therapy. This is the case of metastatic/castration-resistant prostate cancer (CRPC), where there is an unmet need to develop new treatments beyond those that target the androgen receptor. Wnt signaling has been found to be hyperactive in metastatic and CRPC. Wnt proteins signal in a context-dependent manner by binding to a variety of receptors, including ten FZD family members, LRP5/6 and ROR1/2 co-receptors and the context-specific co-receptors PTK7, MuSK, RECK 7 to activate beta-catenin dependent and independent pathways.

Wnt-11 is a b-catenin-independent Wnt that is a potential therapeutic target in prostate, colorectal and pancreatic cancer, showing increased expression in tumors and reduced survival of patients with tumors that express high levels of Wnt-11. In prostate cancer, Wnt-11 signals via FZD8, playing a role in activation of EMT. Gene silencing and ectopic expression studies in prostate cancer cell lines have demonstrated roles for Wnt-11 in neuroendocrine-like differentiation, migration and invasion.

Wnt inhibitors currently in clinical trials either target the acyltransferase porcupine to block Wnt secretion or target Wnt receptors or b-catenin and its associated proteins. These approaches lack specificity at the level of the Wnt ligand and do not directly inhibit b-catenin-independent Wnt signals activated in CRPC. The complexity of Wnt signaling calls for approaches that improve target specificity and do not affect the activities of Wnt ligands associated with normal tissue homeostasis, which in some contexts inhibit tumor progression.

Therefore, there is a need in the art to provide alternative Wnt inhibitors suitable for treating cancer.

### SUMMARY OF THE INVENTION

In a first aspect, the invention relates to an agent binding specifically to the sequence shown in SEQ ID NO: 1, SEQ ID NO: 2 or SEQ ID NO: 3 wherein said agent is an immunoglobulin agent or a non-immunoglobulin agent selected from the group consisting of a peptide aptamer, a nucleic acid aptamer, a DARPin, an affibody, and an anticalin.

In a second aspect, the invention relates to an antibody construct comprising the antigen-binding fragment according to the invention, wherein the antibody construct is selected from the group consisting of scFv, scFv-Fc, minibody, (scFv)₂ and diabody.

In a third aspect, the invention relates to a combination comprising:
a) an agent binding to the sequence shown in SEQ ID NO: 1 , SEQ ID NO: 2 or SEQ ID NO: 3 according to the invention or an antibody construct according to the invention, and
b) an anti-androgen compound.

In a fourth aspect, the invention relates to a nucleic acid selected form the group consisting of:
a) a nucleic acid encoding the agent according to the invention wherein the agent is a protein compound, or the antibody construct according to the invention and
b) a complementary nucleic acid of a nucleic acid as defined in a)
or to an expression cassette comprising the nucleic acid according to the invention, to a vector comprising the nucleic acid according to the invention or the expression cassette according to the invention or to a cell comprising the nucleic acid according to the invention, or the expression cassette according to the invention, or the vector according to the invention.

In a fifth aspect, the invention relates to a nanoparticle comprising the agent according to the invention or the antibody construct according to the invention.

In a sixth aspect, the invention relates to a pharmaceutical composition comprising a therapeutically effective amount of an agent according to the invention, an antibody construct according to the invention, a combination according to the invention or a nanoparticle according to the invention, together with a pharmaceutically acceptable excipient or carrier.

In a seventh aspect, the invention relates to the agent according to the invention, the antibody construct according to the invention, the combination according to the invention, the nanoparticle according to the invention or the pharmaceutical composition according to the invention for use in medicine.

In an eighth aspect, the invention relates to the agent according to the invention, the antibody construct according to the invention, the combination according to the invention, the nanoparticle according to the invention or the pharmaceutical composition according to the invention for use in the treatment of cancer, metastasis or fibrosis.

In a ninth aspect, the invention relates to a peptide having the sequence shown in SEQ ID NO: 3, SEQ ID NO: 24, SEQ ID NO: 35, SEQ ID NO: 36 or a variant thereof having at least 70% sequence identity with said sequences.

In a tenth aspect, the invention relates to a method for screening compounds capable of inhibiting Wnt11 which comprises:
a) contacting the compound with a cell stably expressing high levels of Wnt-11 to allow the binding of the compound to Wnt-11 thereby forming a complex,
b) incubating the complex formed in a) with an anti-Wnt11 antibody in conditions to allow the binding of the antibody to Wnt-11, and
c) quantifying the binding of the antibody to Wnt-11, wherein if the binding is reduced compared to the binding of a control, then the compound is an inhibitor of Wnt11.

### DESCRIPTION OF THE FIGURES

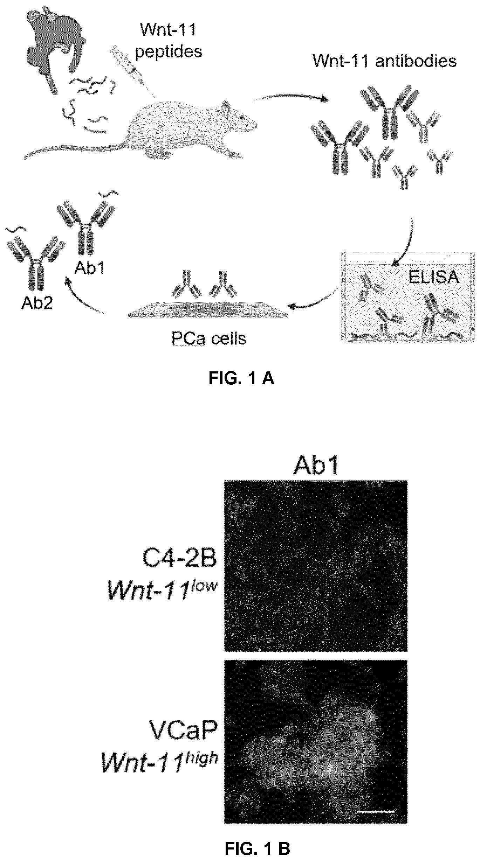

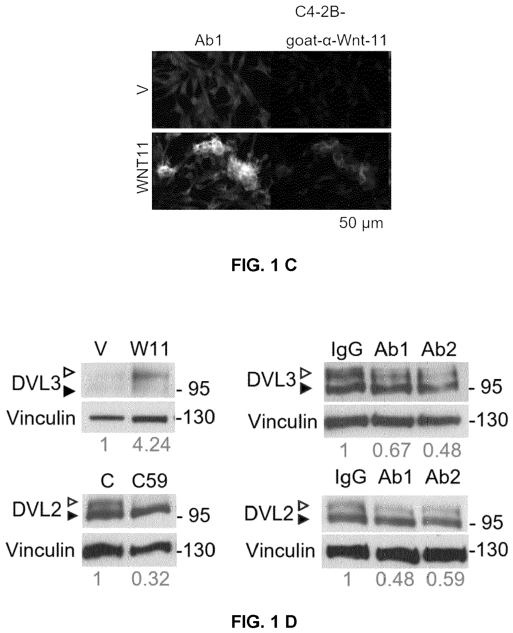

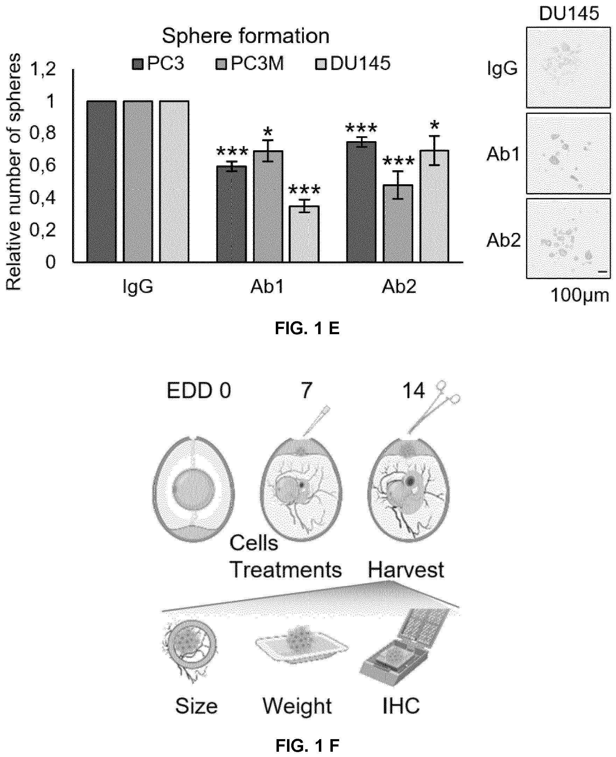

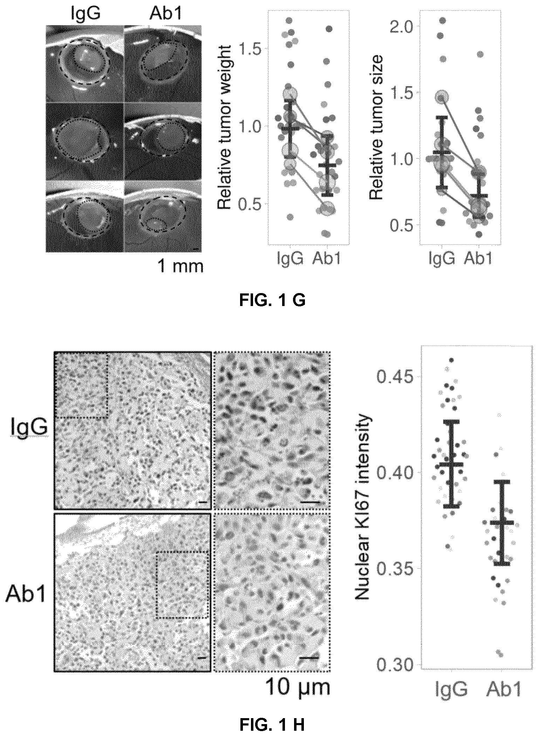

**Figure 1****.** Antibody-mediated inhibition of Wnt-11 signaling reduces prostate cancer cell sphere formation and tumor growth. a) Schematic of anti-Wnt-11 rat monoclonal antibodies Ab1 and Ab2 development and selection. PCa: prostate cancer. b) Immunofluorescence staining of non-permeabilized C4-2B (Wnt-11low) and VCaP (Wnt-11 high) cells using Ab1. c) Immunofluorescence staining of nonpermeabilized C4-2B-V (control vector, Wnt-11 low) and C4-2B-WNT11 cells (overexpressing, Wnt-11high) with Ab1 and goat anti-Wnt-11 (R&D AF2647). d) Representative western blots of extracts from cells transfected with empty vector (V) or Wnt-11 (top left) or treated with vehicle (DMSO, C) or Wnt-C59 (bottom left) or with control IgG, Ab1 or Ab2 (bottom right). e) Numbers of spheres, relative to control, formed by prostate cancer cells treated for 7 days with control IgG, Ab1 or Ab2; PC3, n=5; PC3M and DU145 n=4; Students t test *p<0.05, ** p<0.01, *** p<0.001; representative images of DU145 spheres treated as indicated are shown on the right. f) Schematic of chick chorioallantoic membrane (CAM) assay experiments; EDD embryonic development day. g) Representative images of tumors formed by PC3 cells treated with Ab1 or control IgG for 7 days (left); graphs plotting tumor weight (IgG n=27, Ab1 n=26) and size (IgG n=21, Ab1 n=23) from 5 independent experiments, *p = 0.0173 (weight), p=0.0175 (size), paired Students t test. h) Representative images of Ki67 immunohistochemical staining of sections from tumors formed by PC3 cells treated with Ab1 or control IgG (left) and quantification (right) upon analysis of sections (IgG n=45, Ab1 n=34) from 6 tumors from 2 independent experiments, *p=0.0481 paired Students t test.

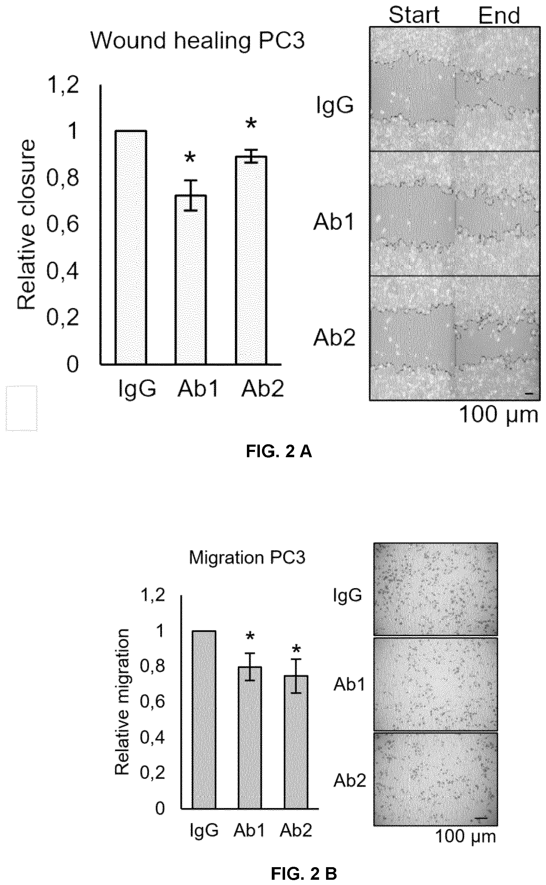

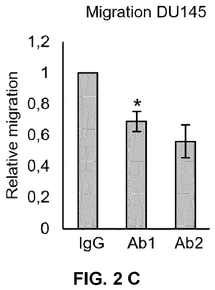

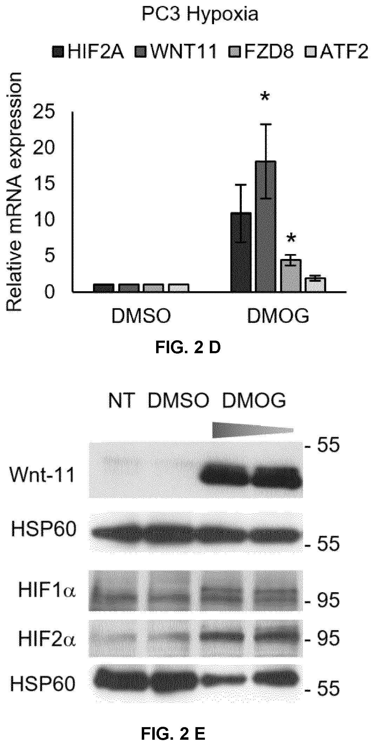

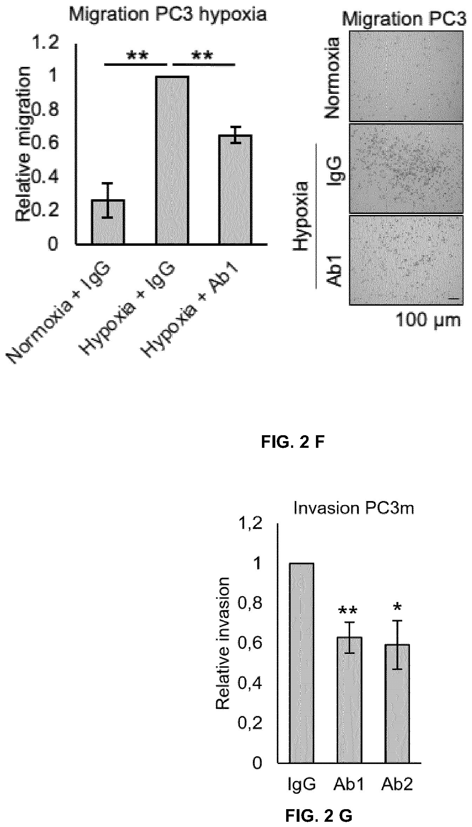

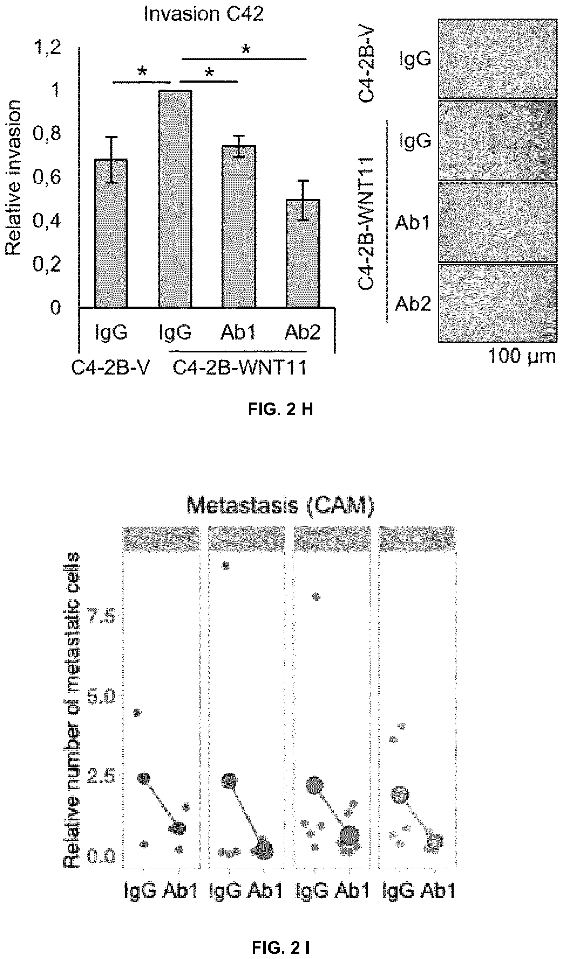

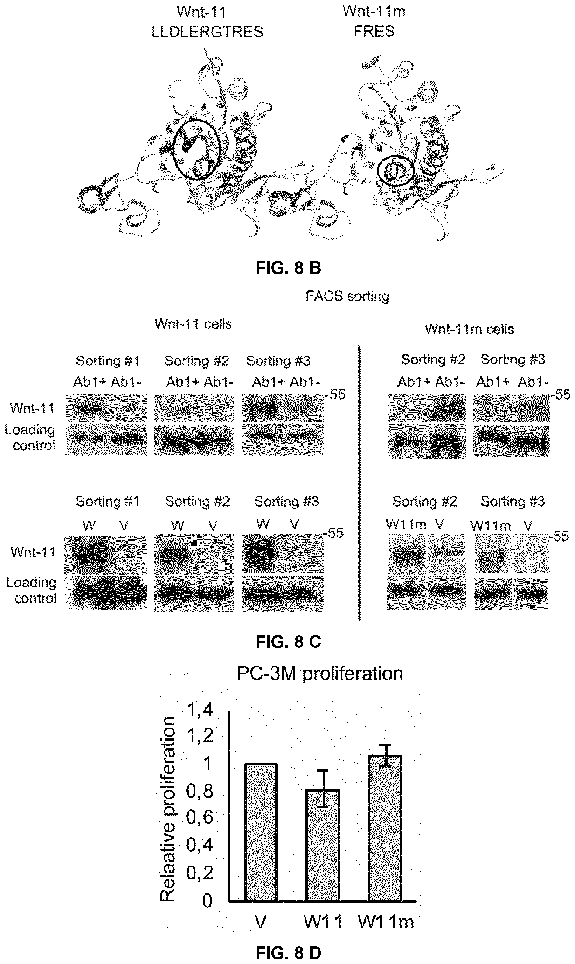

**Figure 2****.** Antibody-mediated inhibition of Wnt-11 reduces prostate cancer cell migration in normoxia and hypoxia, invasion and metastasis, a) PC3 cell migration, as measured using wound healing assays, in cells treated with control (IgG) or Wnt-11 (Ab1 and Ab2) antibodies; graph shows quantification of results from 3 experiments, images show representative results. b) PC3 cell transwell migration upon Ab1 and Ab2 treatments, quantification (n=3; left) and representative images (right). c) DU145 cell migration, as measured using transwell assays, graph shows quantification of results from 3 experiments, images show representative results; Ab2 vs. IgG p=0.054. d) Analysis of WNT11, FZD8, HIF2A, ATF2 mRNA expression on PC3 cells treated with the hypoxia mimetic agent dimethyloxalylglycine DMOG (1 mM, 48 h). e) Western blots of extracts of PC3 cells treated with DMOG (1 mM and 0.2 mM, 24 h) probed for the indicated proteins. f) Transwell migration assays of PC3 cells cultured in normoxia or hypoxia treated with control (IgG) or Wnt-11 antibodies (Ab1), graph shows quantification of results from 3 experiments, images show representative results. g) Graph showing quantitation of transwell invasion assays of PC3m cells treated as indicated (Ab1 n=5, Ab2 n=4); representative images are in Fig.8d. h) Transwell invasion assays of empty vector (V) and Wnt-11-expressing (WNT11) C4-2B cells, showing quantification (n=4) and representative images. i) Graphs showing quantitation of chick distal CAM metastasis assays, data from 4 independent experiments, using 16 IgG-treated and 20 Ab1-treated eggs; p=0.0018, paired Students t test. Other p-values, * p<0.05, ** p<0.01, or as indicated, Students t test.

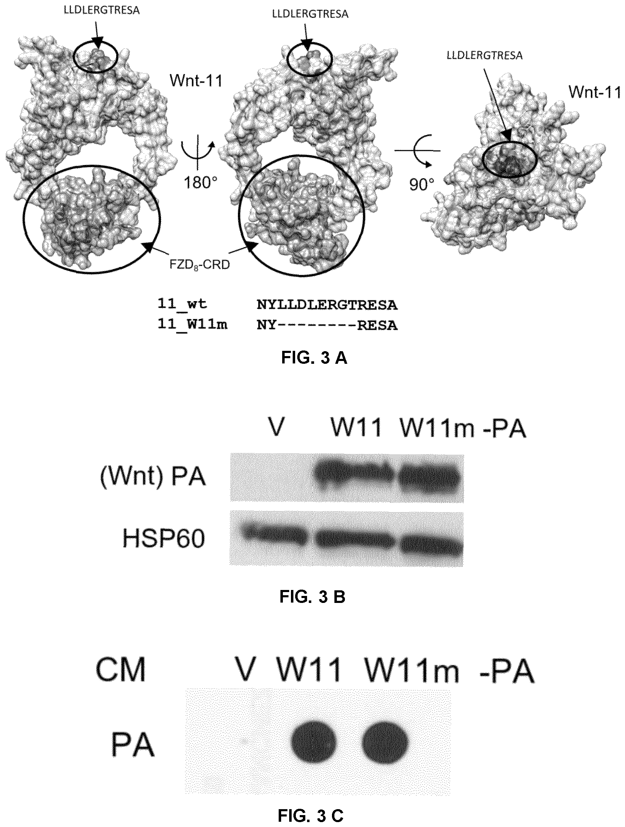

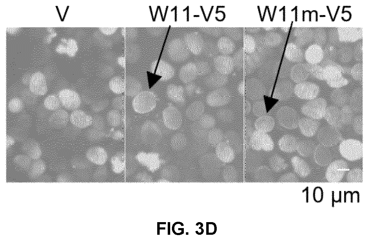

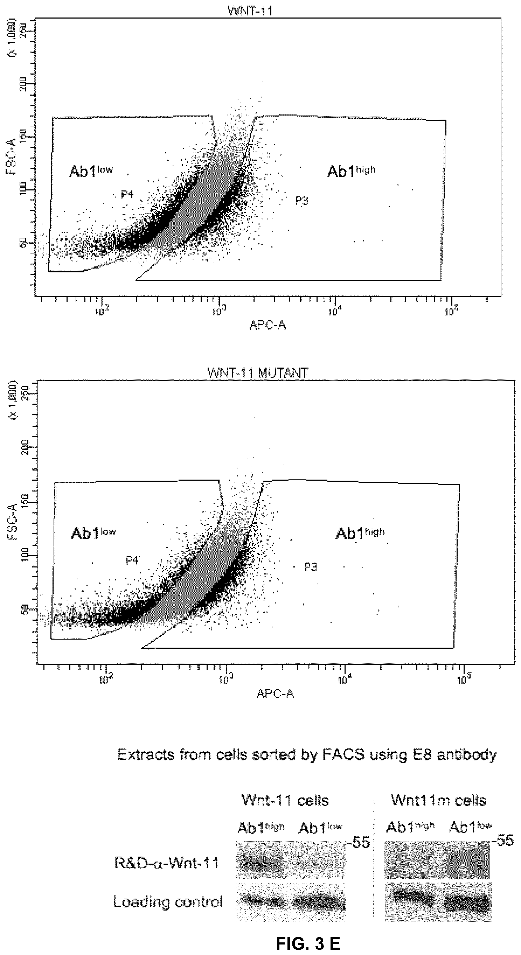

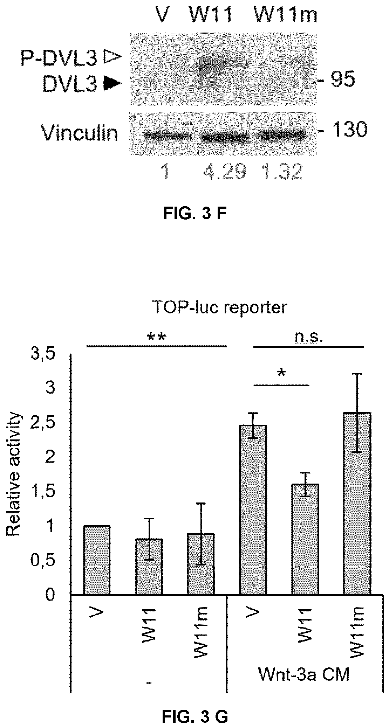

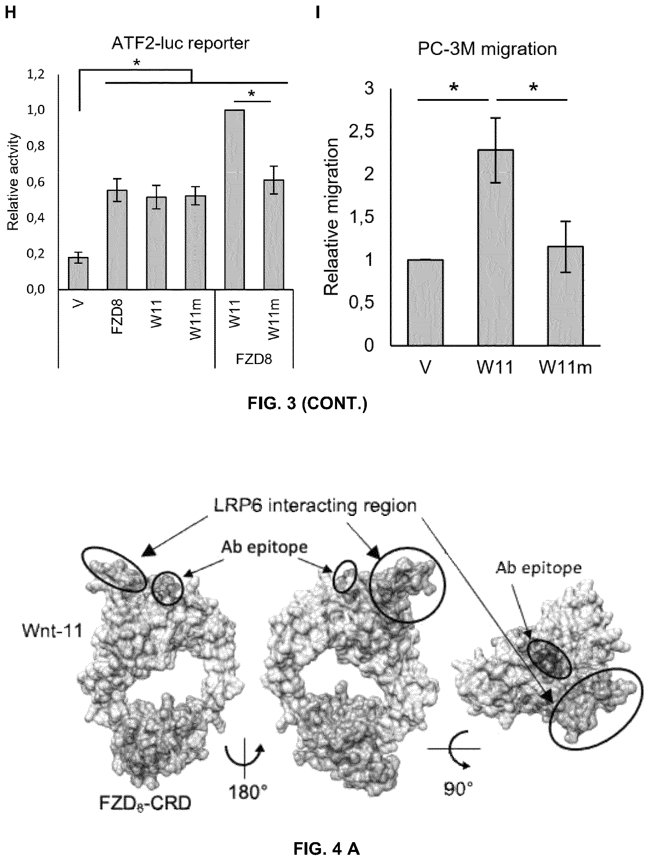

**Figure 3****.** The antibody epitope is required for Wnt-11 signaling and function, a) The antibody epitope region in the structure of predicted Wnt-11 in complex with FZD8-CRD together with the sequence for the Wnt-11 antibody epitope deletion mutant (Wnt-11m). b) Western blot analysis of PA-tagged Wnt-11 and Wnt-11m expression in transfected HEK 293 cells; HSP60 was used as a loading control. c) Anti-PA-tag dot blot analysis of CM from HEK 293 cells transfected with PA-tagged Wnt-11 and Wnt-11m. d) Anti-V5 tag immunocytochemistry of non-permeabilized HEK 293 cells transfected with V5-tagged forms of Wnt-11 and Wnt-11m, showing cell surface localization. e) Ab1 recognition of cells transfected with wild-type Wnt-11 but not with the epitope mutant, Wnt-11m, as assessed by FACS: the representative FACS plots highlight the Ab1^{high} (P3) and Ab^{low} (P4) cell populations that were sorted and from which cell extracts were probed by western blotting using goat anti-Wnt-11 antibodies and a loading control; representative western blots show enrichment of Wnt-11 but not Wnt-11m in the Ab1^{high} population. f) Anti-DVL3 western blot of extracts of HEK 293 cells transfected with Wnt-11 and Wnt-11m; vinculin was used a loading control. g) b-catenin/Tcf (TOP-luc) gene reporter assays of extracts from HEK 293 cells transfected with empty vector (V), Wnt-11 or Wnt-11m and treated with control (-) or Wnt-3a CM. h) ATF2 gene reporter assays of extracts from PC3 cells transfected as indicated. i) Boyden chamber migration assays of PC-3M cells transfected as indicated; * p<0.05, ** p<0.01.

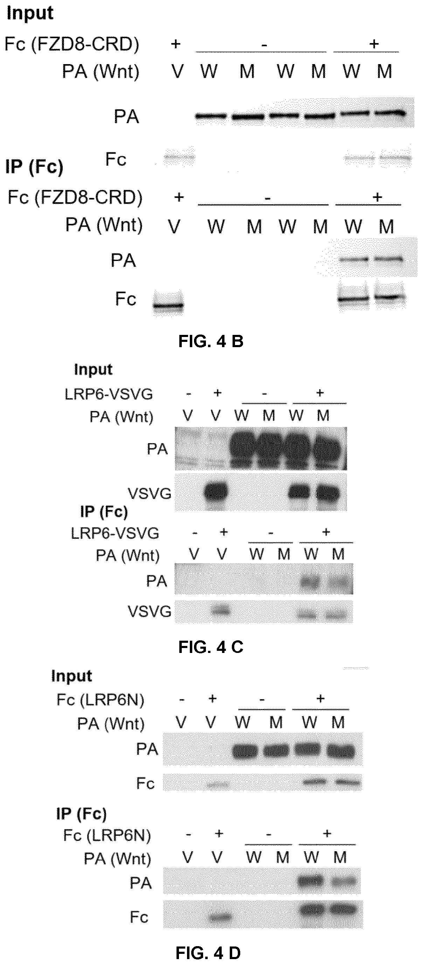

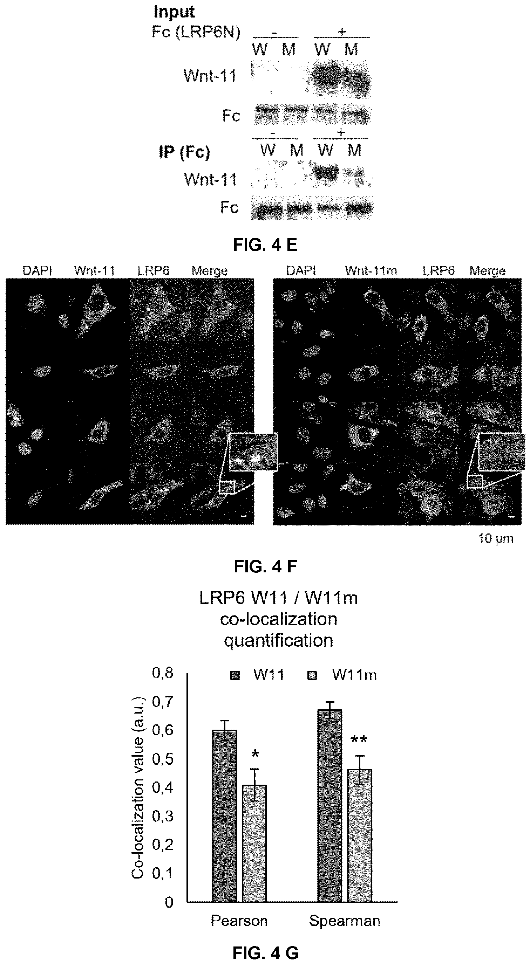

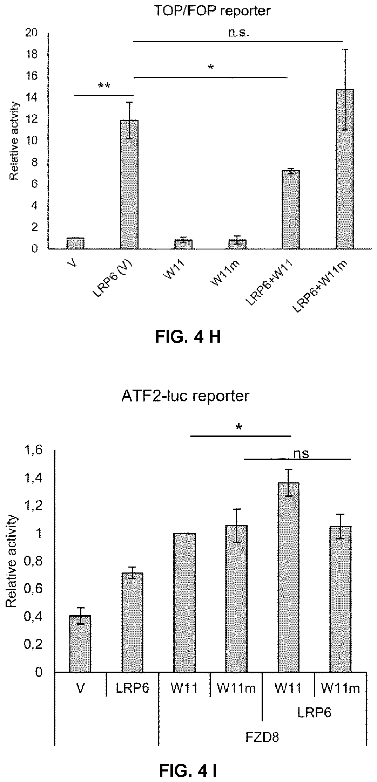

**Figure 4****.** Impact of the antibody epitope on Wnt-11 binding and signaling through LRP6. a) AlphaFold predicted structure of Wnt-11 with the antibody epitope and the proposed LRP6 binding site and FZD8-CRD. b) Representative western blots of extracts (input) and protein A/G-agarose pull-downs (IP) from PC3 cells expressing FZD8-CRD-Fc, PA-tagged Wnt-11 (W), Wnt-11m (M) or empty vector (V), as indicated, probed for PA or Fc. c) Representative blots of extracts and immune precipitates from HEK 293 cells expressing PA-tagged Wnt-11, Wnt-11m, VSV-G-tagged LRP6 or empty vector, as indicated, probed for PA or VSV-G. d) Representative blots of extracts and protein A/G-agarose pull-downs from HEK 293 cells expressing PA-tagged Wnt11, Wnt-11m, Fc-tagged LRP6 extracellular domain (LRP6N) or empty vector, as indicated, probed for PA or Fc. e) Representative blots of extracts and protein A/G agarose pull-downs from PC3 cells expressing Wnt-11, Wnt-11m, LRP6N or empty vector, as indicated, probed for Wnt-11 or Fc. f) Immunofluorescence images of cells transfected with Wnt-11 or Wnt-11m and LRP6 and stained for Wnt-11 (m), LRP6 and nuclei (DAPI,); merged images show colocalization with magnified insets. g) Quantification of immunofluorescence colocalization with LRP6 (n= 25 (Wnt-11) and 16 (Wnt-11m) cells). h) Canonical Wnt (TOP-luc) gene reporter assays from extracts of HEK 293 cells expressing Wnt11, Wnt-11m, LRP6 and empty vector, as indicated, i) Noncanonical Wnt (ATF2-luc) gene reporter assays from extracts of HEK 293 LRP5/6-/- cells expressing Wnt11, Wnt-11m, FZD8, LRP6 and empty vector, as indicated; * p<0.05, ** p<0.01, n.s. not significant.

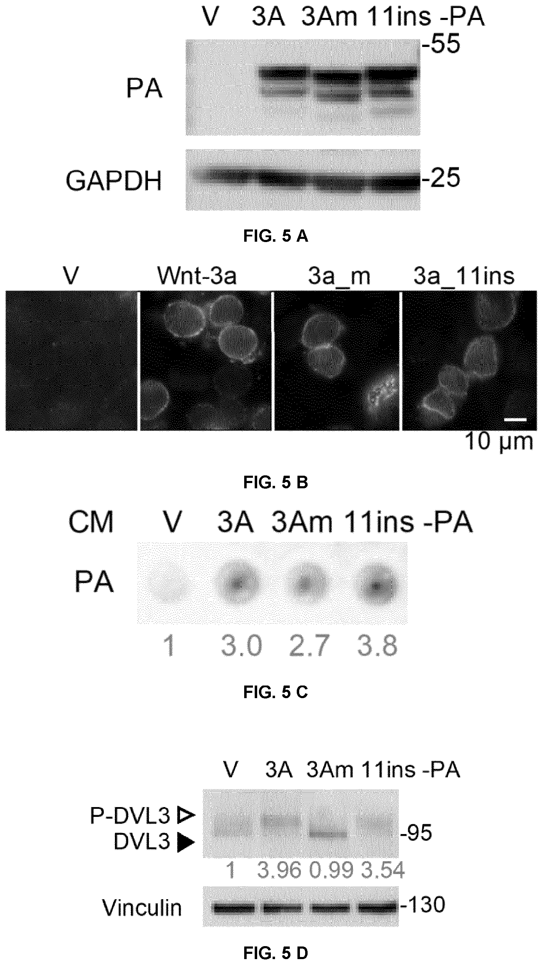

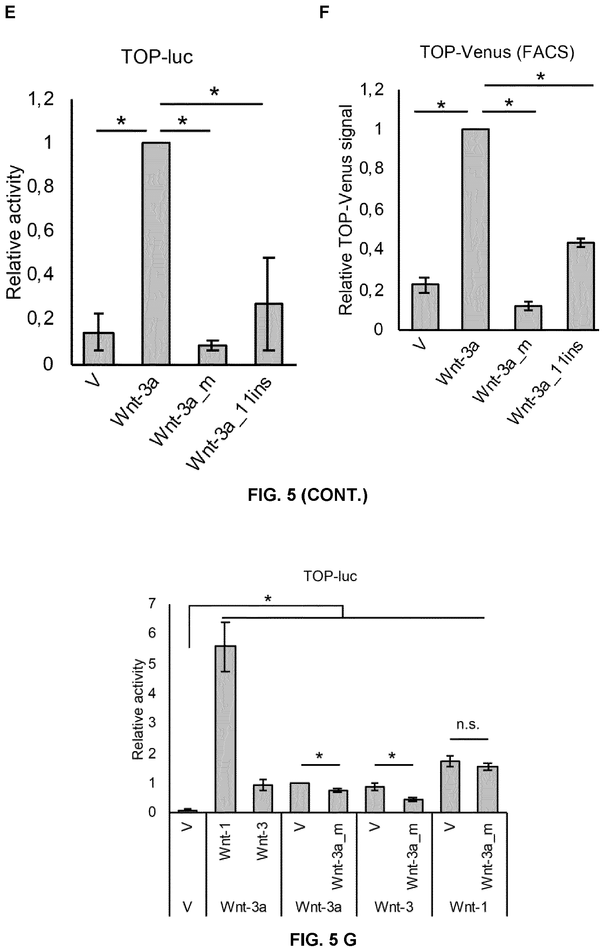

**Figure 5****.** Mutation of Wnt-3a at the site equivalent to the Wnt-11 target site impairs Wnt-3a-dependent signaling. a) Extracts from HEK 293 cells transfected with the indicated PA-tagged Wnt-3a plasmids or empty vector (V) probed for PA-tag and GAPDH as a loading control. b) Immunofluorescence of nonpermeabilized HEK 293 cells transfected with the indicated Wnt-3a constructs or empty vector (V) stained using anti-PA antibodies, c) Dot blot detection of the indicated PA-tagged Wnt-3a proteins in conditioned media (CM) from transfected HT1080 cells; numbers indicate relative spot intensity from densitometry analysis. d) Extracts from extracts of HT1080 cells transfected with the indicated constructs probed for DVL3 and vinculin as a loading control. e) b-catenin/Tcf (TOP-luc) gene reporter assays using extracts of HEK 293 cells transfected with the indicated constructs; graph shows activity relative to Wnt-3a (n=4). f) Fluorescence-based (Venus) gene reporter assays measuring the effects on b-catenin/Tcf-dependent transcription of the indicated constructs in HT1080 cells (n=4). g) b-catenin/Tcf (TOP-luc) gene reporter assays measuring the effects of the indicated constructs; graph shows activity relative to Wnt-3a/V (n=4). *p<0.05, Students t test.

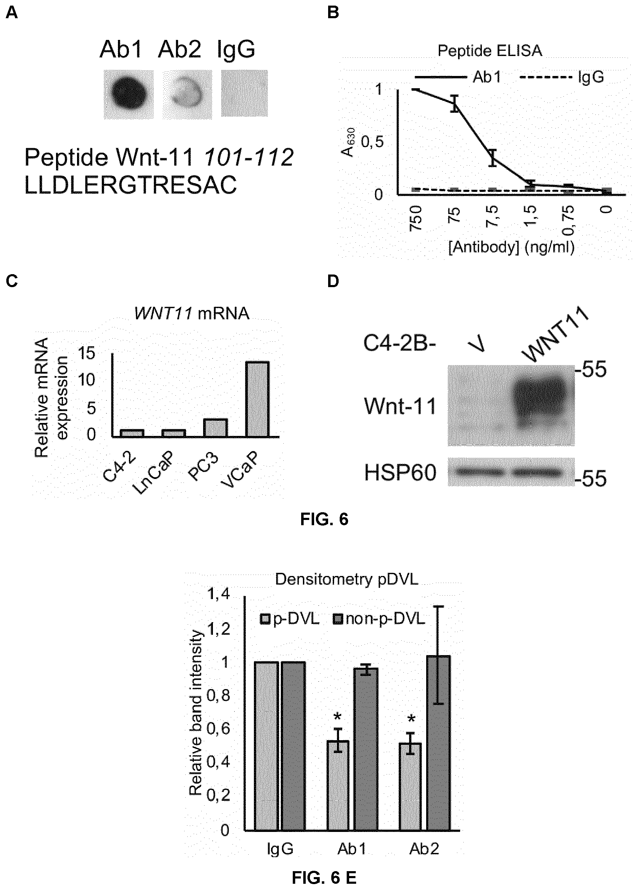

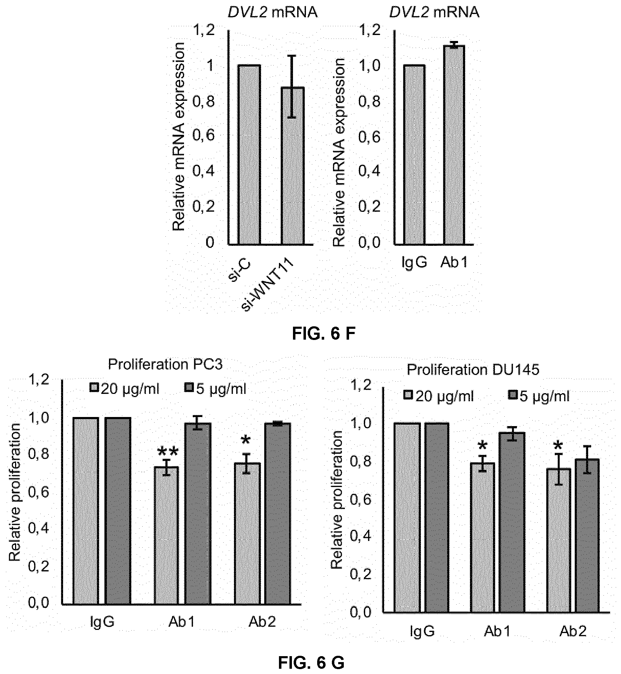

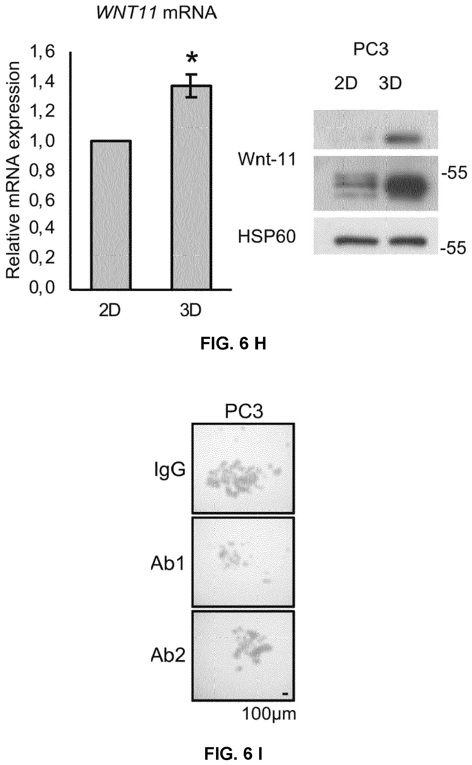

**Figure 6** a) Dot blots using the peptide LLDLERGTRESAC (SEQ ID NO: 76), corresponding to Wnt-11 amino acids 101-112 with a C-terminal cysteine probed using Ab1, Ab2 or rat IgG. b) ELISAs using the peptide LLDLERGTRESAC (SEQ ID NO: 76) comparing Ab1 and control IgG. c) WNT11 mRNA levels, relative to 36B4, in the indicated prostate cancer cell lines. d) Western blots of extracts from C4-2B-V and C4-2BWNT11 cells probed for Wnt-11 and HSP60 as a loading control. e) Densitometric analysis of DVL2 western blots, comparing intensities, relative to control, of the upper (phosphorylated) and lower bands in extracts from cells treated with Ab1, Ab2 and control IgG (n=3). f) DVL2 mRNA expression levels in cells treated with control IgG or Ab1 (left) or transfected with control or WNT11 siRNAs (right). g) Graphs from crystal violet assays for PC3 and DU145 cells treated with 20 or 5 µg/ml Ab1, Ab2 or control IgG for 7 days, results shown are relative to control IgG (n(PC3)=3, n(DU145)=4). h) WNT11 mRNA (left) and Wnt-11 protein (middle) expression in monolayer (2D) and sphere (3D) cultures of PC3 cells. i) Representative images of spheres formed by PC3 cells treated with Ab1, Ab2 and control IgG, associated with Fig.1e. Students t test p-values indicated as follows: * p<0.05, ** p<0.01.

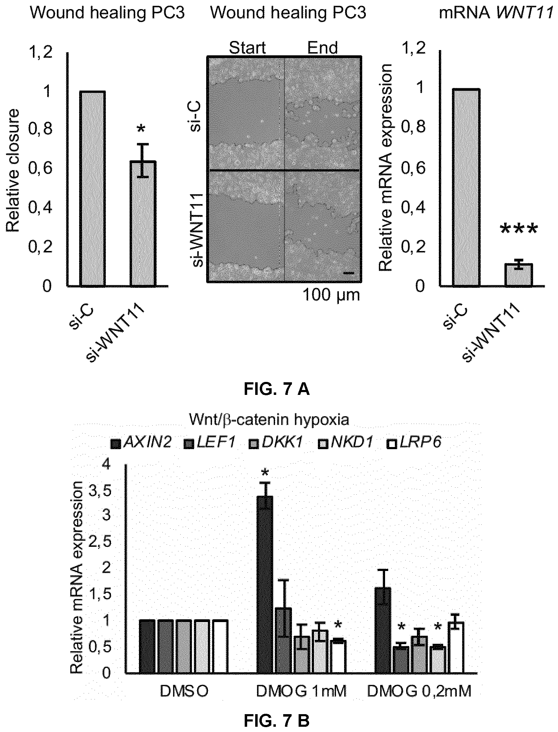

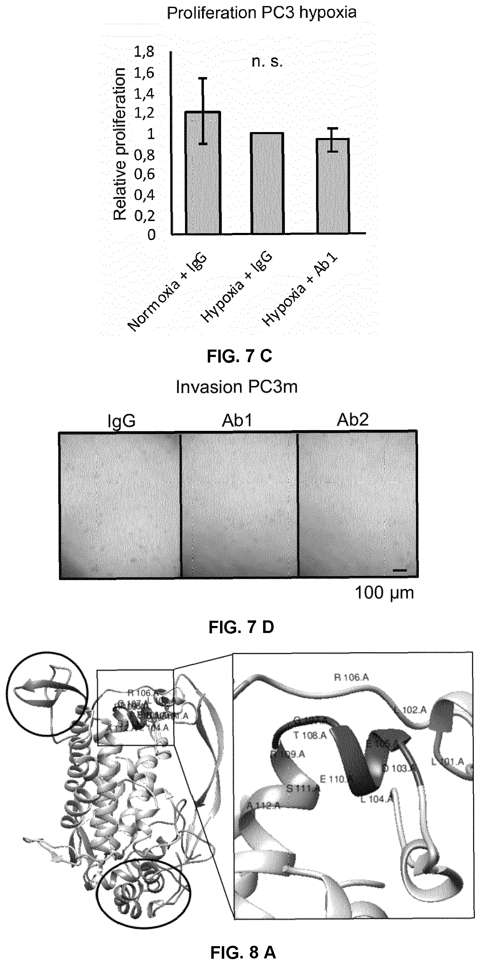

**Figure 7** a) PC3 cell migration by wound healing assay upon WNT11 gene silencing quantification (n=3; left), representative images (middle) and analysis of WNT11 mRNA expression upon WNT11 gene silencing in PC3 cells. b) mRNA expression of AXIN2, LEF1, DKK1, NKD1 and LRP6 by qRT-PCR in PC3 cells silenced treated with the hypoxia mimetic DMOG at 1 mM and 0.2 mM for 48 h. c) Proliferation of PC3 cells in hypoxia and Ab1 treatment, associated with Fig. 2f. d) Representative images of PC3m cell invasion upon Ab1 and Ab2 treatments, associated with Fig. 2g. Students t test p-values indicated as follows: * p<0.05, *** p<0.001, n. s. not significant.

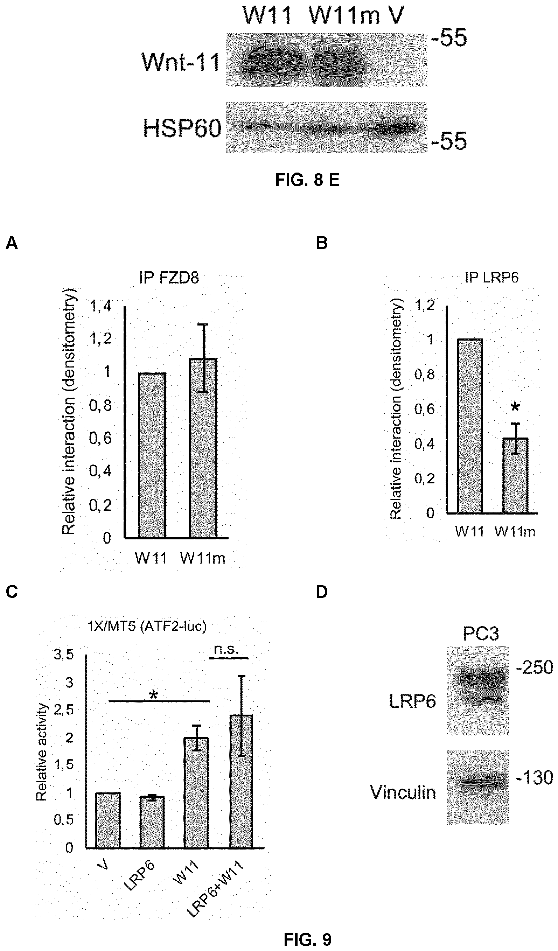

**Figure 8.** a) The structure of predicted Wnt-11 with the antibody epitope region (boxed area with inset) and the LRP6 putative interacting region (circled area on left) highlighted in complex with FZD8-CRD (circled area at bottom), inset with amplification to the antibody epitope region secondary structure. b) Prediction using AlphaFold for the mutant Wnt-11m (right, FRES circled) compared to the wild type Wnt-11 (left, LLDLERGTRESA (SEQ ID NO: 3) circled). c) Ab1 recognition of Wnt-11 and Wnt-11m assessed by FACS and western blot of Wnt-11 in the FACS-sorted Ab1high and Ab1!ow populations, western blots from the replicate experiments. d) Effect of Wnt-11 and Wnt-11m ectopic expression on PC3M cell proliferation, as control for the migration shown in Fig. 3i. e) Western blot of the Wnt-11 and Wnt-11m ectopic expression on PC3M, as control for the migration shown in Fig. 3i.

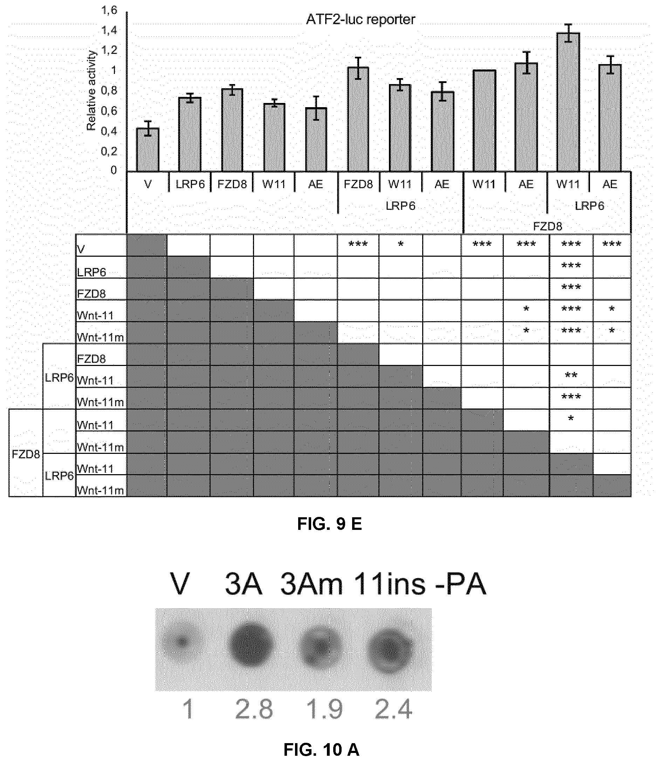

**Figure 9****.** a) Quantification by western blot densitometry of Fc pull-down assays comparing Wnt-11 and Wnt-11m binding to FZD8-CRD (Fig. 4b, n=4). b) Quantification by western blot densitometry of immunoprecipitation assays comparing Wnt-11 and Wnt-11m binding to VSV-G-tagged LRP6 (n=3). c) Corrected ATF2-dependent gene reporter activity (1x/mt5) upon LRP6 and Wnt-11 ectopic expression in PC3 cells (n=3). d) Western blot of PC3 cell extracts probed for LRP6 and vinculin as a loading control. e) ATF2-dependent gene reporter activity upon the ectopic expression of indicated constructs in HEK 293 LRP5/6-/- cells: plot (top) and statistical analysis showing Tukey-corrected p-values of the indicated comparisons (bottom) (n=5). Students t test p-values indicated as follows: * p<0.05, ** p<0.01, *** p<0.001, n. s. not significant.

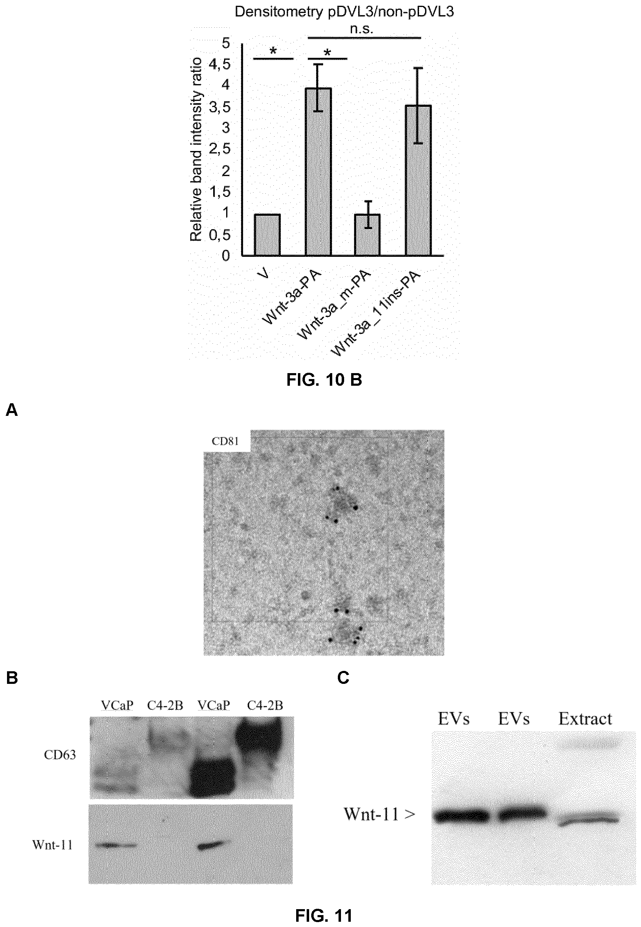

**Figure 10****.** a) Dot blot detection of the indicated PA epitope-tagged Wnt-3a proteins in conditioned media (CM) from transfected HEK 293 cells. b) Densitometry of DVL3 phosphorylation upon ectopic expression of the indicated Wnt-3a constructs (n=3). Students t test p-values indicated as follows: * p<0.05, n. s. not significant.

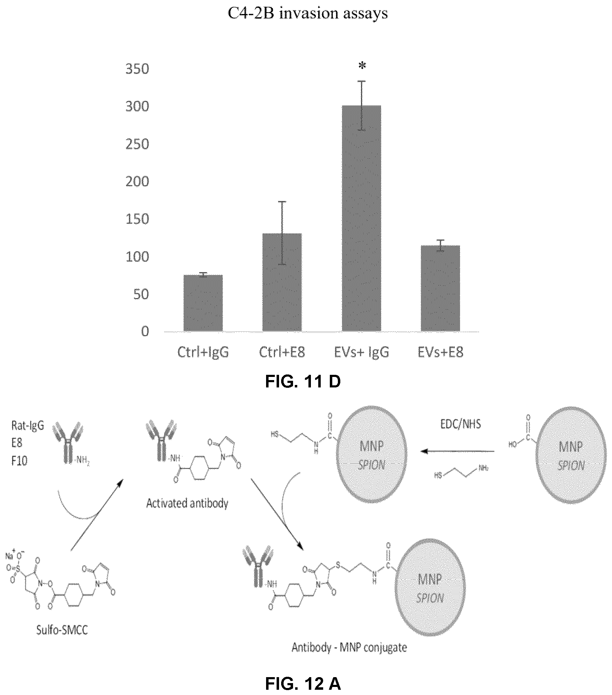

**Figure 11****.** Wnt-11 antibody E8 inhibits C4-2B cell invasion promoted by VCaP cell derived positive extracellular vesicles (EVs). A) Immunoelectron microscopy detection of CD81 on VCaP cell extracellular vesicles (EVs) B) Anti-CD63 and anti-Wnt-11 western blots of EVs from VCaP and C4-2B cells C) Anti-Wnt-11 western blot of VCaP cell extract and VCaP cell EVs used for invasion assays D) Invasion assays comparing the effects of control IgG and Wnt-11 antibody E8 on the C4-2B cell response to VCaP EVs.

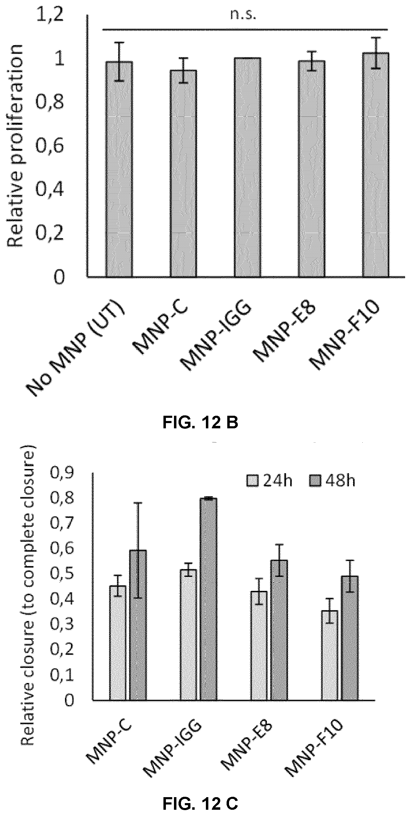

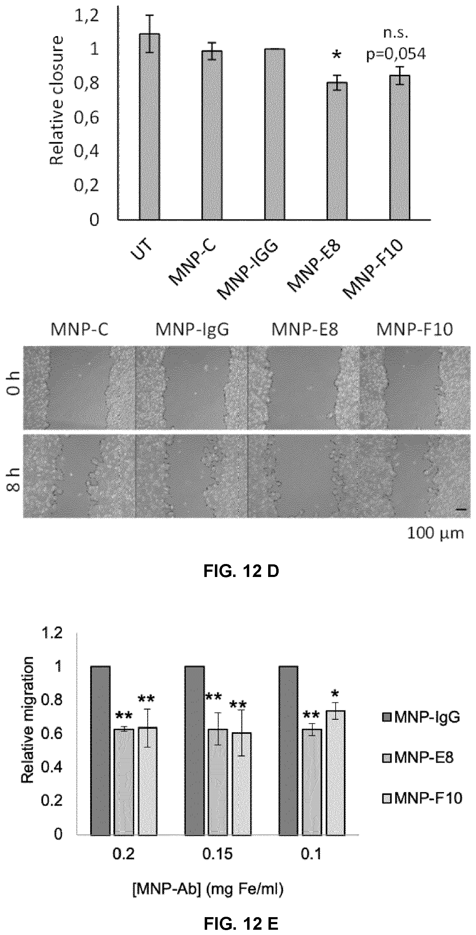

**Figure 12****.** Wnt-11 antibody nanoparticles inhibit PC3 and PC3m cell migration. A) Conjugation of antibodies to magnetic nanoparticles (MNPs) using sulfo-SMCC and ECD/NHS-activated SPIONs. 1-Ethyl-3-(3-dimethylaminopropyl) carbodiimide (EDC); n-hydroxysuccinimide (NHS); sulfosuccinimidyl-4-(N-maleimidomethyl) cyclohexane-1-carboxylate (Sulfo-SMCC), superparamagnetic iron oxide nanoparticles (SPIONs); modified from Trabulo et al., Biochim Biophys Acta Gen Subj . 2017 Jun;1861(6):1597-1605. B) PC3 cell proliferation determined by crystal violet assay after treatment for 5-7 days as indicated; values normalized to treatment with IgG-MNPs (n=3). C) PC3 wound healing assays after 24 and 48 h, error bars indicate SEM (n=1, 6 replicates). D) PC3m wound healing assays after 8 h; error bars indicate SEM (n=5); panel on right shows representative photos of the wounded areas at 0 h and 8 h; UT untreated, MNP-C unconjugated MNPs, MNPs were used at 0.2 mg Fe/ml; statistical differences versus MNP-IgG. E) PC3m Boyden chamber type cell migration assays in the presence of the indicated amounts of MNP-antibodies for 24 h (n=3, ** p < 0.01, * p < 0.05 versus MNP-IgG).

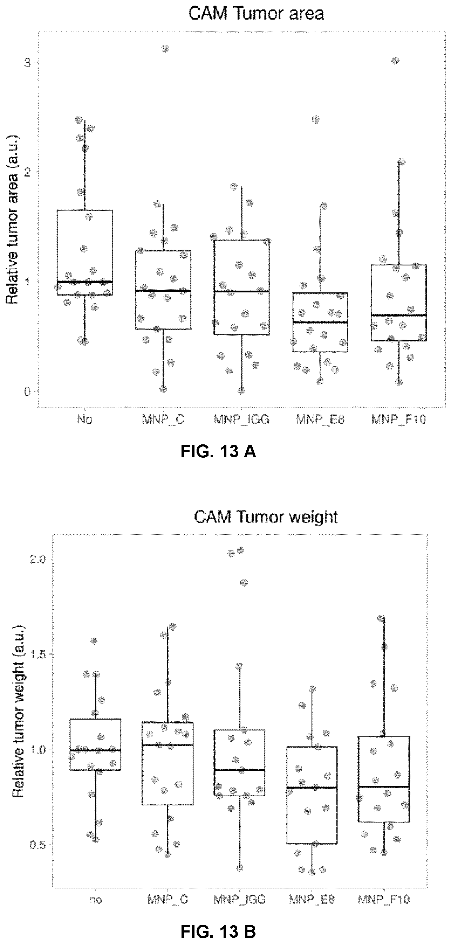

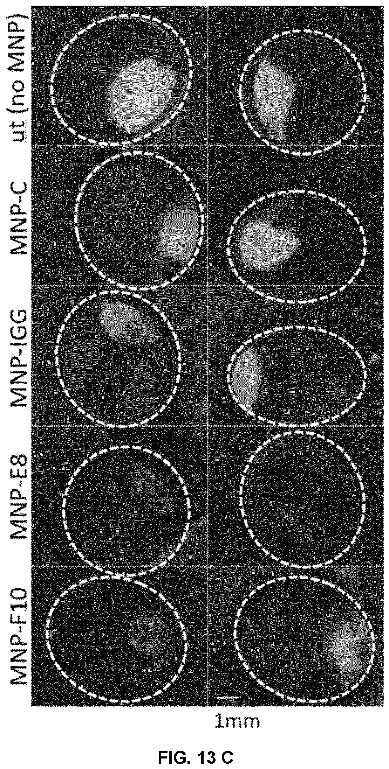

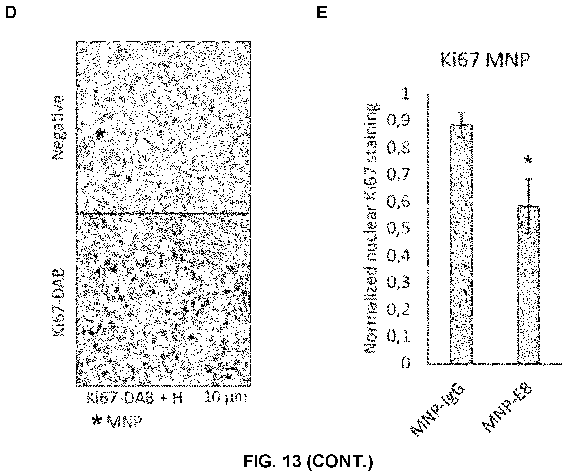

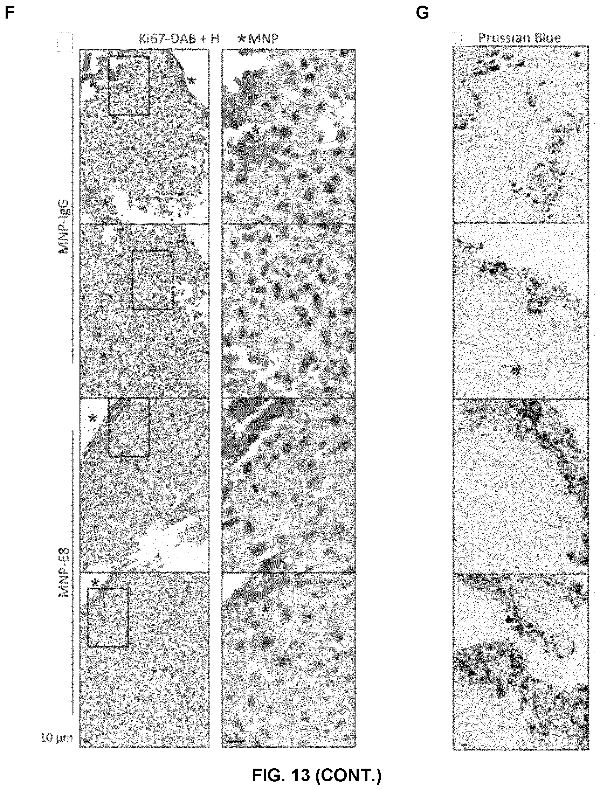

**Figure 13****.** Wnt-11 antibody nanoparticles inhibit tumor growth in chick CAM. A) Boxplots of tumor areas, relative to untreated (UT), formed by PC3-GFP cells after the indicated MNP antibody treatments B) Boxplots of tumor weights, relative to untreated (UT), formed by PC3-GFP cells after the indicated antibody MNP treatments. C) In ovo images from a representative CAM experiment showing MNP treated PC3-GFP tumors at end timepoint; MNP-C unconjugated, MNP-IgG, -E8 and -F10 conjugates for each of the antibodies. D) Example image of Ki67 stained MNP-treated PC3-GFP CAM tumor sections and negative (secondary antibody only) control, which shows some weak signal owing to the presence of MNPs highlighted by *. E) QuPath quantification of nuclear Ki67 intensity from 4 sections corresponding to 2 tumors from one CAM experiment. F) Images at low (left) and high magnification (right) of Ki67-stained specimens of PC3-GFP CAM tumors treated with MNP-IgG control (top two rows) or MNP-E8 (bottom two rows), MNPs highlighted by *. G) Prussian Blue histochemical staining (dark grey) to detect iron contained in the MNPs in the PC3-GFP CAM tumors treated with MNP-IgG control (upper two rows) or MNP-E8 (bottom two rows).

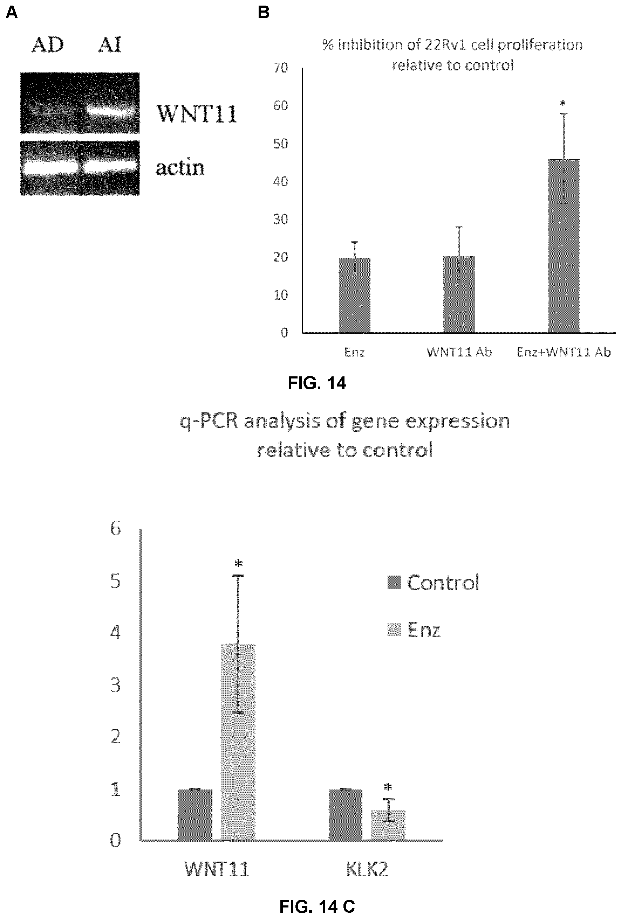

**Figure 14** Wnt-11 antibody enhances inhibition of proliferation of 22Rv1 cells by enzalutamide. A) PCR analysis of WNT11 expression in androgen-dependent (AD) and castrate-resistant (androgen-independent, AI) CWR22 prostate tumor xenografts (from Zhu et al., 2004, doi.org/10.1158/0008-5472.CAN-04-2704). B) Analysis of the inhibition of 22Rv1 cell proliferation by Wnt-11 antibody E8 (10 ug/ml) and/or 10 uM enzalutamide, relative to treatment with control IgG and carrier (DMSO), *p < 0.05 compared to single treatments, n=3. C) WNT11 gene expression in 22Rv1 cells treated with vehicle (DMSO or IgG) or 10-20 uM enzalutamide (Enz) for 48 h, *p < 0.05, versus control, n=3

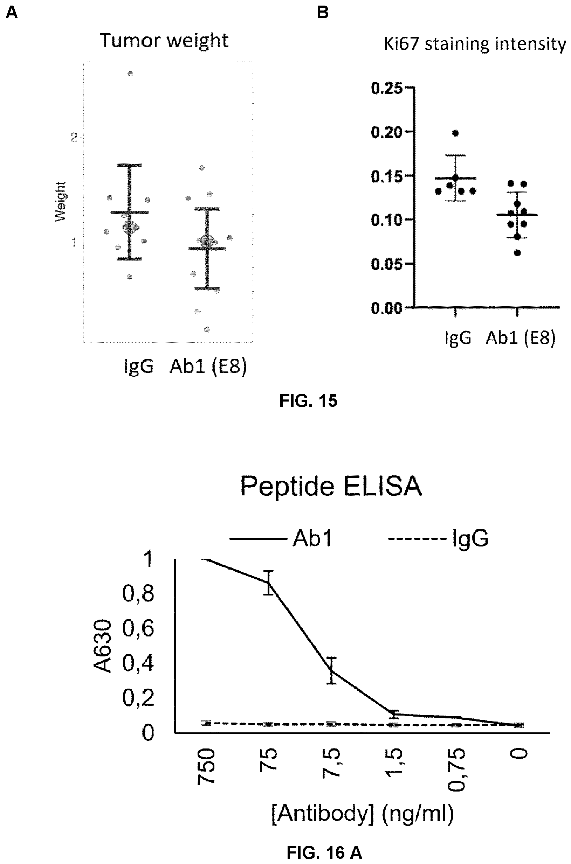

**Figure 15****.** Effects of Wnt-11 antibodies on PC3m tumor growth and metastasis (orthotopic assays in immunocompromised mice). A) Graph plotting tumor weight for mice treated with E8 (Ab1) and control IgG (p = 0.168). B) Graph plotting Ki67 staining intensities of tumors from mice treated with E8 (Ab1) and control IgG (p < 0.01).

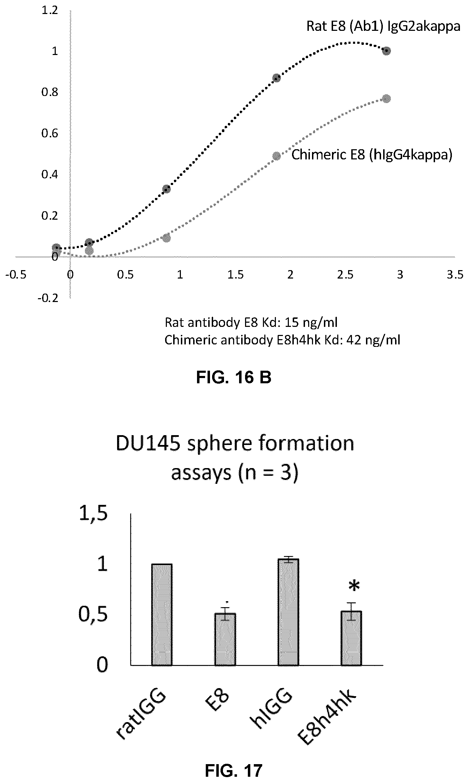

**Figure 16****.** Chimeric WNT11 antibody - peptide ELISA
**Figure 17****.** Chimeric WNT11 antibody - inhibitory activity in sphere formation assays. E8h4hk was used at 2.5-fold higher concentration than E8 based on ELISA

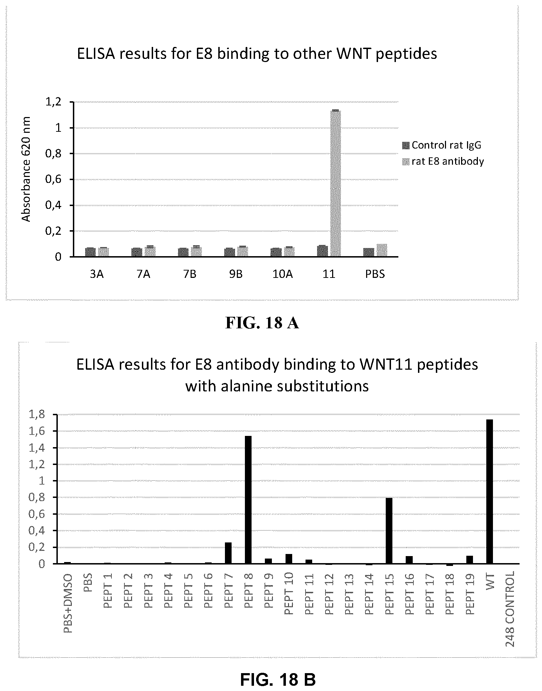

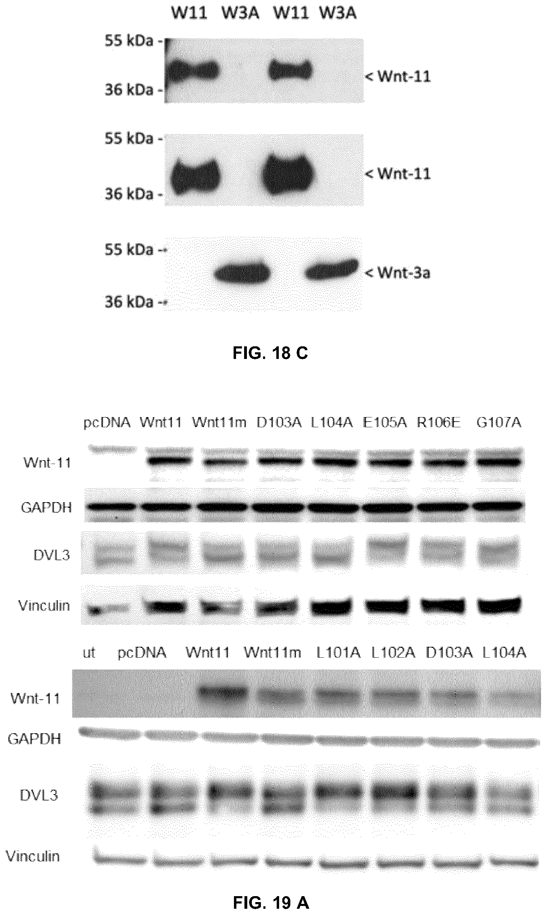

**Figure 18****.** Analysis ofWNT11 antibody epitope (Ab1, E8). A) Elisa results for E8 binding to other Wnt peptides. B) Alanine replacement abolishes E8 recognition at positions 1-6. Positions 9, 10 and 11 may play a role but are not essential or sufficient. This is consistent with E8 not recognising WNT3A, which has the same sequence (bold) at positions 8-12 (GPVLDKA**TRESA** (SEQ ID NO: 94)). C) Recombinant Wnt-11 (W11) and recombinant Wnt-3a (W3A) proteins (0.2 ug, both from R&D systems) were probed by western blotting using rat anti-Wnt-11 (top), goat anti-Wnt- 11 (middle) or mouse anti-Wnt-3a (bottom); blots representative of 3 experiments.

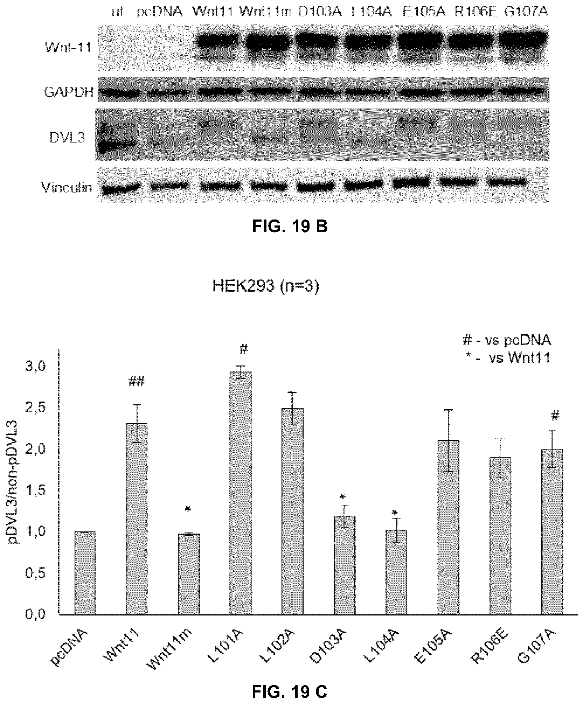

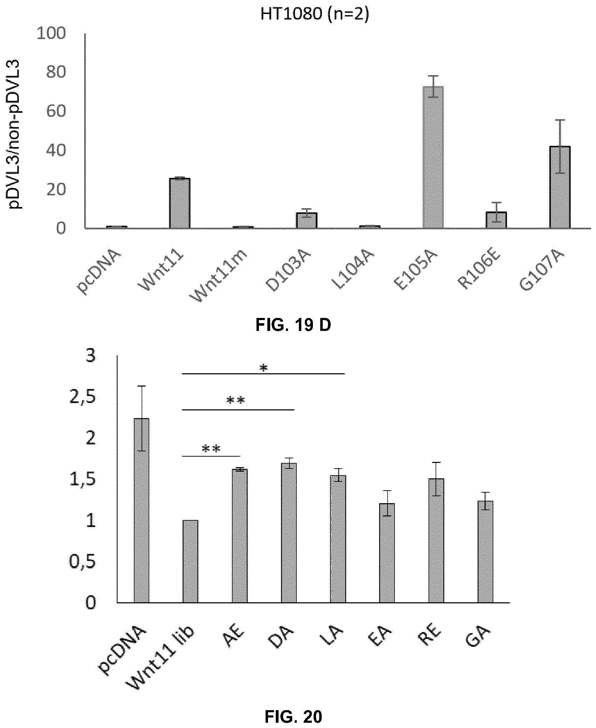

**Figure 19****.** Wnt-11 antibody epitope point mutants - DVL3 shift assays. A) Western blots of extracts from HEK293 cells transfected with plasmids expressing Wnt11, Wnt11m and Wnt11 point mutants probed using the indicated antibodies. B) Western blots of extracts from HT1080 cells transfected with plasmids expressing Wnt11, Wnt11m and Wnt11 point mutants probed using the indicated antibodies. C) Quantitation of densitometry analysis of shift in Dvl3 observed in western blots in HEK 293 cells. D) Quantitation of densitometry analysis of shift in Dvl3 observed in western blots in HT1080 cells
**Figure 20****.** Wnt-11 antibody epitope point mutants - Wnt-3a gene reporter assay. The figure shows iinhibition of WNT3A activation of TCF/beta-catenin signaling.

### DESCRIPTION OF THE INVENTION

The authors of the present invention have developed agents that block Wnt-11 function *in vitro* and *in vivo*, and thus are suitable as therapeutic agents.

In a first aspect, the invention relates to an agent binding specifically to the sequence shown in SEQ ID NO: 1, SEQ ID NO: 2 or SEQ ID NO: 3 wherein said agent is an immunoglobulin agent or a non-immunoglobulin agent selected from the group consisting of a peptide aptamer, a nucleic acid aptamer, a DARPin, an affibody, and an anticalin.

In a preferred embodiment, the agent according to the invention is an agent which binds specifically to the sequence shown in SEQ ID NO: 3. In another preferred embodiment, the agent according to the invention is an agent which binds specifically to the sequence shown in SEQ ID NO: 2. In another preferred embodiment, the agent according to the invention is an agent which binds specifically to the sequence shown in SEQ ID NO: 1.

As used herein, the terms "agent" or "binding agent" are used indistinctively and refer to a molecule with capacity of binding specifically to its cognate target and show little or no binding to other molecules. In general, it is considered that an agent has high affinity for its cognate target whereas it has low affinities for other molecules. In the context of the invention, the agent may be an immunoglobulin agent or a non-immunoglobulin agent.

As used herein, the term "immunoglobulin agent" refers to a polypeptide binding agent having a structure based on an immunoglobulin domain or fold. Proteins having the immunoglobulin domain or fold include cell surface antigen receptors, co-receptors and co-stimulatory molecules of the immune system, molecules involved in antigen presentation to lymphocytes, cell adhesion molecules, certain cytokine receptors and intracellular muscle proteins.

In a preferred embodiment the immunoglobulin agent is an antibody or an antigen-binding fragment of said antibody.

The term "antibody", as used herein, refers to a glycoprotein that exhibits specific binding activity for a particular protein, which is referred to as "antigen". The term "antibody" comprises whole monoclonal antibodies or polyclonal antibodies, or fragments thereof, and includes human antibodies, humanised antibodies, chimeric antibodies and antibodies of a non-human origin, such as murine antibodies, camelid antibodies and immunoglobulin new antigen receptor (IgNAR). "Monoclonal antibodies" are homogenous, highly specific antibody populations directed against a single site or antigenic "determinant". "Polyclonal antibodies" include heterogeneous antibody populations directed against different antigenic determinants. The antibodies may be of any isotype. The choice of isotype typically will be guided by the desired effector functions. Exemplary isotypes are IgG1, IgG2, IgG3, and IgG4.

It is well known that the basic structural unit of an antibody comprises a tetramer. Each tetramer is constituted by two identical pairs of polypeptide chains, each of which is composed by a light chain (25 kDa) and by a heavy chain (50-75 kDa). The amino-terminal region of each chain includes a variable region of about 100-110 or more amino acids, which is involved in antigen recognition. The carboxy-terminal region of each chain comprises the constant region that mediates the effector function. The variable regions of each pair of light and heavy chains form the binding site of the antibody. Therefore, an intact antibody has two binding sites. Light chains are classified as κ or λ. Heavy chains are classified as γ, µ, α, δ and ε, and they define the isotype of the antibody as respectively IgG, IgM, IgA, IgD or IgE.

The variable regions of each pair of light and heavy chains form the binding site of the antibody. They are characterized by the same general structure constituted by relatively preserved regions called frameworks (FR) joined by three hyper-variable regions called complementarity determining regions (CDR). The term "complementarity determining region" or "CDR", as used herein, refers to the region within an antibody where this protein complements an antigen's shape. Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy chain and the light chain are characterized by three CDRs, respectively CDR-H1, CDR-H2, CDR-H3 and CDR-L1, CDR-L2, CDR-L3.

The term "antibody fragment" or "an antigen-binding fragment of an antibody", as used herein, refers to a fragment of an antibody such as, for example, Fv, Fab, F(ab')2, and Fab' fragments. Various techniques have been developed for the production of antibody fragments. Traditionally, these fragments were derived via proteolytic digestion of intact antibodies but more recently these fragments can be produced directly by recombinant host cells. Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')₂ fragment that has two antigen-binding sites and is still capable of cross-linking antigen. "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind the antigen, although with lower affinity than the entire binding site. The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region.

In a more preferred embodiment, the antigen-binding fragment is selected from the group consisting of Fv, Fab, F(ab')2 and Fab'.

In another preferred embodiment, the antibody or antigen-binding fragment comprises within the heavy chain:
- a CDR comprising the amino acid sequence shown in SEQ ID NO: 4 [CDR-H1], a CDR comprising the amino acid sequence shown in SEQ ID NO: 5 [CDR-H2], and a CDR comprising the amino acid sequence shown in SEQ ID NO: 6 [CDR-H3], or a functionally equivalent variant of said CDRs,
and/or comprises within the light chain:
- a CDR comprising the amino acid sequence shown in SEQ ID NO: 7 [CDR-L1], a CDR comprising the amino acid sequence shown in SEQ ID NO: 8 [CDR-L2], and a CDR comprising the amino acid sequence shown in SEQ ID NO: 9 [CDR-L3], or a functionally equivalent variant of said CDRs
or said antibody or said antigen-binding fragment comprises within the heavy chain
- a CDR comprising the amino acid sequence shown in SEQ ID NO: 10 [CDR-H1], a CDR comprising the amino acid sequence shown in SEQ ID NO: 11 [CDR-H2], and a CDR comprising the amino acid sequence shown in SEQ ID NO: 12 [CDR-H3], or a functionally equivalent variant of said CDRs,
and/or within the light chain:
- a CDR comprising the amino acid sequence shown in SEQ ID NO: 13 [CDR-L1], a CDR comprising the amino acid sequence shown in SEQ ID NO: 14 [CDR-L2], and a CDR comprising the amino acid sequence shown in SEQ ID NO: 15 [CDR-L3], or a functionally equivalent variant of said CDRs.

In another preferred embodiment, the antibody or antigen-binding fragment comprises within the heavy chain:
- a CDR comprising the amino acid sequence shown in SEQ ID NO: 4 [CDR-H1], a CDR comprising the amino acid sequence shown in SEQ ID NO: 5 [CDR-H2], and a CDR comprising the amino acid sequence shown in SEQ ID NO: 6 [CDR-H3], or a functionally equivalent variant of said CDRs,
and comprises within the light chain:
- a CDR comprising the amino acid sequence shown in SEQ ID NO: 7 [CDR-L1], a CDR comprising the amino acid sequence shown in SEQ ID NO: 8 [CDR-L2], and a CDR comprising the amino acid sequence shown in SEQ ID NO: 9 [CDR-L3], or a functionally equivalent variant of said CDRs.

In another preferred embodiment, the antibody or antigen-binding fragment comprises within the heavy chain
- a CDR comprising the amino acid sequence shown in SEQ ID NO: 10 [CDR-H1], a CDR comprising the amino acid sequence shown in SEQ ID NO: 11 [CDR-H2], and a CDR comprising the amino acid sequence shown in SEQ ID NO: 12 [CDR-H3], or a functionally equivalent variant of said CDRs,
and within the light chain:
- a CDR comprising the amino acid sequence shown in SEQ ID NO: 13 [CDR-L1], a CDR comprising the amino acid sequence shown in SEQ ID NO: 14 [CDR-L2], and a CDR comprising the amino acid sequence shown in SEQ ID NO: 15 [CDR-L3], or a functionally equivalent variant of said CDRs.

In a preferred embodiment, the antibody or antigen-binding fragment comprises within the heavy chain the CDRs having the sequence shown in SEQ ID NO: 4, 5 and 6 and comprises within the light chain the CDRs having the sequence shown in SEQ ID NO: 7, 8 and 9. In another preferred embodiment, the antibody or antigen-binding fragment comprises within the heavy chain the CDRs having the sequence shown in SEQ ID NO: 10, 11 and 12 and comprises within the light chain the CDRs having the sequence shown in SEQ ID NO: 13, 14 and 15.

The present invention also contemplates functionally equivalent variants of the sequences of the CDRs shown in SEQ ID NO: 4 to 15, which fall within the scope of the invention.

As it is used herein, the term "functionally equivalent variant of a CDR sequence" refers to a sequence variant of a particular CDR sequence having substantially similar sequence identity with it and substantially maintaining its capacity to bind to its cognate antigen when being part of an antibody or antibody fragment as the ones described herein. For example, a functionally equivalent variant of a CDR sequence may be a polypeptide sequence derivative of said sequence comprising the addition, deletion or substitution of one or more amino acids.

Functionally equivalent variants of a CDR sequence according to the invention include CDR sequences having at least approximately 70%, at least 75%, at least 80%, at least 85%, at least 90%, at least 91%, at least 92%, at least 93%, at least 94%, at least 95%, at least 96%, at least 97%, at least 98% or at least 99% sequence identity with the corresponding amino acid sequences shown in one of SEQ ID NOs: 4 to 15. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of SEQ ID NOs: 4 to 15. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of SEQ ID NOs: 4 to 15.

The terms "identical" or "percent identity" in the context of two or more CDR sequences, peptides or polypeptides, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm incorporated into the NBLAST and XBLAST programs (Altschul et al., 1991, Nucleic Acids Res., 25:3389-3402). In certain embodiments, Gapped BLAST can be used. BLAST-2, WU-BLAST-2, ALIGN, ALIGN-2 (Genentech, South San Francisco, California) or Megalign (DNASTAR) are additional publicly available software programs that can be used to align sequences. In certain embodiments, the percent identity between two nucleotide sequences is determined using the GAP program in GCG software (e.g., using a NWSgapdna.CMP matrix and a gap weight of 40, 50, 60, 70, or 90 and a length weight of 1, 2, 3, 4, 5, or 6). In certain alternative embodiments, the GAP program in the GCG software package can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between nucleotide or amino acid sequences is determined using the algorithm of Myers and Miller (CABIOS, 4:11-17 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used. In certain embodiments, the percentage identity "X" of a first amino acid sequence to a second sequence amino acid is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the percent identity may be determined only in the region of overlap between said first and second sequences. In this case, the same formula as above can be used but using as Z value the length of the region wherein the first and second sequence overlaps, said region having a length which is substantially the same as the length of the first sequence.

As a non-limiting example, whether any particular polynucleotide has a certain percentage sequence identity (e.g., is at least 70% identical, at least 75% identical, at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2: 482 489 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of amino acids in the reference sequence are allowed.

In some embodiments, two CDR sequences, peptides or polypeptides of the invention are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 3, about 4, about 5, about 10, about 20, about 40-60 residues in length or any integral value there between, and can be over a longer region than 60-80 residues, for example, at least about 90-100 residues, and in some embodiments, the sequences are substantially identical over the full length of the sequences being compared. In a preferred embodiment, the sequence identity is determined throughout the whole length of the polypeptide of SEQ ID NO: 4 to 15 or throughout the whole length of the variant or of both.

Functionally equivalent variants of a CDR sequence according to the invention will preferably maintain at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 4 to 15 to bind to its cognate antigen when being part of an antibody or antibody fragment as the ones of the invention. This capacity to bind to its cognate antigen may be determined as a value of affinity, avidity, specificity and/or selectivity of the antibody or antibody fragment to its cognate antigen.

The antibodies Ab1 and Ab2 have the ability to inhibit Wnt-11 signalling, as shown in Fig. 3. Therefore, in a particular embodiment, functionally equivalent variants of a CDR sequence according to the invention will preferably maintain at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 4 to 15 to inhibit Wnt-11 signalling when being part of an antibody or antibody fragment as the ones of the invention. The capacity to inhibit Wnt-11 signalling can be analysed by determining reduce NT/LRP6 signaling in gene reporter and DVL shift western blotting assays, since activation of DVL by phosphorylation results in reduced electrophoretic mobility.

The antibodies Ab1 and Ab2 have the ability of blocking the binding of Wnt-11 to LRP6, as shown in Fig 5. Therefore, in a particular embodiment, functionally equivalent variants of a CDR sequence according to the invention will preferably maintain at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 4 to 15 to bind to its cognate antigen when being part of an antibody or antibody fragment as the ones of the invention and at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 4 to 15 the blocking of the binding of Wnt-11 to LRP6 when being part of an antibody or antibody fragment as the ones of the invention. By way of illustrative non-limitative example, the binding of Wnt11 to LRP6 can be determined by immunofluorescence or co-immunoprecipitation assays.

The person skilled in the art will understand that the amino acid sequences of the CDRs of the antibody or antibody fragment according to the invention can include one or more amino acid substitutions such that, even though the primary sequence of the polypeptide is altered, the capacity of the antibody to bind to the sequence shown in SEQ ID NO: 1, SEQ ID NO: 2 or SEQ ID NO: 3 is maintained. Said substitution may be a conservative substitution, which in general indicates that one amino acid is substituted with another amino acid having similar properties. For example, the substitution of glutamic acid (negatively charged amino acid) with aspartic acid would be a conservative amino acid substitution.

The capacity of the binding agents according to the invention, and in particular of the antibody or antibody fragment as described herein, to bind to the sequence shown in SEQ ID NO: 1, SEQ ID NO: 2 or SEQ ID NO: 3 can be determined by a number of assays that are well known in the art. Preferably, the binding capacity of the binding agents is determined by immunoprecipitation or by an *in vitro* binding assay, such as radioimmunoassay (RIA), enzyme-linked immunosorbent assay (ELISA), surface plasmon resonance or by immunofluorescent techniques such as immunohistochemistry (IHC), fluorescence microscopy or flow cytometry. The affinity of the binding agent of the invention for the sequence shown in SEQ ID NO: 1, SEQ ID NO: 2 or SEQ ID NO: 3 is at least 10⁻⁶ M, at least 10⁻⁷ M, at least 10⁻⁸ M, at least 10⁻⁹ M, at least 10⁻¹⁰ M, at least 10⁻¹¹, at least 10⁻¹² M, or more. In another preferred embodiment, the affinity of the binding agent of the invention for the sequence shown in SEQ ID NO: 1, SEQ ID NO: 2 or SEQ ID NO: 3 is about 5x 10⁻⁹ M, or about 5.5 x 10⁻⁹M or about 7.56 x 10⁻⁹M.

In a more preferred embodiment of antibody of the antigen-binding fragment, the heavy chain variable region of the antibody has the sequence shown in SEQ ID NO: 16 (Ab1 E8 heavy chain) or a functionally equivalent variant thereof and the light chain sequence variable region has the sequence shown in SEQ ID NO: 17 (Ab1, E8 light chain) or a functionally equivalent variant thereof, or the heavy chain variable region of the antibody has the sequence shown in SEQ ID NO: 18 (Ab2, F10 heavy chain) or a functionally equivalent variant thereof and the light chain sequence variable region has the sequence shown in SEQ ID NO: 19 (Ab2, F10 light chain) or a functionally equivalent variant thereof.

In another preferred embodiment, the heavy chain variable region of the antibody has the sequence shown in SEQ ID NO: 28.

In another preferred embodiment, the light chain variable region of the antibody has the sequence shown in SEQ ID NO: 29.

In another preferred embodiment, the heavy chain variable region of the antibody has the sequence shown in SEQ ID NO: 30.

In another preferred embodiment, the light chain variable region of the antibody has the sequence shown in SEQ ID NO: 31.

In a particular embodiment, the functionally equivalent variants of the heavy chain variable region of SEQ ID NO: 16 o SEQ ID NO: 18 and the functionally equivalent variants of the light chain variable region of SEQ ID NO: 17 or SEQ ID NO: 19 maintain the amino acid sequence of the CDRs, that is, they comprise the sequence of the CDRs included in the variable regions of SEQ ID NO: 16-19 and the amino acid substitutions, deletions or additions affect only the framework regions.

In another particular embodiment the antibody or the antigen-binding fragment is humanised.

Humanized antibodies, as used herein refers to antibodies from non-human species whose protein sequences have been modified to increase their similarity to antibody variants produced naturally in humans.

In another particular embodiment the antibody or the antigen-binding fragment is human.

By "human antibody" is meant an antibody containing entirely human light and heavy chains as well as constant regions, produced by any of the known standard methods.

In another particular embodiment the antibody or the antigen-binding fragment is murine.

By "murine antibody" is meant an antibody containing entirely murine light and heavy chains as well as constant regions, produced by any of the known standard methods. They are so called murine due to their origin from rodent hosts, more commonly mice and rats from the Muridae family. In a preferred embodiment the murine antibody is from mouse. In another preferred embodiment the murine antibody is from rat.

In another particular embodiment the antibody or antigen-binding fragment is an immunoglobulin new antigen receptor (IgNAR).

"IgNAR" or Immunoglobulin New Antigen Receptors, as used herein relates to an unconventional subset of antibodies recently identified in fish. In domain structure, IgNAR proteins are reportedly similar to other immune effector molecules, being disulphide-bonded homodimers of two polypeptide chains having five constant domains (CNARs) and one variable domain (VNAR).

In another particular embodiment the antibody or antigen-binding fragment is a camelid antibody.

Camelid antibodies, as used herein, relates to antibodies from the Camelidae family of mammals that include llamas, camels, and alpacas. The heavy chain of camelid antibodies is formed by a variable domain (VHH) and two constant domains (CH1 and CH2) but lack the constant domain CH1 usually present in conventional antibodies.

In another preferred embodiment, the antibody or antigen-binding fragment is a murine-derived monoclonal antibody, a human-murine chimeric antibody, or a humanized antibody.

Human-murine chimeric antibody, as used herein, relates to structural chimeras made by fusing variable regions from one species like a mouse or rat, with the constant regions from another species such as a human being.

In another preferred embodiment, the human-murine chimeric antibody comprises a human IgG4k constant sequence. In a more preferred embodiment, the human IgG4k constant sequence is selected from the group consisting of SEQ ID NO: 20 or SEQ ID NO: 21.

In another preferred embodiment, the human-murine chimeric antibody has the heavy chain sequence shown in SEQ ID NO: 22 and the light chain sequence shown in SEQ ID NO: 23.

In another preferred embodiment, the human-murine chimeric antibody has the heavy chain variable region sequence shown in SEQ ID NO: 16 and the light chain variable region sequence shown in SEQ ID NO: 17. In another preferred embodiment, the human-murine chimeric antibody, in particular rat-human chimeric antibody, has the heavy chain sequence shown in SEQ ID NO: 136.

In another preferred embodiment, the human-murine chimeric antibody comprises a human IgG1k constant sequence. In a more preferred embodiment, the human IgG1k constant sequence is SEQ ID No: 32 or SEQ ID No: 21.

In another preferred embodiment, the human-murine chimeric antibody, in particular rat-human chimeric antibody, has the light chain sequence shown in SEQ ID NO: 137.

In another preferred embodiment, the human-murine chimeric antibody, in particular rat-human chimeric antibody, has the heavy chain sequence shown in SEQ ID NO: 136 and the light chain sequence shown in SEQ ID NO: 137.

In another preferred embodiment the antibody is a humanized antibody.

"Humanized antibody" is understood as an antibody from a non-human organism, typically a murine antibody, which conserves the antigen binding properties of the parent antibody, but which is less immunogenic in human beings. This can be achieved by means of different processes, which include (a) grafting the complete nonhuman variable domains into human constant regions to generate chimeric antibodies; (b) grafting only the nonhuman complementarity determining regions (CDR) in a human framework and the constant regions, with or without retaining the critical framework residues; and (c) transplanting the complete nonhuman variable domains, but "concealing them" with a section similar to the human variable domain by means of replacing the surface residues.

In a more preferred embodiment, the humanized antibody has the heavy chain variable region shown in SEQ ID NO: 26. In another preferred embodiment, the humanized antibody has the heavy chain variable region shown in SEQ ID NO: 33. In another preferred embodiment, the humanized antibody has the heavy chain variable region shown in SEQ ID NO: 27. In another preferred embodiment, the humanized antibody has the heavy chain variable region shown in SEQ ID NO: 34. In another preferred embodiment, the humanized antibody has the heavy chain variable region shown in SEQ ID NO: 138.

In another preferred embodiment, humanized antibody has the light chain variable region sequence shown in SEQ ID NO: 110. In another preferred embodiment, the humanized antibody has the light chain variable region shown in SEQ ID NO: 111. In another preferred embodiment, the humanized antibody has the light chain variable region shown in SEQ ID NO: 112. In another preferred embodiment, the humanized antibody has the light chain variable region shown in SEQ ID NO: 113. In another preferred embodiment, the humanized antibody has the light chain variable region shown in SEQ ID NO: 139.

In another preferred embodiment, the humanized antibody has the heavy chain variable region shown in SEQ ID NO: 26 and the the light chain variable region sequence shown in SEQ ID NO: 110. In another preferred embodiment, the humanized antibody has the heavy chain variable region shown in SEQ ID NO: 27 and the the light chain variable region sequence shown in SEQ ID NO: 111. In another preferred embodiment, the humanized antibody has the heavy chain variable region shown in SEQ ID NO: 33 and the the light chain variable region sequence shown in SEQ ID NO: 112. In another preferred embodiment, the humanized antibody has the heavy chain variable region shown in SEQ ID NO: 34 and the the light chain variable region sequence shown in SEQ ID NO: 113.

In another preferred embodiment, the humanized antibody has the heavy chain sequence shown in SEQ ID NO: 138 and the light chain sequence shown in SEQ ID NO: 139.

In another preferred embodiment, the agent binding specifically to the sequence shown in SEQ ID NO: 1, SEQ ID NO: 2 or SEQ ID NO: 3 is a non-immunoglobulin agent selected from the group consisting of a peptide aptamer, a nucleic acid aptamer, a DARPin, an affibody, and an anticalin.

As used herein, the term "non-immunoglobulin agent" refers to binding agents other than immunoglobulins that are based on different molecular natures, topologies, or scaffolds. The term scaffold is meant to describe a protein framework that can carry altered amino acids or sequence insertions that confer on protein variants different functions, usually for binding specific targets. Examples of such non-immunoglobulin agents are well known in the art, and include without limitation peptide aptamers, nucleic acid aptamers, DARPins, affibodies, and anticalins.

DARPins, affibodies, anticalins, and other protein scaffolds are reviewed in Binz et al., 2005 (Nat. Biotech. 23:1257-68). The term "peptide aptamer" refers to a short variable peptide domain that is attached at both ends to a protein scaffold, and that binds to a specific target molecule. The variable loop length is typically composed of ten to twenty amino acids, and the scaffold may be any protein which has good solubility and compacity properties. Currently, the bacterial protein Thioredoxin-A is the most used scaffold protein. The term "nucleic acid aptamer" or "DNA aptamer", as used herein, refers to a short strand of DNA that has been engineered through repeated rounds of selection to bind to specific molecular targets.

The term "nucleic acid", as used herein, refers to polymers formed by the repetition of monomers called nucleotides linked by phosphodiester bonds. The term includes both DNA and RNA.

The term "linear peptide", as used herein, refers to a peptide comprising between 3 and 20 amino acids, having amino and carboxy-terminal free ends and being linear.

The term "cyclic peptide", as used herein, refers to refers to a peptide comprising between 3 and 20 amino acids, and being constrained by cyclisation at either the backbone or a side chain of the peptide.

The term "branched peptide", as used herein, refers to a peptide comprising between 3 and 20 amino acids, and at least an isopeptide bond. An "isopeptide bond" is an amide bond that is not present on the main chain of a peptide, and thereby forms an additional "branched" peptidic chain.

The term "DARPins" or designed ankyrin repeat proteins, as used herein relates to genetically engineered antibody mimetic proteins typically exhibiting highly specific and high-affinity target protein binding.

The term "affibodies", as used herein relates to small, robust proteins engineered to bind to a large number of target proteins or peptides with high affinity, imitating monoclonal antibodies, and are therefore a member of the family of antibody mimetics.

The term "anticalins", as used herein, relates to artificial proteins that are able to bind to antigens, either to proteins or to small molecules. They are derived from human lipocalins which are a family of naturally binding proteins. Anticalin proteins are being used in lieu of monoclonal antibodies but are about eight times smaller with a size of about 180 amino acids and a mass of about 20 kDa.

In a second aspect, the invention relates to an antibody construct comprising the antigen-binding fragment according to the invention, wherein the antibody construct is selected from the group consisting of scFv, scFv-Fc, minibody, (scFv)₂ and diabody.

The term "antibody construct", as used herein, refers to constructs based on antibody binding domains that are typically generated by genetic engineering techniques. Examples of antibody constructs include scFv, scFv-Fc, minibody, (scFv)2 and diabody. These and other antibody constructs are reviewed in Cuesta et al., 2010 (Trends Biotechnol. 28:355-62).

"scFv", as used herein relates to a fusion protein of the variable regions of the heavy (VH) and light chains (VL) of immunoglobulins, connected with a short linker peptide of ten to about 25 amino acids.

"scFv-Fc" consists of an scFv connected to the CH2 and CH3 regions of an IgG constant region, which can then create a homodimer with another copy of itself.

"minibody" is a class of bispecific fragments, scFv-derived bispecific molecules. It is a bivalent fusion molecule with two scFvs fused to CH3.

"scFv29" refers to a monomeric scFv that associates into a stable high-affinity noncovalent dimer.

"diabody" is a dimer of scFv which consists of VH and VL domains connected by a short peptide linker.

Amino acid sequence modification(s) of the binding agents and antibody constructs described herein in positions other than the CDRs or the binding sites are also contemplated. For example, it may be desirable to improve the binding affinity and/or other biological properties of the binding agent. Amino acid sequence variants of the binding agent are prepared by introducing appropriate nucleotide changes into the antibody encoding nucleic acid, or by peptide synthesis. Such modifications include, for example, deletions from, and/or insertions into and/or substitutions of, residues within the amino acid sequences of the binding agent. Any combination of deletion, insertion, and/or substitution is made, provided that the final binding agent possesses the desired characteristics. The amino acid changes may also alter post-translational processes of the protein, such as changing the number or position of glycosylation sites. Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or the antibody polypeptidic chain fused to a cytotoxic polypeptide. Other insertional variants of the molecule include the fusion to the N- or C-terminus of an enzyme, or a polypeptide which increases its serum half-life.

In the particular embodiment of the binding agent being an antibody, another type of amino acid variant of the antibody alters its original glycosylation pattern. By altering is meant deleting one or more carbohydrate moieties found in the molecule, and/or adding one or more glycosylation sites that are not present in it. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of any of these tripeptide sequences in a polypeptide creates a potential glycosylation site. O-linked glycosylation refers to the attachment of one of the monosaccharides or monosaccharide derivatives N-acetylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites). Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

Also, it may be desirable to modify the antibodies described herein in order to improve their effector function, e.g., so as to enhance function blocking of Wnt-11. This may be achieved by introducing one or more amino acid substitutions in an Fc region of an antibody. Glycosyl groups added to the amino acid backbone of glycoproteins e.g., antibodies are formed by several monosaccharides or monosaccharide derivatives in resulting in a composition which can be different in the same antibody produced in cell from different mammals or tissues. It is possible to improve those properties by mean of studying the pattern of glycosylation of antibodies from different sources.

Other modifications suitable for the antibodies described herein include the introduction of cysteine residue(s) in the Fc region, thereby allowing interchain disulfide bond formation in this region to improve the internalisation capability and/or increase blocking of Wnt-11 function and binding to LRP6. The blocking function and binding to LRP6 can be determined by any method known in the art. By way of illustrative non-limitative example, the binding of Wnt to LRP6 is determined by immunofluorescence or co-immunoprecipitation assays. As illustrative non-limitative example blocking Wnt-11 function can be determine by determining reduced WNT/LRP6 signaling in gene reporter and DVL shift western blotting assays, since activation of DVL by phosphorylation results in reduced electrophoretic mobility.

In order to increase the serum half-life of the binding agent, one may incorporate a salvage receptor binding epitope into the agent. As used herein, the term "salvage receptor binding epitope" refers to an epitope of the Fc region of an IgG molecule (e.g., IgG1, IgG2, IgG3, or lgG4) that is responsible for increasing the in vivo serum half-life of the IgG molecule.

In another aspect, the invention relates to a combination comprising:
a) an agent binding to the sequence shown in SEQ ID NO: 1, SEQ ID NO: 2 or SEQ ID NO: 3 according to the invention or an antibody construct according to the invention, and
b) an anti-androgen compound.

"Combination" or "composition", as used herein, indicates that the agent binding to the sequence shown in SEQ ID NO: 1 , SEQ ID NO: 2 or SEQ ID NO: 3 according to the invention or an antibody construct according to the invention and the anti-androgen compound can be formulated in the same medicine formulation or they can also be formulated in different ones, but different medicine formulation shall be included in the same drug through combined package and be used at the same time, in sequence or in turn.

"Anti-androgen compound", as used herein, relates to compounds also known as androgen antagonists or testosterone blockers, and are a class of drugs that prevent androgens like testosterone and dihydrotestosterone (DHT) from mediating their biological effects in the body. The anti-androgen compound can be steroidal or non-steroidal. In a preferred embodiment, the anti-androgen compound is non-steroidal.

Suitable, illustrative examples of anti-androgen compounds are **17α-**Hydroxyprogesterone derivatives, such as Chlormadinone acetate, Cyproterone acetate, Megestrol acetate or Osaterone acetate; 19-Norprogesterone derivatives such as Nomegestrol acetate, 19-Nortestosterone derivatives such as Dienogest or Oxendolone, 17α-Spirolactone derivatives such as Drospirenone or Spironolactone or other compounds suchs as Bicalutamide, Flutamide, Nilutamide, apalutamide, Darolutamide, Enzalutamide, Proxalutamide, cimetidine or Topilutamide.

In a preferred embodiment, the anti-androgen compound is enzalutamide.

Enzalutamide, as used herein relates to the compound having the CAS number 915087-33-1 also known as MDV3100, MDV-3100 or 4-(3-(4-cyano-3-(trifluoromethyl)phenyl)-5,5-dimethyl-4-oxo-2-thioxoimidazolidin-1-yl)-2-fluoro-N-methylbenzamide.

In a third aspect, the invention relates to a nucleic acid selected form the group consisting of:
a) a nucleic acid encoding the agent according to the invention wherein the agent is a protein compound, or the antibody construct according to the invention and
b) a complementary nucleic acid of a nucleic acid as defined in a).

Said nucleic acid of the invention can contain a regulatory sequence operatively linked for the expression of the nucleotide sequence encoding the binding agent or the antibody construct of the invention, thereby forming a gene construct, hereinafter the "gene construct of the invention". As used herein, the term "operatively linked" means that the binding agent or antibody construct encoded by the nucleic acid sequence of the invention is expressed in the correct reading frame under control of the expression control or regulating sequences.

In a fourth aspect, the invention relates to an expression cassette comprising the nucleic acid according to the invention.

In a preferred embodiment, the expression cassette of the invention comprises the nucleic acid according to the invention operatively linked to an expression control sequence. The gene construct of the invention can be obtained through the use of techniques widely known in the prior art.

Control sequences are sequences that control and regulate transcription and, where appropriate, the translation of said antibody, and include promoter sequences, transcriptional regulators encoding sequences, ribosome binding sequences (RBS) and/or transcription terminating sequences. The expression cassette of the present invention may additionally include an enhancer, which may be adjacent to or distant from the promoter sequence and can function to increase transcription from the same. In a particular embodiment, said expression control sequence is functional in prokaryotic cells and organisms, such as bacteria, etc. Whereas in another particular embodiment, said expression control sequence is functional in eukaryotic cells and organisms, for example, insect cells, plant cells, mammalian cells, etc.

Any available promoter can be used in this methodology. In a preferred embodiment of the present invention, the promoter used in the nucleic acid construct of the present invention is active in the specific cell population to be transfected. Illustrative, non-limiting examples of ubiquitous promoters which can be present in the expression cassette of the invention include the human cytomegalovirus promoter (hCMV), SV40 promoter, the EF1-alpha promoter to, and the ubiquitin promoter C. Illustrative, non-limiting examples of cell-type specific promoters and/or tissue specific promoters such as albumin include which is specific for liver, lymphoid-specific promoters, and so on.

Advantageously, the expression cassette of the invention further comprises a marker or gene encoding a motif or phenotype which allows selecting the transformed host cell with said expression cassette. Illustrative examples of said markers that could be present in the expression cassette of the invention include antibiotic resistance genes, genes for resistance to toxic compounds, and in general, all those that allow selecting the genetically transformed cells. Alternatively, the expression cassette can further comprise another sequence, such as such as a secretory leader sequence or a sequence being used for purification (for example, His tag) or for detection (for example, Sv5 epitope tag)

The gene constructs of the invention or the expression cassette of the invention can be inserted into appropriate vectors. Thus, in another aspect, the invention relates to a vector, such as an expression vector, hereinafter "the vector of the invention", comprising said gene constructs or said expression cassettes of the invention. The choice of vector depends on the host cell in which it will be subsequently introduced. As an example, the vector into which is inserted the said nucleic acid sequences may be a plasmid or a vector which, when introduced into a host cell, is integrated or not in the genome of said cell. Obtaining this vector can be performed by conventional methods known to those skilled in the art. In a particular embodiment, said recombinant vector is a vector useful to transfect animal cells.

In a fifth aspect, the invention relates to a vector comprising the nucleic acid according to the invention or the expression cassette according to the invention.

Said vector can be used to transform, transfect, or infect cells susceptible of being transformed, transfected, or infected by said vector. Such cells can be prokaryotic or eukaryotic. Therefore, in another aspect, the invention relates to a cell, hereinafter "the cell of the invention", comprising the nucleic acid or the expression cassette or the vector according to the invention. In order to obtain the cell of the invention, the cell may need to be transformed, transfected, or infected with the vector of the invention. Said transformed cell, transfected, or infected comprises, therefore, a nucleic acid of the invention, a gene construct of the invention or an expression cassette or vector of the invention.

Transformed cells, transfected, or infected may be obtained by conventional methods known to those skilled in the art. Cells suitable for performing the invention include, without limitation, mammalian, plant, insect, fungal and bacterial cells. Bacterial cells include, without limitation, cells from Gram positive bacteria such as species of the genus *Bacillus*, *Streptomyces* and *Staphylococcus* and Gram-negative bacterial cells such as cells of the genus *Escherichia* and *Pseudomonas.* Fungal cells preferably include yeast cells such as *Saccharomyces*, *Pichia pastoris* and *Hansenula polymorpha.* Insect cells include, without limitation, *Drosophila* cells and Sf9 cells. Plant cells include, among others, cells of crop plants such as cereals, medicinal, ornamental or bulbs. Mammalian cells suitable for the present invention include epithelial cell lines, osteosarcoma cell lines, neuroblastoma cell lines, epithelial carcinomas, glial cells, hepatic cell lines, CHO cells, COS cells, BHK cells, HeLa cells, 911 cells, AT1080 cells, A549 cells, HEK 293 and 293T cells, PER.C6 cells, NTERA-2 human ECCs cells, D3 cells of the mESCs line, human embryonic stem cells such as HS293, hMSCs and BGV01, SHEF1, SHEF2 and HS181, NIH3T3 cells, REH and MCF-7 cells.

In a seventh aspect, the invention relates to a nanoparticle comprising the agent according to the invention or the antibody construct according to the invention.

As used herein, a "nanoparticle" is a colloidal, polymeric, or elemental particle ranging in size from about 1 nm to about 1000 nm. In another aspect, the nanoparticle has a size of 10-100 nm, preferably higher than 50 nm and lower than 200 nm.

"Size", as used herein means the diameter of the particle if the particle is spherical or, if the particle is non-spherical, the volume-based particle size. The volume- based particle size is the diameter of the sphere that has the same volume as the non-spherical particle in question. The values of particle size obtained by the techniques known in the art are in agreement within the experimental error.

The size of the particle can be determined by any method known in the art. As a way of illustrative non-imitative examples, the size of the particle can be determined by sieves, sedimentation, electrozone testing, laser diffraction, flow cytometer, microscopy such as scanning or transmission electron microscopy (SEM or TEM), spectroscopic techniques such as Dynamic Light Scattering or UV-vis spectroscopy, or Eyecon2^{™}. In a preferred embodiment,

In a preferred embodiment, the size of the nanoparticle refers to a mean size. The mean particle size of various types of particles may differ from the particle size distribution of the particles. For example, a particle can have a mean particle size of about 5-200 nm, preferably 5-100 nm, more preferably 5-60 nm, more preferably 5-50 nm, more preferably 10-50 nm. In another preferred embodiment, the mean particle size is around 10.21 nm or around 92 nm while its hydrodynamic diameter was around 150-180 nm, more preferably 155-170 nm, more preferably 160 nm, more preferably 161.5 ± 2.12 nm (It should be understood that the particle sizes that are described herein are for particles when they are dispersed in an aqueous medium and the mean particle size is based on the mean of the particle number distribution).

The nanoparticles may contribute to preserve the integrity of the polypeptide in the biological fluids until it reaches the target organ. In addition, nanoparticles can also be modified so as to include moieties which allow the targeting of the nanoparticle to an organ of interest.

Nanoparticles can be made up of silica, carbohydrate, lipid, metals, or polymer molecules. The agent according to the invention or the antibody construct according to the invention can be either embedded in the nanoparticle matrix or may be adsorbed onto its surface. In one example, the nanoparticle may be made up of a biodegradable polymer such as poly(butylcyanoacrylate) (PBCA). Examples of elemental nanoparticles include carbon nanoparticles and iron oxide nanoparticles, which can then be coated with oleic acid (OA)-Pluronic(R). In this approach, a drug (e.g., a hydrophobic or water insoluble drug) is loaded into the nanoparticle. Other nanoparticles are made of silica.

Nanoparticles can be formed from any useful polymer. Examples of polymers include biodegradable polymers, such as poly(butyl cyanoacrylate), poly(lactide), poly(glycolide), poly-s-caprolactone, poly(butylene succinate), poly(ethylene succinate), and poly(p-dioxanone); poly(ethyleneglycol); poly-2-hydroxyethylmethacrylate (poly(HEMA)); copolymers, such as poly(lactide-co-glycolide), poly(lactide)-poly(ethyleneglycol), poly(poly(ethyleneglycol)cyanoacrylate-cohexadecylcyanoacrylate, and poly [HEMA-co-methacrylic acid]; proteins, such as fibrinogen, collagen, gelatin, and elastin; and polysaccharides, such as amylopectin, a-amylose, and chitosan.

Other nanoparticles include solid lipid nanoparticles (SLN). Examples of lipid molecules for solid lipid nanoparticles include stearic acid and modified stearic acid, such as stearic acid-PEG 2000; soybean lecithin; and emulsifying wax. Solid lipid nanoparticles can optionally include other components, including surfactants, such as Epicuron(R) 200, poloxamer 188 (Pluronic(R) F68), Brij 72, Brij 78, polysorbate 80 (Tween 80); and salts, such as taurocholate sodium. Agents can be introduced into solid lipid nanoparticles by a number of methods discussed for liposomes, where such methods can further include high-pressure homogenization, and dispersion of microemulsions.

Nanoparticles can also include nanometer-sized micelles. Micelles can be formed from any polymers described herein. Exemplary polymers for forming micelles include block copolymers, such as poly(ethylene glycol) and poly(ε-caprolactone). (e.g., a PEO- b-PCL block copolymer including a polymer of ε-caprolactone and α-methoxy- ω-hydroxy-poly(ethylene glycol)).

In certain embodiments, the properties of the nanoparticles are altered by coating with a surfactant. Any biocompatible surfactant may be used, for example, polysorbate surfactants, such as polysorbate 20, 40, 60, and 80 (Tween 80); Epicuron(R) 200; poloxamer surfactants, such as 188 (Pluronic(R) F68) poloxamer 908 and 1508; and Brij surfactants, such as Brij 72 and Brij 78.

In another preferred embodiment, the nanoparticle is a magnetic nanoparticle. Magnetic nanoparticles (MNPs) are one class of NPs, the core of which can be formed by pure or mixed metals (Fe, Co, Ni, and others), their alloys, and oxides. Iron oxide nanoparticles can be conveniently coated with various compounds, such as polymers (e.g., polyethylene glycol (PEG), chitosan) and dendrimers, and they can also be encapsulated in silane or gold shells. This is in order to improve the properties of the nanomaterials, such as their stability, interaction with biological molecules, and bioconjugation capabilities. In another preferred embodiment, the magnetic nanoparticle has a core of 1-20 nm, preferably 5-15 nm and a shell with hydrodynamic radios of about 10-180 nm, preferably 20-150 nm.

In a preferred embodiment, the nanoparticle is a magnetic nanoparticle, more preferably superparamagnetic iron oxide nanoparticles (SPIONs). In another preferred embodiment, the superparamagnetic iron oxide nanoparticles (SPIONs) are functionalized with COOH (Micromod, 79-02-201) or (Polyethylene glycol) PEG-COOH (Micromod, 79-56-201) to allow the binding of the agent according to the invention or the antibody construct according to the invention. In another preferred embodiment, the agent according to the invention or the antibody construct according to the invention comprises a linker such as Sulfosuccinimidyl-4-(N-maleimidomethyl) cyclohexane-1-carboxylate (Sulfo-SMCC) linker (ProteoChem c1109) to allow the binding to the nanoparticle.

In another preferred embodiment, the nanoparticles of the invention have superparamagnetic properties.

"Superparamagnetism" is a form of magnetism exhibited by small ferromagnetic or ferrimagnetic nanoparticles. At sizes of less than a hundred nanometres, the nanoparticles are single-domain particles, allowing the magnetization of the nanoparticles to be approximated as one giant magnetic moment by summing the individual magnetic moments of each constituent atom.

In a more preferred embodiment, the nanoparticles of the invention are superparamagnetic iron oxide nanoparticles, more particularly SPIONs) nanomag^{®}-D-spio.

Nanoparticles can optionally be modified to include hydrophilic polymer groups (e.g., poly(ethyleneglycol) or poly(propyleneglycol)), for example, by covalently attaching hydrophilic polymer groups to the surface or by using polymers that contain such hydrophilic polymer groups (e.g., poly[methoxy poly (ethyleneglycol) cyanoacrylate-co-hexadecyl cyanoacrylate]). Nanoparticles can be optionally cross linked, which can be particularly useful for protein-based nanoparticles.

Targeted delivery can be achieved by the addition of ligands without compromising the ability of nanoparticles to deliver the agent according to the invention or the antibody construct according to the invention. It is contemplated that this will enable delivery to specific cells, tissues and organs. The targeting specificity of the ligand-based delivery systems are based on the distribution of the ligand receptors on different cell types. The targeting ligand may either be noncovalently or covalently associated with a nanoparticle and can be conjugated to the nanoparticles by a variety of methods as discussed herein.

### Pharmaceutical compositions

In an eighth aspect, the invention relates to a pharmaceutical composition comprising a therapeutically effective amount of an agent according to the invention, an antibody construct according to the invention, a combination according to the invention or a nanoparticle according to the invention, together with a pharmaceutically acceptable excipient or carrier.

The term "pharmaceutical composition", as used herein, refers to a composition comprising a therapeutically effective amount of the agent according to the present invention, an antibody construct according to the invention or a nanoparticle according to the invention and at least one pharmaceutically acceptable excipient.

The term "therapeutically effective amount", as used herein, refers to the amount of the agent according to the present invention, an antibody construct according to the invention or a nanoparticle according to the invention which is required to achieve an appreciable prevention, cure, delay, reduction of the severity of, or amelioration of one or more symptoms of the disease or condition, such as cancer or fibrosis. The person skilled in the art will be able to determine therapeutically effective amounts of these molecules without undue experimentation by means of conventional techniques well-known in the art.

The terms "pharmaceutically acceptable excipient" or "pharmaceutically acceptable carrier," refer to any compound or combination of compounds that is essentially nontoxic to the subject at the dosage and concentration employed, and is compatible with the other components of a pharmaceutical composition. This includes solvents, dispersion media, coatings, antibacterial and antifungal agents, isotonic and absorption delaying agents, and the like. Thus, an excipient is an inactive substance formulated alongside the active ingredient of a pharmaceutical composition, for the purpose of bulking-up compositions that contain said active ingredients. Bulking up allows convenient and accurate dispensation of a drug substance when producing a dosage form. Excipients also can serve various therapeutic-enhancing purposes, such as facilitating compound (drug) absorption or solubility, or other pharmacokinetic considerations. Excipients can also be useful in the manufacturing process, to aid in the handling of the active substance concerned such as by facilitating powder flowability or non-stick properties, in addition to aiding in vitro stability such as prevention of denaturation over the expected shelf life. The selection of appropriate excipients depends upon the route of administration and the dosage form, as well as the active ingredient and other factors. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn-protein complexes); and/or non-ionic surfactants such as TWEENTM, PLURONICSTM or polyethylene glycol (PEG).

In a particular embodiment, the route of administration of the pharmaceutical composition of the invention is intravenous, intratumoral or parenteral. In a more preferred embodiment, the administration is intravenous. Pharmaceutical compositions according to the invention can be prepared, for instance, as injectables such as liquid solutions, suspensions, and emulsions.

In a particular embodiment, the pharmaceutical composition containing the agent according to the present invention, an antibody construct according to the invention, a combination of the invention or a nanoparticle according to the invention is a pharmaceutical composition for parenteral administration. The term "parenteral" as used herein includes intravenous, intraperitoneal, intramuscular, subcutaneous, rectal, or vaginal administration. The intravenous form of parenteral administration is generally preferred. In addition, the pharmaceutical composition of the invention may suitably be administered by pulse infusion, e.g., with declining doses. Preferably the dosing is given by injections, most preferably intravenous or subcutaneous injections, depending in part on whether the administration is brief or chronic. The pharmaceutical composition suitable for parenteral injection, include physiologically acceptable sterile aqueous or non-aqueous solutions, dispersions, suspensions, or emulsions, or may comprise sterile powders for reconstitution into sterile injectable solutions or dispersions. Examples of suitable aqueous or non-aqueous excipients or carriers, diluents, solvents, or vehicles include water, ethanol, polyols (propylene glycol, polyethylene glycol, glycerol, and the like), suitable mixtures thereof, triglycerides, including vegetable oils such as olive oil, or injectable organic esters such as ethyl oleate. In a particular embodiment, the pharmaceutical composition containing the agent according to the present invention, an antibody construct according to the invention or a nanoparticle according to the invention for use in the present invention is a pharmaceutical composition for intravenous, intramuscular, or subcutaneous administration. Typically, pharmaceutical compositions for intravenous, intramuscular, or subcutaneous administration are solutions in sterile isotonic aqueous buffer. If necessary, the pharmaceutical composition including the compound for use according to the invention also includes a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachet indicating the quantity of active ingredient. Where the pharmaceutical composition is to be administered by infusion, it can be dispensed with an infusion bottle containing sterile pharmaceutical grade water or saline. Where the pharmaceutical composition is administered by injection, an ampule of sterile water for injection or saline can be provided so that the ingredients may be mixed prior to administration.

In another particular embodiment, the pharmaceutical composition containing the agent according to the present invention, an antibody construct according to the invention, the combination of the invention or a nanoparticle according to the invention is a pharmaceutical composition for oral administration.

Solid dosage forms for oral administration include conventional capsules, sustained release capsules, conventional tablets, sustained-release tablets, chewable tablets, sublingual tablets, effervescent tablets, pills, suspensions, powders, granules, and gels. In the solid dosage forms, the active ingredient (the agent according to the present invention, an antibody construct according to the invention or a nanoparticle according to the invention) is admixed with at least one suitable excipient or carrier, such as sodium citrate or dicalcium phosphate or (a) fillers or extenders, such as for example, starches, lactose, sucrose, mannitol, or silicic acid; (b) binders, such as for example, carboxymethylcellulose, alginates, gelatin, polyvinylpyrrolidone, sucrose, or acacia; (c) humectants, such as for example, glycerol; (d) disintegrating agents, such as for example, agar-agar, calcium carbonate, potato or tapioca starch, alginic acid, certain complex silicates, or sodium carbonate; (e) solution retarding agents, such as for example, paraffin; (f) absorption accelerators, such as for example, quaternary ammonium compounds; (g) wetting agents, such as for example, cetyl alcohol or glycerol monostearate; (h) adsorbents, such as for example, kaolin or bentonite; and/or (i) lubricants, as for example, talc, calcium stearate, magnesium stearate, solid polyethylene glycols, sodium lauryl sulfate, or mixtures thereof. In the case of capsules and tablets, the dosage forms may also comprise buffering agents.

Solid formulations of a similar type may also be used as fillers in soft or hard filled gelatin capsules using excipients such as lactose or milk sugar, as well as high molecular weight polyethylene glycols, and the like. Solid dosage forms such as coated tablets, capsules and granules can be prepared with coatings or shells, such as enteric coatings and others known in the art. They may also contain opacifying agents and can be formulated such that they release the active ingredient or ingredients in a delayed manner. Examples of embedding formulations that can be used are polymeric substances and waxes. The active ingredients can also be in micro-encapsulated form, if appropriate, with one or more of the aforementioned excipients.

Liquid dosage forms for oral administration include pharmaceutically acceptable emulsions, solutions, suspensions, syrups and elixirs containing suitable excipients or carriers used in the art. In addition to the active ingredient (the agent according to the present invention, an antibody construct according to the invention or a nanoparticle according to the invention) the liquid dosage form may contain one or more excipients or carriers commonly used in the art, such as water or other solvents, solubilizing agents and emulsifiers, such as for example, ethyl alcohol, isopropyl alcohol, ethyl carbonate, ethyl acetate, benzyl alcohol, benzyl benzoate, propylene glycol, 1,3-butylene glycol, dimethylformamide, oils, particular cottonseed oil, groundnut oil, corn germ oil, olive oil, castor oil, sesame seed oil, Miglyol^{®}, glycerol, tetrahydrofurfuryl alcohol, polyethylene glycols, fatty acid esters of sorbitan, or mixtures of these substances, and the like. In addition to said inert diluents, the formulation can also include adjuvants, such as wetting agents, emulsifying and suspending agents, sweetening agents, flavoring agents, and perfuming agents. Suspensions, in addition to the active ingredient or ingredients, may contain suspending agents, as for example, ethoxylated isostearyl alcohols, polyoxyethylene sorbitol or sorbitan esters, microcrystalline cellulose, aluminum metahydroxide, bentonite, agar-agar, or tragacanth, or mixtures of these substances, and the like.

In another particular embodiment, the pharmaceutical composition containing the agent according to the present invention, an antibody construct according to the invention, the combination of the invention or a nanoparticle according to the invention is a pharmaceutical composition for topical administration. For topical administration, said pharmaceutical composition can be formulated as a cream, gel, lotion, liquid, pomade, spray solution, dispersion, solid bar, emulsion, microemulsion and the like which may be formulated according to conventional methods that use suitable excipients, such as, for example, emulsifiers, surfactants, thickening agents, coloring agents and combinations of two or more thereof.

Several drug delivery systems are known and can be used to administer the agent according to the present invention, an antibody construct according to the invention or a nanoparticle according to the invention, including, for example, encapsulation in liposomes, microbubbles, emulsions, microparticles, microcapsules and the like. The required dosage can be administered as a single unit or in a sustained release form.

Sustainable-release forms and appropriate materials and methods for their preparation are described in the art. In a particular embodiment, the orally administrable form of a pharmaceutical composition comprising the agent according to the present invention, an antibody construct according to the invention or a nanoparticle according to the invention is in a sustained release form that further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of them. Enteric coatings may be applied using conventional processes known to experts in the art.

In another embodiment, the pharmaceutical composition of the invention is a nanoemulsion. "Nanoemulsion" as used herein means a colloidal dispersion of droplets (or particles) which at least some of the droplets have diameters in the nanometer size range. The nanoemulsions are comprised of omega-3, -6 or -9 fatty acid rich oils in an aqueous phase and thermo-dynamically stabilized by amphiphilic surfactants, which make up the interfacial surface membrane, produced using a high shear microfluidization process usually with droplet diameter within the range of about 80-220 nm.

In another preferred embodiment, the agent according to the present invention or the antibody construct according to the invention is present in an exosome.

As used herein, the term "exosome" refers to an extracellular vesicle with a diameter between 20-300 nm (e.g., between 40-200 nm, 40-150nm). Exosomes comprise a membrane that encloses an internal space (i.e., lumen), and, in some instance, can be generated from a cell (e.g., producer cell) by direct plasma membrane budding or by fusion of the late endosome with the plasma membrane. In certain instances, an exosome comprises a scaffold/transmembrane moiety.

All the particular embodiments of the agent binding specifically to the sequence shown in SEQ ID NO: 1, SEQ ID NO: 2 or SEQ ID NO: 3 of the present invention are also applicable to these aspects of the invention.

### Therapeutic uses

The authors of the present invention have shown that the agents binding specifically to the sequence shown in SEQ ID NO: 1, SEQ ID NO: 2 or SEQ ID NO: 3 reduce the proliferation of cancer cells and reduce the size and weight of tumors (Example 1). In addition, the agents of the invention limit cancer cell migration, invasion, and metastasis (Example 2).

In another aspect, the invention relates to the agent according to the invention, the antibody construct according to the invention, the combination according to the invention, the nanoparticle according to the invention or the pharmaceutical composition according to the invention for use in medicine.

The invention also relates to the nucleic acid of the invention, the expression cassette of the invention, the vector of the invention or the cell of the invention for use in medicine.

In another aspect, the invention relates to the agent according to the invention, the antibody construct according to the invention, the combination according to the invention, the nanoparticle according to the invention or the pharmaceutical composition according to the invention for use in the treatment and/or prevention of cancer, metastasis, or fibrosis.

Alternatively, the invention relates to a method for preventing and/or treating cancer or fibrosis which comprises administering the agent according to the invention, the antibody construct according to the invention, the combination according to the invention, the nanoparticle according to the invention or the pharmaceutical composition according to the invention to a subject in need thereof.

The invention also relates to the nucleic acid of the invention, the expression cassette of the invention, the vector of the invention or the cell of the invention for use in the treatment and/or prevention of cancer, metastasis, or fibrosis.

In another aspect, the invention relates to an agent binding specifically to the sequence shown in SEQ ID NO: 1, SEQ ID NO: 2 or SEQ ID NO: 3 wherein said agent is an immunoglobulin agent or a non-immunoglobulin agent selected from the group consisting of a peptide aptamer, a nucleic acid aptamer, a DARPin, an affibody, and an anticalin for use in medicine.

In another aspect, the invention relates to an agent binding specifically to the sequence shown in SEQ ID NO: 1, SEQ ID NO: 2 or SEQ ID NO: 3 wherein said agent is an immunoglobulin agent or a non-immunoglobulin agent selected from the group consisting of a peptide aptamer, a nucleic acid aptamer, a DARPin, an affibody, and an anticalin for use in the treatment and/or prevention of cancer, metastasis or fibrosis.

Alternatively, the invention relates to a method for preventing and/or treating cancer or fibrosis comprising administering an agent binding specifically to the sequence shown in SEQ ID NO: 1, SEQ ID NO: 2 or SEQ ID NO: 3 wherein said agent is an immunoglobulin agent or a non-immunoglobulin agent selected from the group consisting of a peptide aptamer, a nucleic acid aptamer, a DARPin, an affibody, and an anticalin to a subject in need thereof.

The term "subject", as used herein, refers to all animals classified as mammals and includes, without limitation, domestic and farm animals, primates, and humans, e.g., human beings, non-human primates, cows, horses, pigs, sheep, goats, dogs, cats, or rodents. Preferably, the subject is a male or female human of any age or race.

Alternatively, the invention relates to the use of the pharmaceutical composition of the invention for the preparation of a medicament for the prevention and/or treatment of cancer of fibrosis.

The term "prevention", "preventing" or "prevent", as used herein, relates to the administration of a pharmaceutical composition according to the invention to a subject who has not been diagnosed as possibly having the disease, but who would normally be expected to develop said disease or be at increased risk for said disease. The prevention intends to avoid the appearance of said disease. The prevention may be complete (e.g., the total absence of a disease). The prevention may also be partial, such that for example the occurrence of a disease in a subject is less than that which would have occurred without the administration of the composition of the present invention. Prevention also refers to reduced susceptibility to a clinical condition.

The term "treatment", as used herein, refers to any type of therapy, which is aimed at terminating, preventing, ameliorating, or reducing the susceptibility to a clinical condition as described herein. In a preferred embodiment, the term treatment relates to prophylactic treatment (i.e., a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter.

In a preferred embodiment, the agent according to the invention, the antibody construct according to the invention, the combination according to the invention, the nanoparticle according to the invention or pharmaceutical composition of the invention is for use in the treatment of cancer.

In a preferred embodiment, the pharmaceutical composition for use in the treatment of cancer is the pharmaceutical composition comprising the combination of the invention and together with a pharmaceutically acceptable excipient or carrier.

The term "cancer" as used herein, refers to a disease characterized by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. As used herein, the term cancer includes, but is not limited to, the following types of cancer: breast cancer; biliary tract cancer; bladder cancer; brain cancer including glioblastomas, in particular glioblastoma multiforme, and medulloblastomas; cervical cancer; head and neck carcinoma; choriocarcinoma; colon cancer, colorectal cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer, hepatoma; lung cancer, pleural mesothelioma; lymphomas including Hodgkin's disease and lymphocytic lymphomas; neuroblastomas; oral cancer including squamous cell carcinoma; parotid gland cancer; ovarian cancer including those arising from epithelial cells, stromal cells, germ cells and mesenchymal cells; pancreatic cancer; prostate cancer; kidney cancer, suprarenal cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; cervix cancer, endometrial cancer; testicular cancer including germinal tumors such as seminoma, non-seminoma (teratomas, choriocarcinomas), stromal tumors, and germ cell tumors; thyroid cancer including thyroid adenocarcinoma and medullary carcinoma; and renal cancer including adenocarcinoma and Wilms tumor. Other cancers will-be known to one of ordinary skill. In a particular embodiment, the cancer is as gastric, duodenal, liver, hematologic, breast, glioma, bladder, pancreatic, prostate, colorectal, cervical, nasopharyngeal, lung, kidney, metastasis, melanoma, sarcoma, neural, or skin cancer.

In another preferred embodiment, the cancer is selected from the group consisting of prostate cancer, colorectal cancer, breast, melanoma, pancreatic, cervical, glioma, bladder, urothelial, kidney, renal, testicular, thyroid and lung cancer. In a more preferred embodiment, the cancer is colorectal cancer. In another preferred embodiment, the cancer is kidney cancer. In another preferred embodiment, the cancer is pancreatic cancer. In another preferred embodiment, the cancer is prostate cancer. In another more preferred embodiment, the prostate cancer is castration resistant prostate cancer. Castration-resistant prostate cancer (CRPC) is cancer that continues to grow even when the testosterone levels are at or below the castrate level. It can also be called hormone-refractory or hormone-resistant prostate cancer

In another preferred embodiment, the cancer is characterized by presenting tumor cells having high WNT-11 expression. By having high WNT-11 expression means than the gene WNT-11 is highly expressed and therefore high levels of Wnt-11 protein are produced.

By "high WNT-11 expression", as used herein it is understood that the concentrations of Wnt-11 are greater than those occurring in a control sample by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more.

Virtually any conventional method can be used within the frame of the invention to detect and quantify the levels of proteins. By way of a non-limiting illustration, the expression levels are determined by means of antibodies with the capacity for binding specifically to the protein to be determined (or to fragments thereof containing the antigenic determinants) and subsequent quantification of the resulting antigen-antibody complexes. The antibodies that are going to be used in this type of assay can be, for example, polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemiluminescent reagents, enzyme cofactors or substrates, enzyme inhibitors, particles, dyes, etc. There is a wide variety of well-known assays that can be used in the present invention, using non-labeled antibodies (primary antibody), labeled antibodies (secondary antibodies); these techniques include Westernblot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, immunofluorescence, techniques based on the use of biochips or protein microarrays including specific antibodies or assays based on the colloidal precipitation in formats such as reagent strips. Other forms of detecting and quantifying the proteins include affinity chromatography techniques, ligand-binding assays, etc.

On the other hand, the determination of the levels of a protein can be carried out by constructing a tissue microarray (TMA) containing the subject samples assembled and determining the expression levels of the corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous reevaluation. Briefly, the result of immunostaining can be recorded as negative expression (0) versus positive expression, and low expression (1+) versus moderate (2+) and high (3+) expression, taking into account the expression in tumor cells and the specific cut-off for each marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol. 2009; 47: 349-54) or Mulrane, L. et al. (Expert Rev. Mol. Diagn. 2008; 8: 707-25).

In a preferred embodiment, Wnt-11 expression is classified as negative (-), low (±), moderate (+), high (++), or very high (+++) according to the method used in Gorroño, I. et al., Cancers (Basel). 2019 Jul; 11(7): 908.

Control sample is understood as a sample having levels of Wnt -11 which are used as a reference for the determination of the relative levels of Wnt -11 in a test sample. The reference samples are typically obtained from patients who are well documented from the clinical point of view, and who present no disease. In said samples, the Wnt -11 concentration can be determined, for example, by means of the determination of the average concentration in a reference population. In the determination of the reference concentration for a certain marker, it is necessary to take into consideration some characteristics of the type of sample, such as age, gender, the physical state, and the like of the patient. For example, the reference sample can be obtained from identical amounts of a group of at least 2, at least 10, at least 100 to more than 1000 individuals, such that the population is statistically significant. The concentration of Wnt -11 can be determined intracellularly or extracellularly. Since Wnt -11 is a secreted protein, in a preferred embodiment, the concentration of Wnt -11 is determined intracellularly. The levels of Wnt -11 can be determined by means of measuring the amount of protein using immunological methods or the levels of mRNA by any known method in the art, for example using the ΔΔCT method. The level of mRNA can be determined relative to the expression of any housekeeping gene, for example 36B4.

The person skilled in the art will appreciate that the method of the invention can be put into practice using both the absolute level and the relative level of expression of the Wnt -11 protein. Thus, in the present invention, the expression "levels of the WNT-1 1" is used to refer both the absolute levels and the relative levels of said protein.

The expression "absolute levels" refers to the total amount of the protein of interest in a sample. Said value may be given as the concentration of protein expressed in units of mass per unit of volume (e.g. in ng/ml of sample), in the number of protein molecules per unit of volume (e.g. in pmol protein/ml of sample), in the units of mass of Wnt -11 protein per unit of mass of total protein (pg Wnt-11/mg total protein) or in the number of Wnt -11 molecules per unit of mass of total protein (e.g. in pmol Wnt -11/mg of total protein).

The expression "relative levels" refers to the relationship between the levels of expression of the Wnt-11 protein object of the study and of a reference protein, i.e., the concentration of Wnt-11 protein in normalized form with respect to said reference protein is defined. "Control protein" or reference protein in the present invention is understood as a protein the levels of expression of which do not change or only change in limited amounts in the tumor cells with respect to the non-tumor cells. Preferably, the control protein is a protein encoded by genes that are constitutively expressed, that are those genes always active or being transcribed constantly, such that these proteins are constitutively expressed and carry out essential cellular functions. Preferred control proteins that can be used in the present invention include, without limitation, β-2-microglobulin (B2M), ubiquitin, 18-S ribosomal protein, cyclophilin, GAPDH, PSMB4, tubulin and actin.

In another preferred embodiment, the cancer has activated β-catenin independent signals. In a preferred embodiment, β-catenin-independent signals is activated via AP-1/ATF family transcription factors.

Traditionally, Wnt signals have been classified into canonical or β-catenin-dependent and noncanonical or β-catenin-independent pathways. Noncanonical Wnt signals are activated upon Wnt binding to FZD receptors and tyrosine kinase-like co-receptors, leading to the recruitment and activation of DVL.

The Wnt/β-catenin signaling pathway is an evolutionarily conserved mechanism that plays a preeminent role in maintaining cellular homeostasis.

In another preferred embodiment, the cancer is advanced cancer, drug resistant and/or metastatic cancer.

"Advanced cancer", as used herein relates to a cancer that is unlikely to be cured or controlled with treatment. The cancer may have spread from where it first started to nearby tissue, lymph nodes, or distant parts of the body. Treatment may be given to help shrink the tumor, slow the growth of cancer cells, or relieve symptoms.

"Drug resistant", as used herein relates to an intimate occurrence that results when cancer becomes tolerant of pharmaceutical treatment. Drug resistance can occur due to the activation of both intrinsic (pre-existing) or acquired (induced by drugs) mechanisms, and both types of factors play significant roles in the development of drug resistance.

As cancers progress, they may metastasize. The term "metastasis" or "metastatic disease", as used herein, refers to the spread of a cancer or disease from one organ or part to another not directly connected with it. When tumour cells metastasize, the new tumour is called a secondary or metastatic tumour, and its cells are similar to those in the original tumour.

"Metastasis" is the spread of a cancerous source from the initial site of the same to another organ. This normally occurs via the blood or lymphatic system. When the cancerous cells spread and cause a new tumor, this is called a secondary or metastatic tumor.

In another preferred embodiment, the invention relates to the agent according to the invention, the antibody construct according to the invention, the combination according to the invention, the nanoparticle according to the invention or the pharmaceutical composition of the invention for use in the treatment of fibrosis.

"Fibrosis", or pathogenic fibrosis as used herein relates to a disease where pathogenic fibrosis occurs (i.e., when said process is harmful or undesirable), and it relates to any disease giving rise to fibrosis, particularly excessive fibrosis, whether as a main or a secondary symptom. Fibrosis is characterized by the accumulation and reorganization of the extracellular matrix (ECM). Despite having obvious etiological and clinical distinctions, most chronic fibrotic disorders have in common a sustained production of growth factors, proteolytic enzymes, angiogenic factors, and fibrogenic cytokines, which stimulate the deposition of connective tissue elements, especially collagens and proteoglycans, which progressively remodel and destroy normal tissue architecture. Fibrosis is the formation of excess fibrous connective tissue in an organ or tissue in a reparative or reactive process. This can be a reactive, benign, or pathological state. The deposition of connective tissue in the organ and/or tissue can obliterate the architecture and function of the underlying organ or tissue. Pathological fibrosis is this pathological state of excess deposition of fibrous tissue, as well as the process of connective tissue deposition in healing. Pathological fibrosis is characterized by non-resolving or progressive tissue remodeling, which itself can cause tissue damage and organ failure.

Fibrosis include, without limitation, pulmonary fibrosis (including idiopathic pulmonary fibrosis and cystic fibrosis), renal fibrosis, hepatic cirrhosis, endomyocardial fibrosis, atrial fibrosis, mediastinal fibrosis, myelofibrosis, retroperitoneal fibrosis, progressive massive fibrosis of the lungs, nephrogenic systemic fibrosis, Crohn's disease, keloid, Scleroderma/systemic sclerosis of the skin or the lungs, arthrofibrosis, Peyronie's disease, Dupuytren's contracture, some forms of adhesive capsulitis, peritoneal fibrosis, and skin fibrosis.

In a preferred embodiment, the fibrosis is pulmonary fibrosis.

"Lung fibrosis" or "pulmonary fibrosis", as used herein, relates to a condition in which the lungs become scarred over time. Symptoms include shortness of breath, a dry cough, feeling tired, weight loss, and nail clubbing.

In another preferred embodiment, the fibrosis is capsular fibrosis, renal fibrosis or kidney fibrosis.

"Capsular fibrosis", as used herein relates to fibrotic foreign body reaction caused by inflammatory signalling. TNF-α production has been observed in the progression of capsular fibrosis, where it is expressed primarily in collagen-depositing fibroblasts and macrophages around the implant

"In another preferred embodiment, the fibrosis is renal fibrosis.

"Renal fibrosis," as used herein, relates to a disease characterized by glomerulosclerosis, tubulointerstitial fibrosis, loss of renal parenchyme, and inflammatory cell infiltration.

In another preferred embodiment, the fibrosis is kidney fibrosis.

"Kidney fibrosis" is characterized by excessive production and deposition of extracellular matrix (ECM) proteins mainly in the kidney interstitium and results in structural damage.

In another particular embodiment, the fibrosis is cardiac fibrosis.

"Cardiac fibrosis", as used herein relates to the excess deposition of extracellular matrix in the cardiac muscle, but the term may also refer to an abnormal thickening of the heart valves due to inappropriate proliferation of cardiac fibroblasts.

All the definitions and particular embodiments of the agent binding specifically to the sequence shown in SEQ ID NO: 1, SEQ ID NO: 2 or SEQ ID NO: 3 of the present invention are also applicable to this aspect of the invention.

### Peptide of the invention

The authors of the invention have observed that replacing the amino acids D3 or L4 of the sequence SEQ ID NO: 114 by alanine, said sequences inhibits Wnt-11 activity.

In an eleventh aspect, the invention relates to a peptide having the sequence shown in SEQ ID NO: 3, SEQ ID NO: 24, SEQ ID NO: 35, SEQ ID NO: 36 or a variant thereof having at least 70% sequence identity with said sequences.

In another preferred embodiment, the variant has at least approximately 70%, at least 75%, at least 80%, at least 85%, at least 90%, at least 91%, at least 92%, at least 93%, at least 94%, at least 95%, at least 96%, at least 97%, at least 98% or at least 99% sequence identity with the corresponding amino acid sequences shown in SEQ ID NO: 3, SEQ ID NO: 24, SEQ ID NO: 35 or SEQ ID NO: 36 .

In another preferred embodiment, the functionally equivalent variant of SEQ ID NO: 3 or 24 is a peptide sequence having a Methionine residue at the beginning of any one of SEQ ID NO: 3, SEQ ID NO: 24, SEQ ID NO: 35 or SEQ ID NO: 36.

### Method for screening compounds capable of inhibiting Wnt-11

In another aspect, the invention relates to a method for screening compounds capable of inhibiting Wnt-11 which comprises:
a) contacting the compound with a cell stably expressing high levels of Wnt-11 to allow the binding of the compound to Wnt-11 thereby forming a complex,
b) incubating the complex formed in a) with an anti Wnt-11 antibody in conditions to allow the binding of the antibody to Wnt-11,
c) quantifying the binding of the antibody to Wnt-11, wherein if the binding is reduced compared to the binding of a control antibody recognizing the epitope tag, then the compound is an inhibitor of Wnt-11.

In a preferred embodiment of step a) of the method of the invention for screening compounds, the cell stably expressing high levels of Wnt-11 expresses Wnt-11 comprising an epitope tag in a region structurally distant from the epitope of the Wnt-11 antibody. Step a) can be performed in any conditions known in the art, for example typically in a buffered salt solution pH 7.4, such as HBSS, PBS or TBS, or cell culture media, such as DMEM or RPMI containing 0%, 1%, 2%, 5% or 10% foetal calf serum, at 4, 20, 30 or 37 °C, for between 15 minutes and 24 hours.

"Epitope tag" as used herein relates to short peptide sequences, generally composed of 10-15 amino acids, designed to create a molecular handle for a protein. Illustrative non-limitative examples of epitope tags are Avi, c-My, flag, HA, His, HSV, RFP, S1, Strepll, Tap, V5, VSV-G.

Step b) of the method for screening compounds of the invention can be performed in any condition known in the art, for example typically in a buffered salt solution pH 7.4, such as HBSS, PBS or TBS, or cell culture media, such as DMEM or RPMI containing 0%, 1%, 2%, 5% or 10% foetal calf serum, at 4, 20, 30 or 37 °C, for between 15 minutes and 24 hours). In a preferred embodiment of step b) the Wnt-11 antibody is at a concentration between 10 ng/ml and 100 µg/ml.

In a preferred embodiment, the method for screening compounds capable of inhibiting Wnt-11 comprises repeating the steps b) and c), wherein the anti Wnt-11 antibody is different from the anti Wnt-11 antibody previously used. Thus, the compounds that bind to the anti Wnt-11 antibody rather than to Wnt-11 are discarded.

Therefore, in a preferred embodiment the method for screening compounds capable of inhibiting Wnt-11 further comprises
d) contacting again the compound with a cell stably expressing high levels of Wnt-11 to allow the binding of the compound to Wnt-11,
e) incubating the complex formed in a) with a different anti Wnt-11 antibody in conditions to allow the binding of the antibody to the Wnt-11,
f) quantifying the binding of the antibody to Wnt-11, wherein if the binding is reduced compared to a control, then the compound is an inhibitor of Wnt-11.

In a preferred embodiment, the anti Wnt-11 antibody is an antibody binding specifically to the sequence shown in SEQ ID NO: 1, SEQ ID NO: 2 or SEQ ID NO: 3. In a more preferred embodiment, the antibody binds specifically to the sequence shown in SEQ ID NO: 3.

In another preferred embodiment, the antibody comprises within the heavy chain:
- a CDR comprising the amino acid sequence shown in SEQ ID NO: 4 [CDR-H1], a CDR comprising the amino acid sequence shown in SEQ ID NO: 5 [CDR-H2], and a CDR comprising the amino acid sequence shown in SEQ ID NO: 6 [CDR-H3], or a functionally equivalent variant of said CDRs,
   and/or comprises within the light chain:
- a CDR comprising the amino acid sequence shown in SEQ ID NO: 7 [CDR-L1], a CDR comprising the amino acid sequence shown in SEQ ID NO: 8 [CDR-L2], and a CDR comprising the amino acid sequence shown in SEQ ID NO: 9 [CDR-L3], or a functionally equivalent variant of said CDRs
or wherein said antibody comprises within the heavy chain
- a CDR comprising the amino acid sequence shown in SEQ ID NO: 10 [CDR-H1], a CDR comprising the amino acid sequence shown in SEQ ID NO: 11 [CDR-H2], and a CDR comprising the amino acid sequence shown in SEQ ID NO: 12 [CDR-H3], or a functionally equivalent variant of said CDRs,
and/or within the light chain:
- a CDR comprising the amino acid sequence shown in SEQ ID NO: 13 [CDR-L1], a CDR comprising the amino acid sequence shown in SEQ ID NO: 14 [CDR-L2], and a CDR comprising the amino acid sequence shown in SEQ ID NO: 15 [CDR-L3], or a functionally equivalent variant of said CDRs.

In a more preferred embodiment, the heavy chain variable region of the antibody has the sequence shown in SEQ ID NO: 16 and the light chain sequence variable region has the sequence shown in SEQ ID NO: 17, or wherein the heavy chain variable region of the antibody has the sequence shown in SEQ ID NO: 18 and the light chain sequence variable region has the sequence shown in SEQ ID NO: 19.

In another preferred embodiment, the antibody is a murine-derived monoclonal antibody, a human-murine chimeric antibody, or a humanized antibody, preferably a human-murine chimeric antibody, more preferably a human-murine chimeric antibody comprising a human IgG4k constant sequence, more preferably the human IgG4k constant sequence is selected from the group consisting of SEQ ID NO: 20 or SEQ ID NO: 21

In another preferred embodiment, the human-murine chimeric antibody has the heavy chain sequence shown in SEQ ID NO: 22 and the light chain sequence shown in SEQ ID NO: 23.

In another preferred embodiment, the human-murine chimeric antibody has the heavy chain variable region sequence shown in SEQ ID NO: 16 and the light chain variable region sequence shown in SEQ ID NO: 17. In another preferred embodiment, the human-murine chimeric antibody in particular rat-human antibody chimeric, has the heavy chain sequence shown in SEQ ID NO: 136.

In another preferred embodiment, the human-murine chimeric antibody comprising human IgG1k constant sequence. In a more preferred embodiment, the human IgG1k constant sequence is SEQ ID NO: 32 or SEQ ID NO: 21

In another preferred embodiment, the human-murine chimeric antibody, in particular rat-human chimeric antibody, has the light chain sequence shown in SEQ ID NO: 137.

In another preferred embodiment, the human-murine chimeric antibody, in particular rat-human chimeric antibody, has the heavy chain sequence shown in SEQ ID NO: 136 and the light chain sequence shown in SEQ ID NO: 137.

In another preferred embodiment the antibody is a humanized antibody.

In a more preferred embodiment, the humanized antibody has the heavy chain variable region shown in SEQ ID NO: 26. In another preferred embodiment, the humanized antibody has the heavy chain variable region shown in SEQ ID NO: 33. In another preferred embodiment, the humanized antibody has the heavy chain variable region shown in SEQ ID NO: 27. In another preferred embodiment, the humanized antibody has the heavy chain variable region shown in SEQ ID NO: 34. In another preferred embodiment, the humanized antibody has the heavy chain variable region shown in SEQ ID NO: 138.

In another preferred embodiment, humanized antibody has the light chain variable region sequence shown in SEQ ID NO: 110. In another preferred embodiment, the humanized antibody has the light chain variable region shown in SEQ ID NO: 111. In another preferred embodiment, the humanized antibody has the light chain variable region shown in SEQ ID NO: 112. In another preferred embodiment, the humanized antibody has the light chain variable region shown in SEQ ID NO: 113. In another preferred embodiment, the humanized antibody has the light chain variable region shown in SEQ ID NO: 139.

In another preferred embodiment, the humanized antibody has the heavy chain variable region shown in SEQ ID NO: 26 and the light chain variable region sequence shown in SEQ ID NO: 110. In another preferred embodiment, the humanized antibody has the heavy chain variable region shown in SEQ ID NO: 27 and the light chain variable region sequence shown in SEQ ID NO: 111. In another preferred embodiment, the humanized antibody has the heavy chain variable region shown in SEQ ID NO: 33 and the light chain variable region sequence shown in SEQ ID NO: 112. In another preferred embodiment, the humanized antibody has the heavy chain variable region shown in SEQ ID NO: 34 and the light chain variable region sequence shown in SEQ ID NO: 113.

In another preferred embodiment, the humanized antibody has the heavy chain sequence shown in SEQ ID NO: 138 and the light chain sequence shown in SEQ ID NO: 139.

In another preferred embodiment, steps c) and f) comprises quantifying the binding of the corresponding antibody to Wnt-11 and also the blocking function of Wnt-11. The binding and/or blocking function of Wnt-11 can be determining by any method known in the art, such as the methods disclosed in the experimental data.

In a preferred embodiment, the compound is considered an inhibitor of Wnt-11 if the binding determined in c) or F) is reduced at least 30 %, preferably at least 40%, at least 50%, at least 60 % or more compared to a control.

### Other aspects

[1] An agent binding specifically to the sequence shown in SEQ ID NO: 1, SEQ ID NO: 2 or SEQ ID NO: 3, wherein said agent is an immunoglobulin agent or a non-immunoglobulin agent selected from the group consisting of a peptide aptamer, a nucleic acid aptamer, a DARPin, an affibody, and an anticalin.
[2] The agent according to aspect [1] which binds specifically to the sequence shown in SEQ ID NO: 3.
[3] The agent according to any one of aspects [1] or [2], wherein said immunoglobulin agent is an antibody or an antigen-binding fragment of said antibody.
[4] The agent according to aspect [3], wherein said antigen-binding fragment is selected from the group consisting of Fv, Fab, F(ab')₂ and Fab'.
[5] The agent according to any one of aspects [3] or [4], wherein said antibody or said antigen-binding fragment comprises within the heavy chain:
   - a CDR comprising the amino acid sequence shown in SEQ ID NO: 4 [CDR-H1], a CDR comprising the amino acid sequence shown in SEQ ID NO: 5 [CDR-H2], and a CDR comprising the amino acid sequence shown in SEQ ID NO: 6 [CDR-H3], or a functionally equivalent variant of said CDRs,
   and/or comprises within the light chain:
   - a CDR comprising the amino acid sequence shown in SEQ ID NO: 7 [CDR-L1], a CDR comprising the amino acid sequence shown in SEQ ID NO: 8 [CDR-L2], and a CDR comprising the amino acid sequence shown in SEQ ID NO: 9 [CDR-L3], or a functionally equivalent variant of said CDRs
   or wherein said antibody or said antigen-binding fragment comprises within the heavy chain
   - a CDR comprising the amino acid sequence shown in SEQ ID NO: 10 [CDR-H1], a CDR comprising the amino acid sequence shown in SEQ ID NO: 11 [CDR-H2], and a CDR comprising the amino acid sequence shown in SEQ ID NO: 12 [CDR-H3], or a functionally equivalent variant of said CDRs,
   and/or within the light chain:
   - a CDR comprising the amino acid sequence shown in SEQ ID NO: 13 [CDR-L1], a CDR comprising the amino acid sequence shown in SEQ ID NO: 14 [CDR-L2], and a CDR comprising the amino acid sequence shown in SEQ ID NO: 15 [CDR-L3], or a functionally equivalent variant of said CDRs.
[6] The agent according to aspect [5], wherein the heavy chain variable region of the antibody has the sequence shown in SEQ ID NO: 16 and the light chain sequence variable region has the sequence shown in SEQ ID NO: 17, or wherein the heavy chain variable region of the antibody has the sequence shown in SEQ ID NO: 18 and the light chain sequence variable region has the sequence shown in SEQ ID NO: 19.
[7] The agent according to any one of aspects [3] to [5], wherein said antibody is a murine-derived monoclonal antibody, a human-murine chimeric antibody, or a humanized antibody.
[8] The agent according to aspect [7], wherein the human-murine chimeric antibody comprises a human IgG4k constant sequence.
[9] The agent according to aspect [8], wherein the human IgG4k constant sequence is selected from the group consisting of SEQ ID NO: 20 or SEQ ID NO: 21.
[10] The agent according to aspect [9], wherein the human-murine chimeric antibody has the heavy chain sequence shown in SEQ ID NO: 22 and the light chain sequence shown in SEQ ID NO: 23.
[11] An antibody construct comprising the antigen-binding fragment according to any one of aspects [3] to [5], wherein the antibody construct is selected from the group consisting of scFv, scFv-Fc, minibody, (scFv)₂ and diabody.
[12] A combination comprising:
   i. an agent binding to the sequence shown in SEQ ID NO: 1, SEQ ID NO: 2 or SEQ ID NO: 3 according to any one of aspects 1 to 10 or an antibody construct according to aspect 11, and
   ii. an anti-androgen compound.
[13] A nucleic acid selected form the group consisting of:
   a) a nucleic acid encoding the agent according to any one of aspects [1] to [1]0 wherein the agent is a protein compound, or the antibody construct according to aspect [11] and
   b) a complementary nucleic acid of a nucleic acid as defined in a).
[14] An expression cassette comprising the nucleic acid according to aspect [13].
[15] A vector comprising the nucleic acid according to aspect [13] or the expression cassette according to aspect [14].
[16] A cell comprising the nucleic acid according to aspect [13], or the expression cassette according to aspect [14], or a vector according to aspect [15].
[17] A nanoparticle comprising the agent according to any one of aspects [1] to [10] or the antibody construct according to aspect [11] or the combination according to aspect [12].
[18] A pharmaceutical composition comprising a therapeutically effective amount of an agent according to any one of aspects [1] to [10], an antibody construct according to aspect [11], a combination according to aspect [12], or a nanoparticle according to aspect [17], together with a pharmaceutically acceptable excipient or carrier.
[19] Pharmaceutical composition according to aspect [18] for use in medicine.
[20] Pharmaceutical composition according to aspect [18] for use in the treatment of cancer, metastasis, or fibrosis.
[21] The pharmaceutical composition for use according to aspect [20], wherein the cancer is selected from the group consisting of prostate cancer, colorectal cancer, breast cancer, melanoma, pancreatic cancer, cervical cancer, glioma, bladder cancer, urothelial cancer, kidney cancer, renal cancer, testicular cancer, thyroid cancer, and lung cancer.
[22] The pharmaceutical composition for use according to any one of aspects [20] or [21], wherein the cancer is characterized by presenting tumor cells having high WNT-11 expression.
[23] The pharmaceutical composition for use according to any one of aspects [20] to [22], wherein the cancer is advanced cancer, drug resistant and/or metastatic cancer.
[24] A peptide having the sequence shown in SEQ ID NO: 3, SEQ ID NO: 24, SEQ ID NO: 35 or SEQ ID No: 36 or a variant thereof having at least 70% sequence identity with said sequences.
[25] A method for screening compounds capable of inhibiting Wnt11 which comprises:
   a) contacting the compound with a cell stably expressing high levels of Wnt-11 to allow the binding of the compound to Wnt-11 thereby forming a complex and
   b) incubating the complex formed in a) with an anti Wnt11 antibody in conditions to allow the binding of the antibody to Wnt-11
   c) quantifying the binding of the antibody to Wnt 11, wherein if the binding is reduced compared to the binding of a control, then the compound is an inhibitor of Wnt11.
[26] The method according to claim [25] further comprising:
   a) contacting again the compound with a cell stably expressing high levels of Wnt-11 to allow the binding of the compound to Wnt-11,
   b) Incubating the complex formed in a) with a different anti-Wnt11 antibody in conditions to allow the binding of the antibody to the Wnt-11,
   c) quantifying the binding of the antibody to Wnt 11, wherein if the binding is reduced compared to a control then the compound is an inhibitor of Wnt11.

The authors of the invention have shown that specific residues in the Wnt-3a sequence (SEQ ID NO: 25) are required for canonical Wnt signalling (Example 5).

The invention also relates to an agent binding specifically to the sequence shown in SEQ ID NO: 25 (second agent of the invention), wherein said agent is an immunoglobulin agent (second immunoglobulin agent) or a non-immunoglobulin agent (second non-immunoglobulin agent) selected from the group consisting of a peptide aptamer, a nucleic acid aptamer, a DARPin, an affibody, and an anticalin.

In another aspect, the invention relates to an antibody construct comprising the second antigen-binding fragment according to the invention, wherein the antibody construct is selected from the group consisting of scFv, scFv-Fc, minibody, (scFv)2 and diabody.

In another aspect, the invention relates to a nucleic acid (second nucleic acid) selected form the group consisting of:
a) a nucleic acid encoding the agent binding specifically to the sequence shown in SEQ ID NO: 25 according to the invention, or the antibody construct according to the invention and
b) a complementary nucleic acid of a nucleic acid as defined in a).

In another aspect, the invention relates to an expression cassette (second expression cassette) comprising the second nucleic acid according to the invention.

In another aspect, the invention relates to a vector (second vector of the invention) comprising the second nucleic acid according to the invention or the second expression cassette according to the invention.

In another aspect, the invention relates to a cell (second cell of the invention) comprising the second nucleic acid according to the invention, or the second expression cassette according to the invention, or the second vector according to the invention

In another aspect, the invention relates to a nanoparticle (second nanoparticle of the invention) comprising the second agent according to the invention or the second antibody construct according to the invention.

In another aspect, the invention relates to a pharmaceutical composition (second pharmaceutical composition of the invention) comprising a therapeutically effective amount of the second agent according to the invention, a second antibody construct according to the invention or the second nanoparticle according to the invention, together with a pharmaceutically acceptable excipient or carrier.

In another aspect, the invention relates to the second agent of the invention, the second an antibody construct of the invention, the second combination of the invention, the second nanoparticle of the invention or the second pharmaceutical composition according to the invention for use in medicine.

In another aspect, the invention relates to a pharmaceutical composition according to the invention for use in the treatment of cancer, metastasis or fibrosis.

All the definitions and particular embodiments previously defined are equally applicable to these aspects of the invention.

### Examples

### MATERIALS AND METHODS

Human Wnt recombinant proteins were purchased from R&D systems, Bio-Techne (Wnt-11: 6179-WN-010, Wnt-3a: 5036-WN-010). Porcupine inhibitor Wnt-C59 was purchased from Millipore Merck (Porcn Inhibitor II, Wnt-C59 - CAS 1243243-89-1, Calbiochem) and was used at 100 nM. Dimethyloxalylglycine, DMOG (Calbiochem, 400091) was used at 1 mM - 0.2 mM. When applicable carrier controls (i.e., Dimethyl sulfoxide (DMSO)) were added at the same dilution in parallel. The antibodies used in this study are described in (Table 1).

**Table 1. List of antibodies.**

| | | | | Working dilutions | | | |
|---|---|---|---|---|---|---|---|
| Antibody | Company | Reference | Species | WB | IF | FACS | IHC |
| DVL2 | Santa Cruz | SC-13974 | Rabbit | 1:1000 | | | |
| DVL3 | Santa Cruz | SC-8027 | Mouse | 1:500 | | | |
| Fc* (secondary anti-human) | GenScript | A00166 | Goat | 1:10000 | | | |
| GAPDH | Santa Cruz | SC-47724 | Mouse | 1:5000 | | | |
| HIF-1α | Novus Biological | NB100-479 | Rabbit | 1:1000 | | | |
| HIF-2α | Novus Biological | NB100-122 | Rabbit | 1:1000 | | | |
| HSP60 | Santa Cruz | sc-13966 | Rabbit | 1:1000 | | | |
| Ki67 | GenomeMe (GeneAb^{™}) | IHC067 | Mouse | | | | 1:100 |
| LRP6 | Santa Cruz | SC-25317 | Mouse | 1:1000 | 1:50 | | |
| PA (tag) | Novus Biological | NBP-03952 | Rat | 1:1000 | | | |
| V5 (tag) | Invitrogen | R960-25 | Mouse | 1:1000 | 1:50 | | |
| Vinculin | Cell Signaling | 13901 | Rabbit | 1:2000 | | | |
| VSV-G (tag) | Biomol (Bethyl Laboratories) | A190-131A | Rabbit | 1:1000 | | | |
| Wnt-11 | R&D systems | AF2647 | Goat | 1:1000 | 1:50 | | |
| Wnt-11 | R&D systems | MAB3746 | Rat | 1:500 | 1:100 | | 1:100 |
| Rat-lgg (non-targeting) | Sigma | I4131 | Rat | 1:1000 | | | |
| Ab1 (Wnt-11) | *In house* produced | | Rat | 1:1000 | 1:30 | 1:30 | |
| Ab2 (Wnt-11) | *In house* produced | | Rat | 1:1000 | | | |

### Cell culture

PC-3, VCaP, DU145 and 293 cells were obtained from the American Type Culture Collection (Rockville, MD, USA). L-Wnt-3a and control parental cells were purchased also from ATCC for conditioned media (CM) production, which was collected according to manufacturer's instructions. The CM was stored at 4 °C for up to 1 month. For stimulation, CM was used in a 1:1 ratio with fresh media. The parental cell line was used for retrieving control CM in a parallel manner. PC-3M cells were provided by Scott Fraser and Mustafa Djamgoz (Imperial College London), C4-2B cells were from Charlotte Bevan (Imperial College London), HEK 293T LRP5/6- /- knock-out cells were kindly provided by Benoit Vanhollebeke's laboratory (Eubelen et al., Science (80). 1178, eaat1178 (2018)). HT1080 were kindly provided by Konstantin Stoletov and John D. Lewis (University of Alberta).

Cell lines were authenticated by DNA profiling (Eurofins Genomics, Germany) and cells were routinely tested for mycoplasma. Low passage stocks were cultured for up to 6 months after thawing. C4-2B, PC-3M, PC-3 and DU145 cells were cultured at 37 °C, 5% CO2 in RPMI-1640 with GlutaMAX^{™} (Gibco). VCaP cells were cultured in DMEM/Ham's F12 (Gibco). HEK293 and HT1080 cells were cultured in DMEM (Gibco). All growing media were supplemented with 10% fetal bovine serum (FBS; Gibco) and antibiotics (100 units mL-1 penicillin, 100 µg ml-1 streptomycin, Invitrogen, UK). For transfection, cells were transiently cultured in OptiMEM (Invitrogen). C4-2 stable overexpressing Wnt-11 and empty vector pcDNA were developed previously in the lab and cells were maintained in the presence of geneticin selective antibiotic (G418 Sulfate) at 0.4 mg/ml.

### Generation of anti-Wnt-11 monoclonal antibodies

Monoclonal anti-Wnt-11 antibodies Ab1 (E8) and Ab2 (F10) were generated by immunizing rats with a mixture of peptides derived from human Wnt-11. Wnt-11 peptides for the immunization, selection and testing of the anti-Wnt-11 antibodies were purchased from Genscript. After preliminary screening, anti-Wnt-11-Ab1 (Ab1) and anti-Wnt-11-F10 (F10) antibodies were selected. The antibodies were produced in bulk and purified from respective hybridoma cells (Davids Biotechnology GmbH) and stored in PBS at 0.7 mg/ml. Isotypes of antibodies were determined to be IgG2a for Ab1 and IgG2c for F10. Purified rat IgG (Sigma I4131) stored at 0.7 mg/ml in PBS was used as a control. Antibodies were tested in a range of concentrations between 0.2 and 20 µg/ml. Dot blot analysis found that both antibodies recognize the same peptide, LLDLERGTRESA (SEQ ID NO: 3), equivalent to Wnt-11 amino acids L101-A112.

### Generation of chimeric antibodies

Generation of E8 IgG4k chimeric antibodies: the E8 variable region sequences were cloned into the constant region sequences encoding for hIgG4 (S228P) kappa. The constant region sequences can be found in the following vector: https://www.invivogen.com/ptrioz-higg4#page-title); the E8 IgG4k chimeric antibody was expressed and purified by EVITRIA using their proprietary vector.

Generation of E8 IgG1k chimeric antibodies: the E8 variable region sequences were cloned into the constant region sequences encoding for h!gG1 kappa. This was done by Genscript.

### Cell proliferation

For proliferation assays, 5000 PC-3 or DU145 cells per well were plated in 12-well plates in media containing antibodies at the concentrations indicated. Media were replaced every other day 1:1 with fresh media containing antibodies, and cells were harvested at day 7. Cells were washed in PBS and stained using 0.1% crystal violet, 20% methanol, 0.36% PFA in PBS. Absorbance was measured at 595 nm after solubilizing in acetic acid, and readings were calculated relative to the internal control condition.

Alternatively Crystal violet assays were carried out using the prostate cancer cell line 22Rv1 (ATCC), as described by Campa et al., 2014 (Oncotarget. 2014; 5:8173-8187). The extent of growth inhibition was determined after 7 days. Q-PCR was carried out as described by Murillo et al. 2018 (Nature Communications volume 9, Article number: 1747 (2018)). KLK2 primers: GGTTCTCTCCATCGCCTTGT (SEQ ID NO: 37) GGAATGCTTCTCACACTCCCA (SEQ ID NO: 38) (both 5'-3').

### Cell transfection and plasmids

For gene reporter assays, 100,000 (293, HT1080) or 70,000 (PC-3) cells per well were plated in 12-well plates. After 24 h, cells were washed with PBS, incubated in OptiMEM and transfected with reporter or expression plasmids (Table 2 and Table 3) using Lipofectamine LTX with PLUS reagent (Life Technologies), as instructed by the manufacturer. 4 h after transfection fresh complete media were added to the cells.

For generation of the antibody epitope deletion mutant (Wnt-11m), a plasmid encoding human Wnt-11 was mutated by site-directed mutagenesis kit (NE Biolabs, E0554S) following manufacturer's instructions. The mutation generated was a deletion of part of the peptide contained in the antibody epitope, shown here underlined and with flanking sequences: APNY (SEQ ID NO: 39)/LLDLERGT (SEQ ID NO: 40)/RESA (SEQ ID NO: 41) and the primers used for the mutation were the following: forward primer 5'-CGGGAGTCGGCCTTCGTG-3' (SEQ ID NO: 42); reverse primer 5'-ATAGTTGGGGGCGAGCTC-3 (SEQ ID NO: 43). After mutagenesis, plasmids were purified, and Sanger sequenced (STABVIDA) prior to their use.

**Table 2. List of plasmids, (h) human; (m) mouse.**

| # | Plasmid name | Backbone | Insert | Tag* | Source / Reference |
|---|---|---|---|---|---|
| 1 | pcDNA 3.1 | pcDNA | - | - | - |
| 2 | Wnt-11 | pcDNA | Wnt-11 (h) | - | (Uysal-Onganer et al., 2010 Molecular Cancer, 9, 1-11.) |
| 3 | Wnt-3a-PA | pcDNA | Wnt-3a (h) | - | Junichi Takagi (Mihara et al., 2016 Elife . 2016 Feb 23;5:e11621) |
| 4 | Wnt-11-PA | pcDNA | Wnt-11 (h) | PA | Junichi Takagi (Mihara et al., 2016 Elife . 2016 Feb 23;5:e11621) |
| 5 | Wnt-11-V5 | pcDNA | Wnt-11 (h) | V5 | Marian Waterman, Addgene #35941 |
| 6 | AE** | pcDNA | Mutated Wnt-11 (h) from *plasmid 1* | - | *Homemade (mutagenesis)* |
| 7 | AE-PA | pcDNA | Mutated Wnt-11 (h) from *plasmid 2* | PA | *Homemade (mutagenesis)* |
| 8 | AE-V5 | pcDNA | Mutated Wnt-11 (h) from *plasmid 3* | V5 | *Homemade (mutagenesis)* |
| 9 | pCS2 | pCS2 | - | - | - |
| 10 | LRP6-VSV-G | pCS2 | VSV-G | VSV-G | Xi He, Addgene #27282 |
| 11 | LRP6N-IqG | pCS2 | LRP6N | Fc | Xi He, Addgene #27279 |
| 12 | pRK5 | pRK5 | - | - | - |
| 13 | mFZD8-CRD-IgG | pRK5 | FZD8-CRD (m) | Fc | Xi He, Addgene #16689 |

**Table 3. Luciferase plasmids.**

| Plasmid | Backbone | Transcription factors | Promoter | Source / Reference |
|---|---|---|---|---|
| ATF2-luc/ 1x | TATATK-LUC | ATF2 | ATTGCATCA | Christof Niehrs (Van Der Sanden, Meems, Houweling, Helms, & Vaandrager, 2004 Journal of Biological Chemistry, 279(50), 52007-52015) |
| mt5-luc | TATATK-LUC | Mutated ATF2 | ATT --- TCA | Pierre Fafournoux (Bruhat et al., 2000 Molecular and Cellular Biology, 20(19), 7192-7204) |
| Super 8XTOP/FOPFlash | pTA-LUC | TCF/LEF; β-catenin | CCTTTGATC | Randall Moon, Addgene #12456 (Veeman et al., 2003 Developmental Cell, 5(3), 367-377) |
| Renilla tk | pRL-Tk | - | - | Promega |

**Table 4. Description of peptide tags.**

| Tag | Protein origin | Sequence | Location |
|---|---|---|---|
| PA | Podoplanin | GVAMPGAEDDVV (SEQ ID NO: 47) | Wnt-11; N-terminal |
| V5 | SV5 paramyxovirus | GKPIPNPLLGLDST (SEQ ID NO: 48) | Wnt-11; C-terminal |
| VSV-G | Vesicular Stomatitis Virus (VSV) G | YTDIEMNRLGK (SEQ ID NO: 49) | LRP6; N-terminal |
| Fc | Immunoglobulin constant region of heavy chain | 25 kDa | LRP6N; C-terminal mFZD8-CRD; C-terminal |

### Sphere formation

For culturing spheres, single cells were seeded in ultralow attachment plates (Corning) coated poly (2-hydroxyethyl methacrylate) (pHEMA) (Sigma) in quadruplicate at 500 - 1000 cells/well.

Cells were grown in a serum-free DMEM/F12, supplemented with B27 (Invitrogen), 20 ng/mL EGF and 20 ng/mL bFGF (BD Biosciences) and 1% Pen/Strep (100 units/mL penicillin, 100 µg/mL streptomycin, Invitrogen, UK. Antibodies tested were used at 20 µg/ml. Cells were cultured undisturbed for 5-7 days. At endpoint, spheres were counted under a microscope and sphere numbers are presented relative to the control condition.

### Production and isolation of extracellular vesicles (EVs)

Cell culture supernatant was collected from VCaP prostate cancer cells (Wnt-11 high) cultured for 48 h in DMEM with 10% FCS (the media were previously depleted of EVs by overnight centrifugation at 110,000 × g according to Thery et al (Curr Protoc Cell Biol2006 Apr;Chapter 3:Unit 3.22.). Cell culture supernatant was centrifuged at 500 g for 10 min to remove cells and debris. The resultant supernatant was subjected to filtration through 0.22 µm pore filters, followed by ultracentrifugation at 10,000 g and 100,000 g for 30 min and 60 min, respectively. The resulting pellets were washed with PBS and again ultracentrifuged at 100,000 g for 60 min. The final pellet of EVs was resuspended in 1/1000^{th} of the original volume of the culture supernatant and stored at -80°C. Cryoelectron microscopy was carried out as described by Royo et al., 2017 (Scientific Reports volume 7, Article number: 42798 (2017)). Invasion assays were carried out for 24 hours using 50,000 C4-2B prostate cancer cells, as described by Gorrono et al. (Cancers (Basel). 2019 Jun 28;11(7):908). Cells were plated in RPMI containing 1% FCS in the upper chamber with the lower chamber containing RPMI and 20% FCS in the lower chamber), either in the absence or presence of 2 µl of VCaP cell EVs and 10 µg/ml control rat IgG or 10 µg/ml E8 anti-Wnt-11 antibody

### Magnetic nanoparticles (MNPs) functionalization

Magnetic nanoparticles (MNPs) used in this study were superparamagnetic iron oxide nanoparticles (SPIONs) nanomag^{®}-D-spio with the following surface functionalities COOH (Micromod, 79-02-201) or (Polyethylene glycol) PEG-COOH (Micromod, 79-56-201). For the conjugation using the Sulfo-SMCC linker (ProteoChem c1109), 5 ml of MNPs at 2.5 mg Fe/ml were pre-activated by incubating overnight at 37 °C with 50 µmol of cysteamine hydrochloride per g Fe (neutralized previously by 1 molar equivalent of sodium hydroxide (NaOH)), 150 µmol/g Fe of 1-Ethyl-3-(3-dimethylaminopropyl) carbodiimide (EDC) and 100 µmol/g Fe of n-hydroxysuccinimide (NHS). After 16 h, the sample was centrifuged and redispersed in Milli-Q water 3 times. For the conjugation, 150 µg of anti-Wnt-11 antibodies E8, F10, and rat IgG control per ml of MNP were used at 0.7 mg/ml in PBS. Antibodies were incubated with a 40 molar equivalents solution of Sulfosuccinimidyl-4-(N-maleimidomethyl) cyclohexane-1-carboxylate (Sulfo-SMCC) linker (ProteoChem c1109) for 30-40 min at room temperature (RT). After that, the modified antibody was purified by gel filtration through a desalting resin PD-10 (Sephadex G-25, Sigma). Activated MNPs were concentrated by centrifugation through 10 kDa cut-off Amicon filters (Merck-Millipore) and correspondent amounts were incubated with the SMCC modified antibodies at RT overnight. For the thiol-maleimide immobilization, 5 ml of MNPs at 2.5 mg Fe/ml were pre-activated by incubating 4 h at 37 °C with 250 µmol/g Fe of N-(2-Aminoethyl) maleimide, 500 µmol/g Fe of EDC and 500 µmol/g Fe of NHS. Antibodies diluted in HEPES (4-(2-hydroxyethyl)-1-piperazineethanesulfonic acid) buffer pH 8.3 were activated with 50 molar equivalents solution of 2-lminothiolane, also known as Traut's reagent, for 2 h at RT. After removal of excess reactants, activated antibodies and MNPs were incubated together for 2 h at room temperature and further incubated overnight at 10°C. Free maleimide groups were blocked with 250 µmol/g Fe of cysteine for 2 h at RT. After both conjugation procedures, the MNP-antibody conjugates were purified by gel filtration through a sephacryl S200HR (Sigma) column by elution in PBS. Finally, MNPs were washed several times with PBS and stored at 4 °C. They were tested within 1-month of conjugation, which is the estimated time the nanoformulations remain stable. For estimating the conjugation efficiency, the absorbance of consecutive elution fractions was measured at 280 nm in a NanoDrop ND-1000 spectrophotometer using the extinction coefficient estimated for the Ab sequence (210 000 M-1 cm-1). MNP sterilization was carried out by filtration through 0.22 µm Millex-GP filters (Merck-Millipore) and it was followed by sonication for 1 min for redispersion. The final concentration of iron as determined by inductively coupled plasma mass spectrometry (ICP-MS) to be of 2.5 mg Fe/ml in a total of 1- 2 ml with approximately 40 µg Ab per mg Fe. A concentration of 0.15-0.2 mg Fe/ml was used in functional assays. Hydrodynamic diameter and zeta potential were determined by dynamic light scattering (DLS) using a Zetasizer Nano-ZS device (Malvern Instruments) from dilute sample suspensions (0.1 mg Fe per ml) in water at pH 7.4.

### Gene reporter assay

For gene reporter assays, 100000 (293, HT1080) or 70000 (PC-3) cells per well were plated in triplicate in 12-well plates. After 24 h, cells were washed with PBS, incubated in OptiMEM and transfected using LTX/Plus reagents (Life Technologies), as instructed by the manufacturer. The plasmids used are presented in Table 2 and Table 3. 250 ng luciferase containing plasmid (ATF2, TOP-flash), 200 ng Wnt-11/Wnt-3a overexpression plasmids and 50 ng of renilla plasmid were used per well. For experiments with Wnt receptors and co-receptors the amounts of plasmids transfected per well was the following: 10 ng renilla, 50 ng ATF2-luc, 100 ng FZD8 (1D4, full length), 100 ng LRP6 (VSV-G, full length), 200 ng ofWnt-11 or Wnt-11m (untagged). 4 h after transfection cells media was removed and changed to fresh complete media and at this time treatments, when pertinent, were added. For Wnt-3a stimulation, the conditioned media (CM) collected from L-Wnt-3a cells and control parental cells were added 3-5 h post-transfection at the time of the media change, in a 1:1 dilution with fresh complete media. Gene reporter experiments were analyzed 24 h post transfection. Cells were washed in PBS and lysed using 130 µl/well of Passive Lysis Buffer (Promega). The Dual Glo Luciferase Assay System (Promega) or the Luciferase Assay Kit (PJK, Germany) were used according to manufacturer's instructions. Gene reporter activities were calculated as the luciferase:renilla ratio, triplicates were averaged and normalized to the internal control condition. Data correspond to at least 3 independent experiments.

### Gene silencing

Cells were plated to be 60% confluent at the time of transfection, washed in PBS and changed to OptiMEM (Gibco). RNAiMAX (Invitrogen) was used according to manufacturer's instructions. Briefly, both RNAiMAX and siRNA (small interfering RNA), were prediluted in OptiMEM for the subsequent incubation of 5 min at a 1:1 ratio of siRNA: RNAiMAX. siRNAs for WNT11 silencing (Wnt-11 SMARTpool, Dharmacon L009474-00-0005); as well as non-targeting siRNA pool control (Dharmacon D-001810-10-20) were used at a final concentration of 50 nM. The mixture was added to the cells for 3-5 h and then complete media was added on top in a 1:1 volume ratio. Next day media were renewed, and transfected cells were used for experiments. Generally, the extent of silencing was evaluated 48 h after transfection.

### Cell migration and invasion assays

50000 cells/well (C4-2B and PC3-m) and 20000 cells/ well of PC-3 were resuspended in RPMI with 0.5% FBS in duplicate uncoated or Matrigel-coated 8 µm pore transwell filters with a polycarbonate membrane (Corning) for migration and invasion assays, respectively. Antibodies tested were used at 10 µg/ml and added to the upper chamber, only considering its volume. In all cases, inserts were set in 24-well plates with media containing 20% FBS in the lower chamber. As a control for cell viability, cells were plated in parallel at the same density in 24-well plates. Migration and invasion were evaluated after 24 and 48 h, respectively. Non-migrating/invading cells were removed using a cotton swab and migrating/invading cells were stained using 0.1% crystal violet, 20% methanol, 0.36% PFA in PBS. Migrating/invading cells were imaged under a microscope using a 10X magnification lens by taking 5 pictures in predetermined fields for manual or automated cell counting. The control plate was also stained using crystal violet and absorbance measured at 595 nm after solubilizing in acetic acid (10%). These data were then used to normalize the numbers of migrating/invading cells to the numbers of viable cells.

### Wound healing assay

Cells were plated at high confluency in 6-well plates and the next day, two linear thin scratches (or wounds) in the confluent cell monolayer were performed with a 20-200 µl pipette tip. At that moment (time 0 h) pictures were taken in predetermined areas. After 8 h-16 h images of the cells filling the gap were taken again in the same areas. Images were processed using imaged for determination of wound closure using the MRI WoundHealing Tool ( https://github.com/MontpellierRessourceslmagerie/imagej_macros and scripts/wiki/W ound-Healing-Tool). Data are presented relative to the control condition or relative to complete closure, as indicated.

### Fluorescence activated cell sorting (FACS)

FACS analysis and sorting was performed in a FACSAria cytometer with FACSDiva software. For experiments involving protein analysis after FACS sorting, HEK 293 cells were transfected with Wnt-11 and antibody epitope mutant (Wnt-11m) plasmids. 24 h later, cells were detached with TrypLE (1x, Gibco) and pipetted onto 96-well V-shaped bottom plates for in-well staining. Cells were fixed in PBS/4% PFA (Santa Cruz) for 15 min, washed in PBS and permeabilized for 10 min at room temperature in 0.3% saponin (Sigma, 57900-25G). After 3 PBS washes, cells were blocked in 40% FBS in PBS for 30 min. Cells were then incubated with primary antibodies (Ab1 at 30 µg/ml and rat anti-Wnt-11 (R&D MAB3746) at 10 µg/ml, diluted in 1% bovine serum albumin (BSA, Sigma)/PBS for 30 min at 4 °C. After 3 PBS washes, cells were incubated for 30 min in the dark with secondary antibodies diluted 1:5000 in 1% BSA/PBS (anti-rat AlexaFluor647, Life Technologies). Cells were washed 3 times in PBS and transferred to 5 ml tubes prior to FACS sorting. Gates for the highest and lowest 10-20% staining intensities were set and those cells presenting high and low (+/-) staining were collected. Approximately 100000-200000 cells were retrieved for each condition and were then processed for protein analysis by western blot by directly lysing in preheated Laemmli buffer followed by incubation at 95 °C for 5 min. Bulk, unsorted cells were also retrieved and processed in parallel as control for the transient overexpression.

### RNA extraction and quantitative real-time PCR (Polymerase Chain Reaction)

Total RNA purification from cells was extracted using the NucleoSpin RNA (Macherey-Nagel GmbH, 740955-250) following the manufacturer's instructions). For RNA extraction from tumors growing on the chick CAM, tissues were broken up with a tissue homogenizer (Qiagen) for a few seconds and then lysed in 600 µl of RNA lysis buffer and RNA extracted using Illustra^{™} RNAspin Mini Isolation Kit (GE Healthcare) Equal amounts of total RNA from experimental samples (generally 2 µg), were retro-transcribed into complementary DNA (cDNA) using M-MLV Reverse Transcriptase and RNase OUT Ribonuclease Inhibitor (Invitrogen), according to manufacturer's instructions The retro-transcription reaction was then carried out in a thermocycler for 1 h at 37 °C followed by 5 min at 95 °C.

Quantitative-PCR was performed using PerfeCTa SYBR^{®} Green Supermix, Low Rox (Quanta, Barcelona, Spain) in a Viia7 Real-Time PCR System (Applied Biosystems, Madrid, Spain) with the following conditions: Taq polymerase activation 95 °C 3 min, denaturation 95 °C 15 s, annealing/extension 62 °C 1 min, melting curve 95 °C 15 s, 60 °C 1 min, 95 °C 15 s, 40 cycles. Relative levels of mRNA were determined according to the ΔΔCT method, relative to the housekeeping gene 36B4 (RPLP0). Primers are listed in **Table 5.**

**Table 5**

| **Gene** | **F/R** | **Sequence** | **Concentration** | | **Amplicon size** | |
|---|---|---|---|---|---|---|
| *36B4* | F | GTGTTCGACAATGGCAGCAT (SEQ ID NO:50) | | 300 | | 103 |
| *36B4* | R | AGACACTGGCAACATTGCGGA(SEQ ID NO:51) | | 300 | | |
| *LRP6* | F | TGAGGGTCTGCGTGAAATCC(SEQ ID NO:52) | | 300 | | 120 |
| *LRP6* | R | TGGGAACAACCCCCATTGTC(SEQ ID NO:53) | | 300 | | |
| *FZD8* | F | GCTCTACAACCGCGTCAAGA(SEQ ID NO:54) | | 900 | | 70 |
| *FZD8* | R | GCTGAAAAAGGGGTTGTGGC(SEQ ID NO:55) | | 900 | | |
| *WNT11 CDS* | F | TGAAGGACTCGGAACTCGTC(SEQ ID NO:56) | | 600 | | 119 |
| *WNT11 CDS* | R | GTCGCTTCCGTTGGATGTCT(SEQ ID NO:57) | | 600 | | |
| *WNT11 no-CDS* | F | CTCCTCCTGGGTGTGACC(SEQ ID NO: 58) | | 600 | | 175 |
| *WNT11 no-CDS* | R | CAGTGTTGCGTCTGGTTCAG(SEQ ID NO: 59) | | 600 | | |
| *AXIN2* | F | AAGTGCAAACTTTCGCCAAC(SEQ ID NO:60) | | 300 | | 122 |
| *AXIN2* | R | ACAGGATCGCTCCTCTTGAA(SEQ ID NO:61) | | 300 | | |
| *NKD1* | F | ACTTCCAGCCGAAAGTCGT(SEQ ID NO:62) | | 900 | | 90 |
| *NKD1* | R | CACCATAGGCCGAAGCAC(SEQ ID NO: 63) | | 900 | | |
| *LEF1* | F | AGCACGGAAAGAAAGACAGC(SEQ ID NO:64) | | 300 | | 92 |
| *LEF1* | R | TCGTTTTCCACCTGATGCAGA(SEQ ID NO:65) | | 300 | | |
| *DKK1* | F | ATGCGTCACGCTATGTGCT(SEQ ID NO:66) | | 900 | | 147 |
| *DKK1* | R | TCTGGAATACCCATCCAAGG(SEQ ID NO:67) | | 900 | | |
| *ATF2* | F | GATGTGGGCTGTGCAGTTTG(SEQ ID NO: 68) | | 300 | | 72 |
| *ATF2* | R | AGTCCTTTACCTCACCCAGAGT(SEQ ID NO: 69) | | 300 | | |
| *HIF2A* | F | GTCACCAGAACTTGTGC(SEQ ID NO: 70) | | 600 | | 249 |
| *HIF2A* | R | CAAAGATGCTGTTCATGG(SEQ ID NO: 71) | | 900 | | |
| *DVL2* | F | GCCCACTTTCTCCTACCAATACC(SEQ ID NO:72) | | 600 | | 129 |
| *DVL2* | R | CTCCGGCTGCCCTCACT(SEQ ID NO: 73) | | 600 | | |
| *SOX2* | F | TGCTGCGAGTAGGACATGCTGTAGG(SEQ ID NO: 74) | | 900 | | 207 |
| *SOX2* | R | GCACATGAACGGCTGGAGCAACG(SEQ ID NO: 75) | | 900 | | |

### Protein extraction and western blotting

For total cell extracts, cells were lysed for 10 min in RIPA (50 mM Tris-HCl, pH 7.4, 150 mM NaCl, 0.25% deoxycholic acid, 1% NP-40) with cOmplete^{™} EDTA-free Protease Inhibitor Cocktail (Roche), PhosSTOP Phosphatase Inhibitor Cocktail Tablets (Roche), and 0.1 % SDS (Life Technologies) and then centrifuged for 15 min at 14000 × g. Extracts were separated on SDS polyacrylamide gels using a Mini Protean System (BioRad). For detection of the DVL mobility shift 6% polyacrylamide gels were used for achieving the necessary doublet band separation. The protein content of the polyacrylamide gels was then transferred to 0.22 µm pore nitrocellulose membranes (Bio-Rad) using a Trans-Blot^{®} Turbo^{™} Transfer System (Bio-Rad, Turbo or High Molecular weight setting: 1.3 A constant, up to 25 V per gel during 7-10 min) with Bio-Rad transfer buffer containing 20% EtOH. After blocking with 3% BSA in TBS-T (Tris buffered saline, 0.1% Tween 20), blots were incubated with primary antibodies at 4 °C overnight (Table 1). After washing, blots were incubated for 1 h in blocking buffer with HRP-conjugated secondary antibodies diluted 1:20000 (Jackson ImmunoResearch). Membranes were developed using a developer (AGFA, Curix 60) or imaged using an iBright^{™} FL1500 Imaging System (Thermo). Quantification of band intensities was performed using imaged built-in functions.

### Immunoprecipitation

160000 PC-3M or 200000 HEK293 cells were plated in 6-well plates and transfected with 1 µg of total DNA (HEK293: 1:1 ratio for FZD8-1D4:Wnt-11-PA,; LRP6-Fc:Wnt-11-PA; PC3: 1:3 ratios for FZD8-1D4:Wnt-11-PA,; LRP6-Fc:Wnt-11-PA) . After 24 h, cells were washed twice with PBS and lysed in 1 ml of IP lysis buffer (50 mM Tris-HCl, pH 8, 150 mM NaCl, 1% Triton X-100, 1 mM EDTA (Millipore) with cOmplete^{™} EDTA-free Protease Inhibitor Cocktail (Roche) and PhosSTOP Phosphatase Inhibitor Cocktail Tablets (Roche)) for 5 min on ice. Samples were then centrifuged at 4 °C for 12 min at 15000 × g. For inputs, 40 µl of supernatant was combined with 2x Laemmli Buffer (Sigma) and heated for 10 min at 95°C. For IPs (for direct fc tag pull-down this step was skipped), supernatants were incubated for 90 min on ice with 1 µg of antibody (anti-PA or anti-1D4; Table 1). Protein A/G PLUS-Agarose (Santa Cruz) was added to each sample and incubated for 1 h on a rotating wheel at 4 °C. IPs were then collected by centrifugation at 500 × g for 30 s. Pellets were washed three times with IP buffer and once with ice-cold TBS. After washes, samples were re-suspended in 15 µl of 2× Laemmli Buffer and heated for 10 min at 95 °C. After that, the samples were subjected to SDS-PAGE electrophoresis for western blotting.

### Peptide ELISA

Clear polystyrene enzyme linked immunosorbent assay (ELISA) microplates (R&D systems, DY990) were coated with Wnt-11 peptides at 1 µg/ml by overnight incubation at 4 °C. Next day, several TBS washes were performed, and the plate was blocked overnight with TBS containing 0.1% Tween 1% BSA. Antibodies (Ab1 and rat IgG control) were tested by serial dilution (1 µg/ml, starting dilution 1:1000 as indicated) and incubated for 1.5-2 h at RT. Plates were washed twice with TBS containing 0.1% Tween 1% BSA. Anti-rat HRP-conjugated secondary antibody (1: 2000; Jackson ImmunoResearch) was incubated for 1 h at RT. Plates were washed three times with TBS containing 0.1% Tween 1% BSA. Signal was developed with 3,3',5,5'-Tetramethylbenzidine (TMB) ELISA high sensitivity kit (Abcam ab171523), and absorbance measured at 370 nm.

### Peptide dot blot

1 µg of peptide was dot blotted on nitrocellulose membranes and left to dry. The membranes were blocked in TBS-T containing 3% BSA. The subsequent steps were as for western blotting, starting from the blocking step and incubation was done with Ab1 and Ab2 antibodies from hybridoma cells conditioned media.

### Cell supernatant dot blot

HEK 293 and HT1080 cells were transfected and cultured in 5% FBS containing media for 48 h prior to cell supernatant or conditioned media (CM) collection. CM was centrifuged at 500 g for 10 min and filtered through a 0.22 µm membrane to remove any cell debris. From a total of 1 ml cleared CM, 100 µl was blotted on a nitrocellulose membrane using a concentrator manifold system (MinifoldTM). After complete drying of the membrane for at least 2 h, it was blocked in TBS-T buffer containing 3% BSA and analyzed by western blotting starting from the blocking step.

### Immunofluorescence (IF)

Cells were plated on 12 mm sterile coverslips (VWR, 0111250) in 24-well or 12-well plates. If appropriate, cells were transfected by following the procedure described above. For immunofluorescence (IF) staining, cells were washed in PBS and fixed at RT in PBS containing 4% PFA (Santa Cruz) for 15 min followed by 3 washes in PBS. Permeabilization was done for 10 min at RT in PBS containing 0.1% Triton X-100 (this step was skipped for cell surface staining of antigens, referred to also as non-permeabilized staining). Samples were blocked for 30-60 min in 2% BSA, 50 mM glycine, 0.01% NaN3 in PBS (IF blocking buffer). After that, coverslips were incubated overnight at 4 °C with primary antibodies diluted in IF blocking buffer (Table 1). Coverslips were then washed 3 times in PBS and incubated with secondary antibodies diluted 1:500 in IF blocking buffer for 40 min at RT (AlexaFluor488, AlexaFluor594, AlexaFluor647, Life Technologies). Coverslips were washed in PBS for 3-5 times before a final wash in ddH2O. Mounting the coverslips onto slides (Thermo 20 mm frosted) was performed by placing them cell- side down on a drop of 5-10 µl Vectashield mounting medium with DAPI (Vector Labs), leaving them to dry for 1 h and then stored at 4 °C. Immunofluorescence was visualized using an upright fluorescent microscope (Axioimager D1) and a confocal microscope (Leica TCS SP8) with a 20x objective and a 63x oil immersion objective. Co-localization analyses of LRP6 and Wnt-11/AE were performed using Fiji from the indicated numbers of cells corresponding to 2 independent experiments.

### Prussian blue staining

Prussian blue histochemical reaction for the demonstration of iron was performed using a kit (Polyscience, 24199) and according to manufacturer's instructions. Samples were incubated with a 1:1 mixture of 4% potassium ferrocyanide and 4% hydrochloric acid (Prussian blue staining solution) for 20 min at RT, renewing the Prussian blue staining solution once after the first 10 min. They were then washed with PBS three times. Counterstaining was done with Nuclear Fast Red for 2 min at RT to stain the cytoplasm, followed by washing thoroughly in PBS. After that, samples were mounted accordingly and imaged using an Axiolmager D1 light microscope. IHC tumor tissue specimens were processed for Prussian Blue staining after the fixed specimens had been paraffin-embedded, sectioned, placed onto slides and de-paraffinized as described in the section above. After Prussian blue reaction, specimens were dehydrated through ascending alcohols to xylene and mounted with DPX mounting media (Sigma). For the MNP cell-staining assays, a modified IF protocol was followed. Briefly, cells were seeded on coverslips placed into 24-well plates. MNP conjugates were incubated at 0.2 mg Fe/ml for 2-4 h at 37 °C or at 4 °C (control). Cells were then washed twice with PBS and fixed with 4% PFA for 10 min at RT. Cells were then washed again twice with PBS and subjected to the Prussian Blue histochemical reaction described above. Samples were mounted with Vectashield without DAPI (Vector Labs).

### Immunohistochemistry (IHC)

Formalin/EtOH fixed specimens were paraffin-embedded and 5 µm sections were cut, placed onto slides. Histo-Clear II (National Diagnosis, USA) was used for de-paraffinization, followed by four changes of 100%, 96%, 70% EtOH and H2O. Antigen retrieval was conducted for 30 min in a vapor cooker filled with Tris EDTA buffer at pH 9.0 for Ki67 staining and with citrate buffer pH 6.0 for Wnt-11 staining. Endogenous peroxidase activity was quenched by incubation for 10 min in 3% H2O2. Then, avidin/biotin were blocked for 15 min each (avidin/biotin blocking kit (Vector Labs)). After PBS washes, specimens were blocked in 5% horse serum for 30 min at RT. Primary antibody to Ki67 was incubated 1 h RT and additionally overnight at 4 °C and primary antibody to Wnt-11 was incubated overnight at 4 °C (Table 1). After PBS washes, sections were incubated for 30 min with biotinylated secondary antibody (antimouse/rabbit/goat IgG Antibody (H+L), Universal Pan-Specific biotinylated BA-1300, Vector Labs) followed by Vectastain^{®} Elite ABC reagent (Vector Labs) for 30 min. 3,3'-diaminobenzidine (DAB) (DAKO) reagent was used as the chromogenic developing reagent for 1-2 min and then, sections were counterstained with Mayer's hematoxylin. Samples were then dehydrated through ascending alcohols to xylene and mounted with dibutyl-phthalate polystyrene xylene (DPX) mounting media (Sigma). Specimens were imaged on an Axiolmager D1 light microscope. QuPath software was used for quantitation of intensity of nuclear Ki67. For hematoxylin/eosin (H&E) staining, specimens were only deparaffinized and rehydrated, then stained with consecutive Mayer's hematoxylin and eosin solutions followed by dehydration and DPX-mounting.

### Chick chorioallantoic membrane (CAM) assay

Fertilized chicken eggs were purchased from Gibert (Tarragona). Upon arrival, the surfaces of the eggs were cleaned superficially with 70% ethanol. Eggs were then placed into a wine refrigerator and maintained at 12 °C usually for 2 days, but up to 1 week. At embryo development day (EDD) 0 the eggs were placed with the pointed end facing down in the rotary incubator set at 37 °C, 60% humidity and with rotation of 100 degrees every 20 min. On EDD 4 a hole was made with the help of a Dremel tool and a syringe (Braun Sterican) in the pointed end of the eggs to induce the dropping of the developing CAM and its separation from the outer shell (referred to as dropped CAM). Holes were covered with tape and eggs placed in a stationary incubator (37 °C, humidity 60-80%). On EDD 7, holes were enlarged with the help of forceps to a ~ 1 cm diameter window to expose the CAM. Plastic rings cut from plastic Pasteur pipettes of 5-6 mm diameter were placed on the CAMs. For the implantation of cells, they were detached with TrypLE (1x, Gibco), counted, and resuspended at a density of 500000 PC3 cells/egg in a volume of 12.5 µl/egg of media 0% FBS and 1% Pen/Strep antibiotics (Gibco). Treatments were added where pertinent, and the suspension was mixed 1:1 (12.5 µl/egg) with Matrigel matrix growth factor reduced (BD Biosciences, 356230). Then, the 25 µl/egg of the cell: Matrigel mixture was implanted by placing it drop-wise into the plastic ring. Tumors were allowed to form and grow for 5-7 days. Antibodies were added to the cell: Matrigel implantation mixture at 3 µg/egg and then tumors received topical doses every 2-3 days with 3 µg antibody/egg.

On endpoint (EDD 12-14), holes were enlarged for imaging tumors in ovo and excised for weight determination in a precision balance (Sartorius TE1245). Tumor area was established as a ratio of tumor area to ring using in imaged. When tumor specimens were processed for IHC, they were fixed overnight in 10% formalin solution (Merck, HT5012) and subsequently changed to EtOH 50% for up to two weeks and specimens were then paraffin embedded for IHC analysis. 5-10 eggs were used per condition in each experiment and data were normalized to the IgG-treated condition for combining at least 3 independent experiments. Data are presented using the SuperPlotsOfData webtool52 (https://huygens.science.uva.nl/PlotsOfData/) showing individual datapoints for each tumor grouped by each experiment average represented as a bigger color-coded point, lines between conditions representing paired data and overlapping mean and SD bars in black. CAM assays are exempt from ethical approval.

Metastasis was established by retrieval of the distal CAM and detection of human DNA by Alu sequences as described before Crespo, P., & Casar, B (2016). The Chick Embryo Chorioallantoic Membrane as an in vivo Model to Study Metastasis. Bio- Protocol, 6(20).

### Body Weight Measurement and Autopsy-imaging assay

Body weight of the animals was measured twice per week. Orthotopic tumor growth and possible metastasis were monitored weekly by bioluminescent imaging. At the conclusion of the study, each animal was euthanized and ex vivo autopsy-imaging performed using BLI for examining possible metastasis in lymph nodes, lung, liver, spleen, colon, and kidneys.

Data related to tumor volume, tumor weight, and body weight presented as mean and the standard error of the mean (SEM). Statistical analyses were conducted using Student's t-test. P< 0.05 is considered statistically significant. * and ** indicate P< 0.05 and P< 0.01, respectively.

At the end of the study, the prostates, lymph nodes and other organs were collected, the tumors were weighed, the lymph nodes and organs were photographed, fixed with formalin and made as FFPE blocks. Immunohistochemistry for Ki67 was carried out in the same way as for the CAM tumors.

### Bioinformatic tools and resources

Protein structures were retrieved from the RCSB Protein Data Bank (PDB; http://www.rcsb.org/pdb/; PBD IDs: 6AHY, 4F0A, 4QCI, 1IGT) and analysis and molecular graphics were done using UCSF Chimera (J. Comput. Chem. 25, 1605-1612 (2004)).

Protein sequences were retrieved from the UniProt database https://www.uniprot.org alignments were performed using MUSCLE55 with default parameters and visualized with Jalview (Waterhouse, Procter, Martin, Clamp, & Barton, 2009), Jalview Version 2 - a multiple sequence alignment editor and analysis workbench. Bioinformatics 25(9):1189-91; doi: 10.1093/bioinformatics/btp033.

The conservation score provided in the alignment is given as an index reflecting the conservation of physico-chemical properties accompanied by a histogram providing the score for each column. Conserved columns (score of 11) are indicated by '*', and columns with property-conserving mutations are marked with a '+' (score of 10).

Basic image analysis was carried out using ImageJ's (NIH, Bethesda, MD, USA) Fiji distribution56. Homemade macros were used for automated cell number quantification, area determination and fluorescence image mounting. Co-localization tests were done using the coloc2 plugin (https://imagej.net/Coloc2).

Qupath software (Bankhead, P. et al Sci. Rep. 7, 1-7 (2017)) was used for the analysis of IHC images. For Ki67 nuclear intensity quantification the following method was used. Briefly: images were imported to a Qupath project and tumor area was selected, automated cell segmentation tool based on hematoxylin staining was used with manual supervision and DAB mean intensity was obtained for each of the segmented nuclei. Data of the nuclei from each picture were averaged and statistically analyzed as independent as described below.

### Statistical analysis

Data presented are the result of 3 independent experiments, unless stated otherwise. When data shows the fold-change relative to the internal control it is indicated in the y-axis label as relative. Data were handled on Microsoft Office Excel spreadsheets, aided by the use of GraphPad Prism 5.0 (GraphPad, La Jolla, CA, USA), PlotsOfData and SuperPlotsOfData webtools (Postma & Goedhart, 2019) (https://huygens.science.uva.nl/PlotsOfData/) https://www.molbiolcell.org/doi/10.1091/mbc.E20-09-0583. Data are represented as bar plots with the mean ± standard error of the mean (SEM) indicated by error bars. For CAM experiments plots show replicates measurements grouped by experiment and mean and error bars are indicated. For determination of statistically significant differences, pairwise comparisons were analyzed using two-tailed Student's t tests. For normalized or relative data unequal variances were assumed, and heteroscedastic t-tests were calculated; for paired observations, paired t tests were performed. In boxplots p-values are calculated for the pairwise comparison of the different experiments. Large group comparisons (>6) were analysed using one-way analysis of variance (ANOVA) and p-values were then obtained as post-hoc Tukey corrected p-values. A p-value<0.05 was considered statistically significant: * is indicative of p<0.05; ** of p<0.01; *** of p<0.001; and n.s. notation was used to indicate non-significant differences; specific p-values may be noted on the figure legend.

### Example 1- Antibody-mediated inhibition of Wnt-11 signaling reduces prostate cancer cell sphere formation and tumor growth.

Monoclonal antibodies were raised in rats to a mixture of peptides corresponding to unique and potentially antigenic regions on human Wnt-11 (Fig.1a). After initial screening, two antibodies (Ab1 (E8) and Ab2 (F10)) were selected for further analysis. Dot blot experiments found that both antibodies recognize the same peptide, confirmed by ELISA for Ab1 (Fig. 6a, b). Immunofluorescence analysis found strong binding of Ab1 to VCaP cells but not to C4-2B cells (Fig. 1b), consistent with their high and low expression of WNT11, respectively (Fig. 6c). In addition, ectopic expression of Wnt-11 in C4-2B cells conferred increased binding of Ab1 with the same staining pattern as that of a commercial Wnt-11 antibody (Fig.1c, Fig. 6d). To determine if the antibodies affected Wnt-11 signaling, western blotting was used to examine their effects on DVL proteins, whose activation by phosphorylation results in reduced electrophoretic mobility. In control PC3 cells, DVL2 and DVL3 migrated as doublets, with the relative intensities of the upper bands providing an indication of endogenous Wnt signaling activity. The upper band intensities were increased by Wnt-11 expression and reduced by treatment of cells with the porcupine inhibitor Wnt-C59, which blocks Wnt secretion (Fig.1d). Both anti-Wnt-11 antibodies significantly reduced the intensity of the upper band, compared to IgG control, consistent with inhibition of Wnt signaling (Fig.1d, Fig. 6e). DVL2 mRNA expression levels were unaffected by anti-Wnt-11 Ab1 antibody or WNT11 silencing (Fig. 6f).

Next, experiments were carried out to determine the effects of the Wnt-11 antibodies on prostate cancer cell growth. The antibodies had modest effects on the proliferation of PC3 and DU145 when cells were cultured as monolayers (Fig. 6g), consistent with WNT11 gene silencing studies. In contrast, the antibodies reduced sphere formation by PC3, PC3-M and DU145 cells (Fig. 1e), suggesting that cells are more dependent on Wnt-11 in this context. Consistent with this, WNT11 mRNA and Wnt-11 protein levels were found to be higher in cells cultured as spheres, compared to in monolayers (Fig. 6h). Finally, chick embryo chorioallantoic membrane (CAM) assays were used to test the effects of the antibodies on prostate cancer cell tumor growth (Fig. 1f). Ab1 treatment significantly reduced the size and weight of tumors formed by PC3 cells, compared to control IgG (Fig. 1g). The effect of Ab1 on tumor cell proliferation was demonstrated by quantitative immunohistochemistry of Ki67, which showed significantly lower nuclear staining intensity in Ab1-treated tumors, compared to control tumors (Fig. 1h).

### Example 2-Antibody-mediated inhibition of Wnt-11 signaling limits prostate cancer cell migration, invasion and metastasis.

Previous studies have found Wnt-11 plays roles in cancer cell migration. Accordingly, WNT11 gene silencing reduced PC3 cell migration in wound healing assays (Fig. 7a), consistent with previously reported results from Boyden chamber assays. The anti-Wnt-11 antibodies reduced PC3 cell migration in wound healing assays (Fig. 2a) and in Boyden chamber assays (Fig. 2b). Similar results were observed using DU145 cells (Fig. 2c), although Ab2 was less effective. Wnt-11 levels are increased by hypoxia in some cancer cell lines, and this promotes hypoxia-dependent migration. In accordance with that study, we observed increased expression of WNT11 mRNA in PC3 cells treated with the hypoxia mimetic dimethyloxalylglycine (DMOG) (Fig.2d). Expression of FZD8 mRNA, which is required for PC3 cell migration, was also upregulated, and there was a trend for increased expression of ATF2 mRNA, which is required for non- canonical Wnt signaling. In contrast, there were mixed effects on genes involved in Wnt/b- catenin signaling (Fig. 7b). DMOG treatment of PC3 cells increased the levels of hypoxia markers HIF1a and HIF2a, as expected, and dramatically increased Wnt-11 protein levels (Fig.2e). PC3 cells pre-treated with DMOG were subjected to Boyden chamber migration assays in an atmosphere with 1% oxygen to maintain the hypoxic state. Hypoxic cells migrated to a greater extent than control cells, and Wnt-11 antibody Ab1 attenuated this effect (Fig. 2f) without affecting proliferation (Fig. 7c).

Given the role of Wnt-11 in cancer cell invasion, Boyden chamber assays were also used to test the effects of the anti-Wnt-11 antibodies in this setting, with both antibodies found to reduce invasion of PC3-M cells (Fig. 2g and Fig. 7d). Moreover, stable expression of Wnt-11 in C4-2B cells increased their capacity for invasion, and this was reduced by treatment with the anti-Wnt-11 antibodies (Fig. 2h). Given the effects of the Wnt-11 antibodies on prostate cancer cell invasion, experiments were carried out to test for effects on metastasis. Here, we used an in ovo spontaneous metastasis assay, where tumor cells are grown on the upper CAM of the uncracked egg and cancer cells metastasize to the lower CAM, where they can be detected 5 days after tumor cell inoculation (Fig. 1f). Ab1 significantly reduced the numbers of cells metastasizing to the distal CAM in this assay (Fig. 2i).

### Example 3- Mutation of Wnt-11 at the antibody recognition site impairs Wnt-11 signaling.

The anti-Wnt-11 antibodies were raised to the same peptide in the sequence of Wnt-11, LLDLERGTRESA (Fig. 6a). The structure of Wnt-11 predicted by AlphaFold was used to visualize the location of this sequence in a structural context. The overall predicted structure, which is similar to the crystal structures of xWnt823 and human Wnt-3, shows the first part of the peptide (LLDLERGT) forms a short alpha-helical loop on the Wnt-11 surface, with the remainder (RESA) partially buried and forming the first part of a core alpha helix (Fig. 3a, Fig. 8a). To determine the importance of this region in Wnt-11, a mutant was generated in which the sequence LLDLERGT was deleted (Wnt-11^{Δ101-108} (Wnt-11m)). Western blotting of cell extracts (Fig. 3b) and dot blot analysis of conditioned media (CM) (Fig. 3c) from cells transfected with plasmids expressing PA-tagged wild-type Wnt-11 or Wnt-11m, found both proteins to be similarly expressed and secreted. In addition, immunofluorescence analysis of non-permeabilized cells found similar levels of transfected wild-type Wnt-11 and Wnt-11m on the cell surface (Fig. 3d). Together, these results suggest that deletion of the region containing the Wnt-11 antibody epitope does not affect Wnt-11 stability or its folding, which is necessary for secretion. This would be consistent with the AlphaFold predicted structure of Wnt-11m, which is similar to that of Wnt-11 (Fig. 8b), with the caveat that AlphaFold may not predict destabilizing mutations accurately. To confirm that the deletion prevented Ab1 recognition of Wnt-11, HEK 293 cells transfected with Wnt-11 or Wnt-11m were stained using Ab1 and sorted by FACS (Fluorescence Activated Cell Sorting). Extracts from Ab1^{high} and Ab1^{low} FACS-sorted cell populations were probed by western blotting using a commercial anti-Wnt-11 antibody recognizing a sequence outside the deleted region. While the Ab1^{high} population from Wnt-11-transfected cells was enriched for Wnt-11, this was not the case for Wnt-11m-transfected cells (Fig. 3e and Fig. 8c), consistent with Ab1 not recognizing Wnt-11m.

To determine if the mutation in Wnt-11 affected its ability to signal, extracts from cells transfected with wild-type Wnt-11 and Wnt-11m were probed for DVL3. This revealed that Wnt-11m was significantly impaired in its ability to induce a DVL3 mobility shift (Fig. 3f). Wnt-11 does not normally activate canonical (b-catenin/Tcf-dependent) Wnt signaling, and in some settings, it inhibits the activity of canonical Wnts, such as Wnt-3a. We therefore carried out gene reporter assays to measure this inhibitory activity. Wnt-11m was significantly impaired in its ability to repress Wnt-3a activation of b-catenin/Tcf signaling (Fig. 3g). In prostate cancer cells, Wnt-11 functions in concert with FZD8 to activate ATF2-dependent signaling. Gene reporter assays found that Wnt-11 and Wnt-11m similarly increased ATF2-dependent transcription but Wnt-11m was impaired in its ability to cooperate with FZD8 (Fig. 3h). To determine if these impairments in Wnt-11m signaling had a physiological outcome in cells, cell migration assays were carried out using PC-3M cells, whose migration is increased by ectopic expression of Wnt-11. Transfection of Wnt-11 but not of Wnt-11m enhanced PC-3M cell migration (Fig. 3i and 8d) (there were no effects of Wnt-11 or Wnt-11m on proliferation (Fig. 8e). In summary, deletion of the antibody epitope sequence LLDLERGT (SEQ ID NO: 2) in Wnt-11 impairs its ability to activate DVL, inhibit Wnt-3a activation of b-cateninΓΓcf signaling, cooperate with FZD8 to activate ATF2 signaling and promote PC-3M cell migration.

### Example 4- Mutation of Wnt-11 at the antibody recognition site impairs Wnt-11 interaction with LRP6 and LRP6 signaling.

The predicted structure of Wnt-11 indicates that the antibody recognition site is distant from the FZD binding site (Fig. 4a). It is, however, close to a region identified in the xWnt8 structure named pseudo-site 3, which was speculated to mediate asymmetric Wnt/Fz dimerization and thus be involved in ligand-induced receptor clustering and signalosome assembly and also close to a region proposed in other studies to be involved in Wnt binding to LRP6. To address the possibility that the antibody recognition site might be involved in such interactions, co- immunoprecipitation assays were carried out using epitope-tagged forms of Wnt-11, Wnt-11m and the Wnt receptors FZD8 and LRP6. Wnt-11 and Wnt-11m associated to the same extent with the extracellular cysteine-rich domain (CRD) of FZD8 (Fig. 4b, Fig 9a), consistent with the site of the deletion being far from the FZD-binding sites. In contrast, LRP6 bound less efficiently to Wnt-11m than to wild-type Wnt-11. Reduced binding of Wnt-11m was observed in experiments using full-length LRP6 (Fig. 4c) and using the extracellular domain of LRP6 (Fig. 4d). These results were further confirmed in PC3 cells using untagged Wnt-11 constructs (Fig. 4e). Quantitation of western blots from multiple experiments indicated Wnt-11m binding to LRP6 was 50-70% of that observed for wild-type Wnt-11 (Fig 9b). In accordance with these results, immunofluorescence analysis found that the extent of colocalization of Wnt-11m with LRP6 was significantly lower than that for wild-type Wnt-11 (Wnt-11m, Pearson 0.4, Spearman 0.46; Wnt- 11, Pearson 0.6, Spearman 0.67) (Fig. 4f, g).

Gene reporter assays were carried out to determine the effects of the antibody epitope mutation on the ability of Wnt-11 to regulate LRP6-dependent signaling. Ectopic expression of LRP6 activated b-catenin/Tcf-dependent transcription and this was inhibited by Wnt-11 but not by Wnt- 11m (Fig. 4h), consistent with the observed reduction in binding of Wnt-11m to LRP6. We previously found that ectopic expression of LRP6 resulted in a small increase in ATF2-dependent gene reporter activity in HEK 293 cells but had no effect in L or L-Wnt-3a cells. Studies in Xenopus and mouse have found Lrp5 and Lrp6 inhibit noncanonical Wnt signaling. In PC3 cells, ectopic expression of LRP6 did not affect ATF2-dependent transcription or the response to Wnt-11 (Fig 9c). To exclude the possibility that the high endogenous LRP6 levels in PC3 cells (Fig. 9d) prevented detection of a response, experiments were carried out using LRP5/6-/- HEK 293 cells. In this case, LRP6 increased ATF2-dependent transcription (Fig. 4i, Fig 9e). We therefore compared the activities of Wnt-11 and Wnt-11m in these cells. In the absence of LRP6, expression of Wnt-11 or Wnt-11m with FZD8 similarly increased ATF2-dependent transcription (Fig. 4i). In contrast, when LRP6 expression was co-expressed, Wnt-11, but not Wnt-11m, further increased gene reporter activity (Fig. 4i). These results suggest that reduced binding of Wnt-11m to LRP6 indirectly affects noncanonical Wnt-11/FZD8 signaling.

### Example 5- Mutation of Wnt-3a at the site equivalent to the Wnt-11 target site impairs Wnt-3a- dependent signaling

Alignment of human Wnt family protein sequences shows low conservation in the region corresponding to that targeted by Wnt-11 function-blocking antibodies (Fig.5a).
**Wnt-11 (SEQ ID NO: 3)**
Wnt-1 (SEQ ID NO: 115)
Wnt-2 (SEQ ID NO: 116)
Wnt-2B (SEQ ID NO: 117)
Wnt-3 (SEQ ID NO: 118)
Wnt-3A (SEQ ID NO: 119)
Wnt-4 (SEQ ID NO: 120)
Wnt-5A (SEQ ID NO: 121)
Wnt-5B (SEQ ID NO: 122)
Wnt-6 (SEQ ID NO: 123)
Wnt-7A (SEQ ID NO: 124)
Wnt-7B (SEQ ID NO: 125)
Wnt-8A (SEQ ID NO: 126)
Wnt-8B (SEQ ID NO: 127)
Wnt-9A (SEQ ID NO: 128)
Wnt-9B (SEQ ID NO: 129)
Wnt-10A (SEQ ID NO: 130)
Wnt-10B (SEQ ID NO: 131)
Wnt-16 (SEQ ID NO: 132)

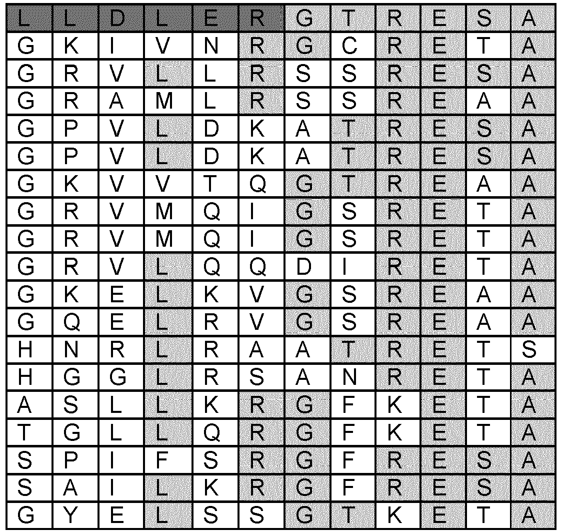

To determine if this region also plays a role in activation of canonical Wnt signaling, the equivalent deletion was made in Wnt-3a, removing the sequence GPVLDKAT (SEQ ID NO: 25) (Wnt-3a^{Δ99-106}) to make Wnt-3a_m.

| | | |
|---|---|---|
| 3A(wt) | DSLAIFGPVLDKATRESA | **SEQ ID NO: 133** |
| 3A_m | DSLAIF--------RESA | **SEQ ID NO: 134** |
| 3A_11ins | DSLAIFLLDLERGTRESA | **SEQ ID NO: 135** |

In addition, this sequence was replaced by the equivalent Wnt-11 sequence (LLDLERGT (SEQ ID NO: 2)) to make Wnt-3a_11ins). Analysis of HEK 293 cells found similar levels of expression of wild-type Wnt-3a and the two Wnt-3a mutants in transfected cell extracts (Fig. 5a) and on the cell surface (Fig.5b). Analysis of CM from transfected cells also found that Wnt-3a and the Wnt-3a mutants were similarly secreted by HT1080 (Fig. 5c) and HEK 293 cells (Fig. 10a), albeit to a lesser extent in HEK 293 cells. Western blot analysis of DVL3 from transfected HT1080 cells found that Wnt-3a_m was impaired in its ability to induce a shift in DVL3 (Fig. 5d and Fig. 10b), as was also observed for Wnt-11m in HEK 293 cells (Fig. 1f). In contrast, Wnt-3a_11ins induced a shift in DVL3 similar to that for wild-type Wnt-3a (Fig. 5d and Fig. 10b). However, neither Wnt-3a mutant activated b-catenin/Tcf-dependent transcription in HEK 293 cells (Fig. 5e). Given the lower levels of Wnt-3a_m secretion in HEK 293 cells (Fig. 10a), we also compared the activities of the mutants using HT1080 cells expressing the fluorescent protein Venus under the control of a b-catenin/Tcf- responsive promoter. FACS analysis of transfected cells found that the activity of Wnt-3a_m was severely impaired but Wnt-3a_11ins retained some activity (Fig. 5f). In summary, the sequence deleted in Wnt-3a appears to be more important for b-cateninΓΓcf signaling than for DVL3 activation, consistent with the results for Wnt-11, where the mutation affects binding to LRP6 rather than to FZD8.

Canonical signaling activity in the presence of Wnt-3a_m appeared to be lower than that in control vector-transfected cells (Fig. 5f), suggesting this mutant interferes with endogenous Wnt signaling, for example by competing with endogenous Wnt ligands for binding to Wnt receptors. To test this possibility, co-transfection experiments were carried out using Wnt-1 and Wnt-3, both of which activate b-cateninΓΓcf signaling but by binding to different domains on LRP6 (E1E2 and E3E4, respectively). As expected, wild-type Wnt-3a and Wnt-1 had additive effects on gene reporter activity (Fig. 5g), consistent with their binding to different domains on LRP6. Wnt-3a_m did not affect Wnt-1-dependent transcription, suggesting it does not act in a dominant-negative manner in this context. In contrast, Wnt-3a and Wnt-3 did not have additive effects (Fig. 5g), consistent with their binding to the same region on LRP6. Moreover, Wnt-3a_m inhibited the responses to Wnt-3 and to wild-type Wnt-3a, indicating it has a dominant effect only on signaling by highly related Wnt family members. The lack of effect of Wnt-3a_m on Wnt-1 signaling might reflect differences their binding to FZD receptors or to different domains on LRP6. The latter would be consistent with the results for Wnt-11, where the equivalent deleted region affects binding to LRP6.

### Example 6- Wnt-11 antibody E8 inhibits C4-2B cell invasion promoted by VCaP cell derived positive extracellular vesicles (EVs)

Cryoelectron microscopy of EVs prepared from VCaP cell culture supernatant confirmed the presence of CD81-positive EVs. Western blotting using goat anti-Wnt-11 antibody (1:1,000, R&D systems) indicated the presence of Wnt-11 in VCaP cell EVs, as well as in cell extracts. Cell invasion assays found that in the absence of VCaP EVs, there was no significant difference in invasion when C4-2B cells were treated with control IgG or E8 antibody. Treatment with VCaP cell EVs increased C4-2B cell invasion in the presence of control IgG. In contrast, in the presence of the same amount of E8 Wnt-11 antibody, the VCaP EVs did not increase C4-2B cell invasion (Fig. 11). These results are consistent with the E8 antibody inhibiting C4-2B cell invasion driven by Wnt-11-positive VCaP cell EVs.

### Example 7- Wnt-11 antibody nanoparticles inhibit PC3 and PC3m cell migration

To improve the properties of the anti-Wnt-11 antibodies in the context of *in vivo* delivery and imaging, functionalization of superparamagnetic iron oxide nanoparticles (SPIONs), here referred to as magnetic nanoparticles (MNPs), was carried out with the anti-Wnt-11 antibodies.

In a parallel testing to that of the anti-Wnt-11 antibodies, the anti-Wnt-11 MNP conjugates were characterized for their effects on PC3 cell proliferation and migration. Conjugation, even when efficient, resulted in the dilution of the active antibodies. This was accounted for by increasing the concentration of the conjugated-antibody treatments for the assays, without exceeding the maximum tolerated dose of iron of 0.2 mg Fe/ml (Aires, Cadenas, Guantes, & Cortajarena, Nanoscale, 9(36), 13760-1377 2017; Aires et al Nanotechnology, 27(6)., 2016), which was the concentration used for experiments (corresponding to approximately 7 µg/ml of antibody).

As previously reported for other cell models (Aires et al., 2016 cited supra; Trabulo et al., Biochimica et Biophysica Acta - General Subjects, 1861(6), 1597-1605 2017), the iron oxide nanoparticles alone (MNP-C) did not have an effect on PC3 cell proliferation in 5-7-day assays (Figure 12b). This was also the case for IgG, E8 and F10 conjugates in accordance with previous results using naked antibodies, where proliferation was only affected at high doses (20 µg/ml,).

To measure the effects of the antibody conjugates in migration assays, experiments were carried out in media containing 5% FBS to avoid the aggregation of the nanoparticles observed in low serum conditions. Because these conditions could be incompatible with migration and invasion assays, which are performed in media containing 0.5% FBS, wound healing assays were carried out instead. A range of time points (24 h, 48 h) were used with the first batch of MNPs (2018), but only a pilot experiment could be performed (Figure 32b, n=1). Later, a short time point (8 h) was decided as optimal for testing the latest MNP batch (2021) to avoid confounding variables (e.g., possible reduced cell proliferation). A consistent reduction in the migration of cells treated with MNP-E8 compared to MNP-IgG control was determined (Figure 12b, c, d). In addition, MNP-F10 conjugates showed a non-significant trend for a similar inhibitory effect.

In addition, experiments were carried out in the same way as those using unconjugated antibodies. E8- and F10-MNPs significantly reduced PC3m cell migration at and above 0.1 mg Fe/ml (Fig. 12e).

### Example 8 - Effect in vivo using chick CAM assays

CAM assays were used to test the activity of the anti-Wnt-11 magnetic nanoparticle (MNP) formulations in vivo on PC3-GFP cells. MNP treatments were performed as for naked antibodies but using lower equivalent amounts of MNP-conjugated antibodies. The MNPs and antibody MNPs were not toxic at the doses used. There were no significant differences for tumor growth among untreated, MNP-C-treated and MNP-IgG-treated cells. For MNP-F10, there was a trend for a reduction in tumor growth, and for MNP-E8 there was a significantly reduction in tumor weight and area compared to MNP-IgG.

Immunohistochemical analysis of MNP-treated tumor sections found a reduction in nuclear Ki67 staining in MNP-E8-treated tumors, compared to MNP-IgG-treated (Figure 13). MNPs are partially visible as a light grey stain in non-DAB-stained sections, due to their iron content, and this could be also distinguished in Ki67 (DAB) stained sections. Prussian Blue staining also showed accumulation of MNPs at the site of the tumors, consistent with MNPs being applied topically, with some MNP penetration into the bulk of the tumors.

### Example 9- Enhancement of inhibitory effects of an anti-androgen on prostate cancer cell proliferation

Previous studies have shown that inhibition of WNT secretion by silencing of the Wntless gene (WLS) sensitizes enzalutamide-resistant 22Rv1 prostate cancer cells to enzalutamide treatment (Lombard et al., Am J Clin Exp Urol 2019; 7:203-214). 22Rv1 cells are derived from a xenograft that was serially propagated in mice after castration-induced regression and relapse of the parental, androgen-dependent CWR22 xenograft (Sramkoski et al., 1999, 10.1007/s11626-999-0115-4). The authors previously showed that WNT11 gene expression is elevated in castrate-resistant CWR22 tumour xenografts (Zhu et al., 2004, Cancer Research, 64(21), 7918-7926). Therefore, to determine if Wnt-11 plays a role in castrate-resistance of 22Rv1 cells, cell proliferation assays were carried out in the presence of Wnt-11 antibodies and the anti-androgen enzalutamide.

Crystal violet assays were used to compare the proliferation of 22Rv1 prostate cancer cells treated with vehicle (DMSO), control IgG (10 ug/ml), Wnt-11 E8 antibody (10 ug/ml) and the anti-androgen enzalutamide (10 µM), or a combination of each. The extent of growth inhibition was determined after 7 days. The result indicated that treatment with either enzalutamide or Wnt-11 antibody E8 alone reduced 22Rv1 cell proliferation to a small extent (Figure 14). Combined treatment with enzalutamide and Wnt-11 antibody E8 further inhibited 22Rv1 cell proliferation. Q-PCR analysis found that treatment of 22Rv1 cells with enzalutamide increased the expression of WNT11. These observations are consistent with the Wnt-11 antibodies sensitizing enzalutamide-resistant prostate cancer cells to enzalutamide.

### Example 10- Orthotopic mouse tumor assays to measure effects of the antibodies on prostate cancer cell growth and metastasis in vivo

PC-3M cells growing in exponential growth phase were harvested by trypsinization, followed by centrifugation at 335xg relative centrifugal force (RCF) in a centrifuge. The supernatant was removed by aspiration. Cell pellet resuspended in approximately 10× volume of cell culture medium and counted. The cell suspension centrifuged again and processed as above and finally resuspended in HBSS-/- with Matrigel (v/v, 1:1) at a density of 4×107 cells per ml. Cell viability determined to be ≥95% by trypan blue exclusion.

Forty-five mice inoculated by orthotopic injection with a single volume of 50 µl cell suspension (2×10⁶ cells) to establish prostate tumors; thirty mice selected based on bioluminescent imaging.

Groups 1 to 3 consist of 10 animals each. Test antibodies administered according to the regimen below.

### Group Designation and Dosing Schedule

| **Group** | **Treatment** | **Dose (mg/kg)** | **Dosing volume (ml/kg)** | **Frequency of dosing** | **No. of Animals** | **Route of administration** | **Treatment duration** |
|---|---|---|---|---|---|---|---|
| 1 | Control Rat IgG | 10 | 8 | 2 times a week | 10 | i.p. | 4 weeks |
| 2 | Wnt-11 antibody E8 | 8.75 | 7 | 2 times a week | 10 | i.p. | 4 weeks |
| 3 | Wnt-11 antibody F10 | 10 | 8 | 2 times a week | 10 | i.p. | 4 weeks |

10 mice, average weight 30 g = 300 g, each treatment at 10 mg/kg = 3 mg. Total of 8 antibody treatments (3 × per week for 4 weeks) = 24 mg total antibody.

Control antibody: 2 × 10 mg provided - resuspended in sterile PBS at 1.25 mg/ml (total 8 ml), prepare aliquots and store at -20 °C. Use at 10 mg/kg (e.g 4 ml/kg; 300 ug per mouse based on 30 g mouse = 120 ul/mouse).
Antibody E8 (1.25 mg/ml): 7 ml/kg (262.5 ug per mouse based on 30 g mouse = 210 ul/mouse). 10 mice = 80 treatments at 210 ul = 16.8 ml (15 ml provided)
Antibody F10 (1.25 mg/ml) = 8 ml/kg (300 ug per mouse based on 30 g mouse = 120 ul/mouse). 10 mice = 80 treatments at 120 ul = 9.6 ml (10 ml provided)
In a pilot experiment designed to test effects on metastasis, not tumor growth, anti-Wnt-11 antibodies E8 (A, 8.75 mg/kg) and F10 (B, 10 mg/kg) did not show statistically significant effects on tumor growth, compared to control IgG (10 mg/kg)

The following Table shows average tumor weights in the three groups of mice treated as indicated and extent of inhibition by Wnt-11 antibodies.

| Tumor weight | Av (g) | SD (g) | SEM (g) | p value | Tumor inhibition (C-T)/C×100% |
|---|---|---|---|---|---|
| 1: Control IgG | 1.282 | 0.547 | 0.183 | | |
| 2: Wnt-11 antibody A | 0.936 | 0.504 | 0.159 | 0.168 | 27.03% |
| 3: Wnt-11 antibody B | 1.136 | 0.539 | 0.171 | 0.566 | 11.40% |

The following Table shows numbers and % of the indicated organs with metastases in the three groups of mice, differences highlighted in bold

| | 1: Control IgG | | 2: Ab1 E8 | | 3:Ab2 F10 | |
|---|---|---|---|---|---|---|
| Lymph nodes | 20/27 | 74% | 22/30 | 73% | 22/30 | 73% |
| Lungs | 2/9 | 22% | 1/10 | 10% | 1/10 | 10% |
| Liver | 3/9 | 33% | **2/10** | **20%** | 3/10 | 30% |
| Spleen | 5/9 | 56% | 5/10 | 50% | 6/10 | 60% |
| Kidneys | 8/18 | 44% | **4/20** | **20%** | 8/20 | 40% |

However, tumor size was reduced in some E8 (Ab1)-treated mice. Immunohistochemical analysis of tumors from control IgG- and E8-treated mice found a significant reduction in Ki67 staining intensity in Ab1 (E8)-treated mice. Analysis of metastasis found Ab1 (E8) reduced metastasis to liver and kidney in some mice. Neither antibody showed toxicity, based on animal body weight. (Figure 15).

### Example 11- Chimeric antibody peptide ELISA

96-well ELISA plates (Nunc, Thermo Fisher Scientific) were coated at 4oC for 16 hours with 100 ul per well of 10 mg/ml Wnt-11 peptide (LLDLERGTRESAC SEQ ID NO: 76) dissolved in DMSO. The plates were rinsed three times with PBS, blocked with 1% BSA/PBS for 1 hour, incubated with different concentrations of control human IgG, control rat IgG, rat E8 and F10 and chimeric E8h4hk antibodies, each diluted in 1% BSA/PBS, washed, probed with HRP-conjugated anti-rat IgG (Jackson Immunoresearch 712-035-153) or anti-human IgG (Genscript A01854) for 1 hour, washed again, probed with streptavidin-HRP for 1 hour, washed again and developed with TMB ELISA Substrate (Highest Sensitivity) (AbCam, ab171522) in the dark for 30 minutes and read in a plate reader, according to manufacturer's instructions. kDs were calculated after subtraction of background obtained using control IgG antibody and binding to control peptide.

Figure 16 Left graph shows peptide ELISAs measuring binding (absorbance at 360 nM) of the antibody E8 (Ab1) and control IgG to the peptide LLDLERGTRESA (SEQ ID NO: 3). Right graph shows that peptide ELISAs measuring binding (absorbance at 360 nM) of the antibody E8 (Ab1) and chimeric E8 (hlgG4kappa) binding to the same peptide LLDLERGTRESA (SEQ ID NO: 3) with the calculated Kd values indicated under the graph.

### Example 12-Chimeric WNT11 antibody - inhibitory activity in sphere formation assays

DU145 sphere formation assays were carried out as described in the materials and methods section.

Cells were detached using TrypLE (1x, Gibco) and then seeded in either ultra-low attachment 24-well (Corning) in quadruplicate at 500 - 1000 cells/well. Sphere medium consisted of serum-free DMEM: F12 supplemented with B27 (Invitrogen), 20 ng/mL EGF (Epithelial Growth Factor, Life Technologies), 20 ng/mL bFGF (basic fibroblast growth factor, BD Biosciences) and 1% Pen/Strep (100 units/mL penicillin, 100 µg/mL streptomycin, Invitrogen, UK). Cells were cultured undisturbed for 5-7 days. At endpoint spheres were counted manually under a microscope using a 10X magnification lens and sphere numbers are presented relative to the control condition.

The chimeric anti-Wnt-11 antibody E8h4hk, when used at 40 ug/ml, had a similar inhibitory effect as the rat E8 antibody used at 20 ug/ml. Sphere formation was similar in the presence of their respective control antibodies (human IgG at 40 ug/ml and rat IgG at 20 ug/ml) (figure 17).

### Example 13- Analysis of WNT11 antibody epitope

ELISA assays were carried out using peptides corresponding to the same region in several other WNT family members. The results indicate that the E8 antibody did not recognise any of these peptides, consistent with them not recognising other WNT family members. Note all peptides include a C-terminal cysteine, also present in the WNT11 peptide injected into rats to generate the E8 antibody (Figure 18A).

### Peptides:

| | |
|---|---|
| WNT3/3A | DSLAIFGPVLDKATRESAC (SEQ ID NO: 142) |
| WNT7A | GKELKVGSREAAC (SEQ ID NO: 143) |
| WNT7B | GQELRVGSREAAC (SEQ ID NO: 144) |
| WNT9A | RASLLKRGFKETAC (SEQ ID NO: 145) |
| WNT10A | YESPIFSRGFRESAC (SEQ ID NO: 146) |
| WNT11 | LLDLERGTRESAC (SEQ ID NO: 76) |

ELISA assays were also carried out using a panel of peptides in which each single residue in the original Wnt-11 peptide sequence LLDLERGTRESA was changed to alanine (Figure 18 B).

### Peptides:

wt101 LLDLERGTRESAC (SEQ ID NO: 76)
P01 **A**LDLERGTRESA (SEQ ID NO: 77)
P02 L**A**DLERGTRESA (SEQ ID NO: 78)
P03 LL**A**LERGTRESA (SEQ ID NO: 35)
P04 LLD**A**ERGTRESA (SEQ ID NO: 36)
P05 LLDL**A**RGTRESA (SEQ ID NO: 79)
P06 LLDLE**A**GTRESA (SEQ ID NO: 80)
P07 LLDLER**A**TRESA (SEQ ID NO: 81)
P08 LLDLERG**A**RESA (SEQ ID NO: 82)
P09 LLDLERGT**A**ESA (SEQ ID NO: 83)
P10 LLDLERGTR**A**SA (SEQ ID NO: 84)
P11 LLDLERGTRE**A**A (SEQ ID NO: 85)
P12 **AA**DLERGTRESA (SEQ ID NO: 86)
P13 LL**AA**ERGTRESA (SEQ ID NO: 87)
P14 LLDL**AA**GTRESA (SEQ ID NO: 88)
P15 LLDLER**AA**RESA (SEQ ID NO: 89)
P16 LLDLERGT**AA**SA (SEQ ID NO: 90)
P17 **AAAA**ERGTRESA (SEQ ID NO: 91)
P18 LLDL**AAAA**RESA (SEQ ID NO: 92)
P19 LLDLERGT**AAAA** (SEQ ID NO: 93)

In addition, pairs of residues were changed to alanine and groups of four residues were changed to alanine. The results indicated that residues at positions 1-6 (LLDLER (SEQ ID NO: 1)) are required for E8 antibody binding, residues at positions 7 and 8 (GT) are not required, and residues at positions 9-11 (RES) are not essential or sufficient. This is in keeping with E8 not recognising recombinant WNT3A protein in western blots (Figure 18 C), as WNT3A has the same sequence (bold) at positions 8-12 (GPVLDKA**TRESA** (SEQ ID NO: 94)). Experiments were also carried out for Wnt-11 antibody F10, and the results were similar, again finding that the integrity of the sequence LLDLER (SEQ ID NO: 1) was most important for peptide binding.

### Example 14- Point mutations in the Wnt-11 antibody epitope site

Site-directed mutagenesis of human WNT11 expression plasmid was used to generate point mutants in which amino acid residues in the region of the antibody epitope sequence LLDLERG (SEQ ID NO: 95) were altered to alanine (L, L, D, L, E and G) or to aspartic acid (R).

Sequence removed in antibody epitope deletion mutant Wnt11 m (AE) was the following

| | |
|---|---|
| Wnt11 | NY L¹⁰¹L¹⁰²D¹⁰³L¹⁰⁴E¹⁰⁵R¹⁰⁶G¹⁰⁷TRESA **(SEQ ID NO: 140)** |
| Wnt11m | NY - - - - - - - RESA **(SEQ ID NO: 141)** |

The following primers for mutagenesis were used
L101A forward 5'-3' gacctcGAGAGAGGGACCCGGGAG (SEQ ID NO: 96)
L101A reverse 5'-3 aagcgcATAGTTGGGGGCGAGCTC (SEQ ID NO: 97)
L102A forward 5'-3' cctcGAGAGAGGGACCCGGGAG (SEQ ID NO: 98)
L102A reverse 5'-3' tcagcCAAATAGTTGGGGGCGAGC (SEQ ID NO: 99)
D103A forward 5'-3' CTATTTGCTTgccctcGAGAGAGGGACC (SEQ ID NO: 100)
D103A reverse 5'-3' TTGGGGGCGAGCTCAATG (SEQ ID NO: 101)
L104A forward 5'-3' agaggtACCCGGGAGTCGGCCTTC (SEQ ID NO: 102)
L104A reverse 5'-3' ctccgcGTCAAGCAAATAGTTGGGGGCG (SEQ ID NO: 103)
E105A forward 5'-3' aggtACCCGGGAGTCGGCCTTC (SEQ ID NO: 104)
E105A reverse 5'-3' ctcgcCAGGTCAAGCAAATAGTTGGGGG (SEQ ID NO: 105)
R106E forward 5'-3' gaagggACCCGGGAGTCGGCCTTC (SEQ ID NO: 106)
R106E reverse 5'-3' ctcgagGTCAAGCAAATAGTTGGGGGCG (SEQ ID NO: 107)
G107A forward 5'-3' agcgaccCGGGAGTCGGCCTTCGTG (SEQ ID NO: 108)
G107A reverse 5'-3' ctctcgagGTCAAGCAAATAGTTGGGGGC (SEQ ID NO: 109)

The mutations were confirmed by sequencing. Cells (HT1080 and HEK293) were transiently transfected with plasmids expressing wild-type Wnt-11, the antibody epitope deletion mutant (AE, deleted for LLDLERG (SEQ ID NO: 95)) and the point mutants. Western blotting of cell extracts using goat anti-Wnt-11 antibodies and GAPDH as a loading control was used to compare their expression levels. The results showed that the point mutations did not affect Wnt-11 protein expression (Figure 19 B and C). It did also not affect their secretion, as measured by dot blot analysis (not shown). Western blotting of cell extracts was then used to compare the signaling activities of the Wnt-11 mutants. Expression of wild-type Wnt-11 resulted in the mobility shift of Dvl-3, whereas expression of the antibody epitope deletion mutant did not (Figure 19 D and E). Wnt-11 with point mutations at either position 3 (D to A) or 4 (L to A) in the sequence LLDLERG (SEQ ID NO: 95) had significantly lower activity than wild-type Wnt-11, while Wnt-11 with a point mutation at position 6 (R to E) had lower activity in HT1080 cells but not in HEK 293 cells. Point mutations at positions 1 (L to A), 2 (L to A), 5 (E to A) or 7 (G to A) did not significantly affect activity in this assay.

The activities of some of the Wnt-11 point mutants were also compared using gene reporter assays measuring Wnt-11 inhibition of Wnt-3a-dependent transcription (Figure 20). The results indicated that Wnt-11 with point mutations at either position 3 (D to A) or 4 (L to A) in the sequence LLDLERG (SEQ ID NO: 95), as for the antibody epitope deletion mutant AE, had significantly lower activity than wild-type Wnt-11. Point mutations at positions 5 (E to A) or 7 (G to A) did not affect activity in this assay, while point mutation at position 6 (R to E) had variable effects that did not reach significance.

To summarise, DL, in the sequence LLDLERG (SEQ ID NO: 95) in Wnt-11 are important for Wnt-11 to increase Dvl-3 phosphorylation, as measured by mobility shift in western blots, and to inhibit Wnt-3a activation of beta-catenin/Tcf-dependent transcription, as measured using gene reporter assays.

## Claims

1. An agent binding specifically to the sequence shown in SEQ ID NO: 1, SEQ ID NO: 2 or SEQ ID NO: 3, wherein said agent is an immunoglobulin agent or a non-immunoglobulin agent selected from the group consisting of a peptide aptamer, a nucleic acid aptamer, a DARPin, an affibody, and an anticalin.

2. The agent according to claim 1 which binds specifically to the sequence shown in SEQ ID NO: 3.

3. The agent according to any one of claims 1 or 2, wherein said immunoglobulin agent is an antibody or an antigen-binding fragment of said antibody.

4. The agent according to claim 3, wherein said antigen-binding fragment is selected from the group consisting of Fv, Fab, F(ab')₂ and Fab'.

5. The agent according to any one of claims 3 or 4, wherein said antibody or said antigen-binding fragment comprises within the heavy chain:
- a CDR comprising the amino acid sequence shown in SEQ ID NO: 4 [CDR-H1], a CDR comprising the amino acid sequence shown in SEQ ID NO: 5 [CDR-H2], and a CDR comprising the amino acid sequence shown in SEQ ID NO: 6 [CDR-H3], or a functionally equivalent variant of said CDRs,
and/or comprises within the light chain:
- a CDR comprising the amino acid sequence shown in SEQ ID NO: 7 [CDR-L1], a CDR comprising the amino acid sequence shown in SEQ ID NO: 8 [CDR-L2], and a CDR comprising the amino acid sequence shown in SEQ ID NO: 9 [CDR-L3], or a functionally equivalent variant of said CDRs
or wherein said antibody or said antigen-binding fragment comprises within the heavy chain
- a CDR comprising the amino acid sequence shown in SEQ ID NO: 10 [CDR-H1], a CDR comprising the amino acid sequence shown in SEQ ID NO: 11 [CDR-H2], and a CDR comprising the amino acid sequence shown in SEQ ID NO: 12 [CDR-H3], or a functionally equivalent variant of said CDRs,
and/or within the light chain:
- a CDR comprising the amino acid sequence shown in SEQ ID NO: 13 [CDR-L1], a CDR comprising the amino acid sequence shown in SEQ ID NO: 14 [CDR-L2], and a CDR comprising the amino acid sequence shown in SEQ ID NO: 15 [CDR-L3], or a functionally equivalent variant of said CDRs.

6. The agent according to claim 5, wherein the said antibody or said antigen-binding fragment comprises within the heavy chain:
- a CDR comprising the amino acid sequence shown in SEQ ID NO: 4 [CDR -H1], a CDR comprising the amino acid sequence shown in SEQ ID NO: 5 [CDR-H2], and a CDR comprising the amino acid sequence shown in SEQ ID NO: 6 [CDR-H3] and comprises within the light chain:
- a CDR comprising the amino acid sequence shown in SEQ ID NO: 7 [CDR-L1], a CDR comprising the amino acid sequence shown in SEQ ID NO: 8 [CDR-L2], and a CDR comprising the amino acid sequence shown in SEQ ID NO: 9 [CDR-L3] or wherein said antibody or said antigen-binding fragment comprises within the heavy chain
- a CDR comprising the amino acid sequence shown in SEQ ID NO: 10 [CDR-H1], a CDR comprising the amino acid sequence shown in SEQ ID NO: 11 [CDR-H2], and a CDR comprising the amino acid sequence shown in SEQ ID NO: 12 [CDR-H3] and within the light chain:
- a CDR comprising the amino acid sequence shown in SEQ ID NO: 13 [CDR-L1], a CDR comprising the amino acid sequence shown in SEQ ID NO: 14 [CDR-L2], and a CDR comprising the amino acid sequence shown in SEQ ID NO: 15 [CDR-L3].

7. The agent according to any one of claims 5 or 6, wherein the heavy chain variable region of the antibody has the sequence shown in SEQ ID NO: 16 and the light chain sequence variable region has the sequence shown in SEQ ID NO: 17, or wherein the heavy chain variable region of the antibody has the sequence shown in SEQ ID NO: 18 and the light chain sequence variable region has the sequence shown in SEQ ID NO: 19.

8. The agent according to any one of claims 3 to 6, wherein said antibody is a murine-derived monoclonal antibody, a human-murine chimeric antibody, or a humanized antibody.

9. The agent according to claim 8, wherein the human-murine chimeric antibody comprises a human IgG4k constant sequence, particularly the human IgG4k constant sequence is selected from the group consisting of SEQ ID NO: 20 or SEQ ID NO: 21, particularly the human-murine chimeric antibody has the heavy chain sequence shown in SEQ ID NO: 22 and the light chain sequence shown in SEQ ID NO: 23.

10. An antibody construct comprising the antigen-binding fragment according to any one of claims 3 to 6, wherein the antibody construct is selected from the group consisting of scFv, scFv-Fc, minibody, (scFv)₂ and diabody.

11. A combination comprising:
a) an agent binding to the sequence shown in SEQ ID NO: 1 , SEQ ID NO: 2 or SEQ ID NO: 3 according to any one of claims 1 to 9 or an antibody construct according to claim 10, and
b) an anti-androgen compound.

12. A nucleic acid selected form the group consisting of:
a) a nucleic acid encoding the agent according to any one of claims 1 to 9 wherein the agent is a protein compound, or the antibody construct according to claim 10 and
b) a complementary nucleic acid of a nucleic acid as defined in a) or
an expression cassette comprising said nucleic acid or
a vector comprising said nucleic acid or said expression cassette, or a cell comprising said nucleic acid, or said expression cassette, or said vector.

13. A nanoparticle comprising the agent according to any one of claims 1 to 9 or the antibody construct according to claim 10 or the combination according to claim 11.

14. A pharmaceutical composition comprising a therapeutically effective amount of an agent according to any one of claims 1 to 9, an antibody construct according to claim 10, a combination according to claim 11, or a nanoparticle according to claim 13, together with a pharmaceutically acceptable excipient or carrier.

15. An agent according to any one of claims 1 to 9, an antibody construct according to claim 10, a combination according to claim 11, a nanoparticle according to claim 13 or a pharmaceutical composition according to claim 12 for use in medicine.

16. An agent according to any one of claims 1 to 9, an antibody construct according to claim 10, a combination according to claim 11, a nanoparticle according to claim 13 or a pharmaceutical composition according to claim 12 for use in the treatment of cancer, metastasis or fibrosis, particularly wherein the cancer is selected from the group consisting of prostate cancer, colorectal cancer, breast cancer, melanoma, pancreatic cancer, cervical cancer, glioma, bladder cancer, urothelial cancer, kidney cancer, renal cancer, testicular cancer, thyroid cancer and lung cancer, and/or particularly wherein the cancer is **characterized by** presenting tumor cells having high WNT-11 expression and /or wherein the cancer is advanced cancer, drug resistant and/or metastatic cancer.
